# EUROPEAN PATENT APPLICATION

(11) **EP 4 692 345 A1**
(43) Date of publication of application: **11.02.2026**
(21) Application number: 24778227.9
(22) Date of filing: 29.03.2024
(51) Int. Cl.: C12N 15/11, C12N 15/67, C12N 15/68

(54) **POLYNUCLEOTIDE MOLECULE COMPRISING POLY(A) AND USE THEREOF**

(30) Priority: 29.03.2023 WO PCT/CN2023/084701
(71) Applicant: Shenzhen Shenxin Biotechnology Co., Ltd., Shenzhen, Guangdong 518000 (CN)
(72) Inventor: HUANG, Hui, Shenzhen, Guangdong 518000 (CN); LI, Linxian, Shenzhen, Guangdong 518000 (CN)
(74) Representative: Nederlandsch Octrooibureau
(86) International application number: PCT/CN2024/084805
(87) International publication number: WO 2024/199441

(57) **Abstract**

A polynucleotide, the polynucleotide comprising a modified poly(A) having at least 30 nucleotides. The modified poly(A) comprises: i) at least two R elements, the R elements being defined as polynucleotide composed of A nucleotide; and ii) at least one S element. The S element: a) is a nucleotide that is not an A nucleotide, or b) is composed of at least two nucleotides, and the nucleotides at both ends are not A nucleotide. The total number of S elements is one less than the total number of R elements, the S elements are connected to the R elements, and each S element is flanked by the R elements. The present invention also relates to a vector and a host cell comprising the polynucleotide, a method for using the polynucleotide to prepare RNA and the RNA prepared therefrom, a pharmaceutical composition or LNP comprising the polynucleotide, the RNA, the vector, and/or the host cell, and a use thereof.

## Description

### Field of the invention

The invention belongs to the field of biotechnology. In particular, the invention relates to a polynucleotide molecule comprising a modified poly(A) and its use in preparing a medicament.

### Background

mRNAs in eukaryotic cells comprise a polynucleotide of various lengths during transcription (also known as poly(A) sequence, poly(A) tail, or poly(A)). Although its sequence composition is simple, poly(A) at the 3' end plays a key role in several aspects of the transcript life cycle: i) poly(A) mediates the transfer of mature mRNA to cytoplasm; ii) poly(A) has a key regulatory role in improving protein translation efficiency; and iii) poly(A) can modulate the half-life of mRNA, preventing mRNA from nuclease degradation. When mRNA is not required for translation, poly(A) shortening is one of the key steps for initiating mRNA attenuation. Thus, poly(A) is one of the key elements to regulate mRNA.

In addition, mRNA has the potential to synthesize any kind of protein, and due to its economic, safe, fast, and flexible properties, mRNA drugs have great application potentials in the fields of infection prevention, cancer, and many diseases including rare diseases. For example, mRNA vaccines as novel vaccines have a short research and development cycle, making it possible to rapidly develop new candidate vaccines against various pathogens. In mRNA drug development, there are several important elements, such as 5'-UTR, 3'-UTR and poly(A). However, the natural poly(A) is prone to random increase or decrease in plasmid so that mRNA has less stability and/or translation efficiency, which is unfavorable for control or quality determination in process, and also leads to uncontrollable inter-batch mRNA activity. Although modified poly(A)s have been reported, there still is a need to develop new poly(A) to improve translation efficiency (protein expression level) and/or mRNA stability.

### Summary of the invention

In order to solve the instability problem of poly(A) in mRNA and improve the translation efficiency of mRNA (increase protein expression level) and/or mRNA stability, the invention provides a modified poly(A) polynucleotide. In addition, the invention further provides a vector, a host cell, a lipid nanoparticle and a pharmaceutical composition comprising the polynucleotide as well as use thereof in preparation of a medicament. In addition, the invention further provides a method of preparing RNA by using the polynucleotide and an RNA prepared by the method, a method of preparing a polypeptide or protein by using the polynucleotide and a polypeptide or protein prepared by the method, and use of the RNA, the polypeptide or protein, and a pharmaceutical composition comprising the RNA or the polypeptide or protein in preparation of a medicament.

In a first aspect, the invention provides a polynucleotide comprising a modified poly(A), wherein the modified poly(A) comprises at least 30 nucleotides, and the modified poly(A) comprises:
i) at least two R elements, which are defined as polynucleotides consisting of A nucleotide; and
ii) at least one S element, wherein the S element:
   a) is a nucleotide that is not A nucleotide, or
   b) consists of at least two nucleotides, wherein the nucleotides at both ends are not A nucleotide;
wherein the total number of the S element is 1 less than the total number of the R element, the S element is connected to the R element, and each of the S elements is flanked by the R element at both ends.

In some embodiments, the modified poly(A) satisfies one of the followings:
(1) the number of the nucleotides of the modified poly(A) is in a range of 30-70, 30-80, 30-90, 30-100, 30-110, 30-120, 30-130, 30-140, 40-70, 40-80, 40-90, 40-100, 40-110, 40-120, 40-130, 40-140, 50-70, 50-80, 50-90, 50-100, 50-110, 50-120, 50-130, 50-140, 60-70, 60-80, 60-90, 60-100, 60-110, 60-120, 60-130, 60-140, 70-80, 70-90, 70-100, 70-110, 70-120, 70-130, 70-140, 80-90, 80-100, 80-110, 80-120, 80-130, 80-140, 90-100, 90-110, 90-120, 90-130 or 90-140;
   preferably, the number of the nucleotides of the modified poly(A) is in a range of 31-70, 35-70, 41-70, 45-70, 51-70, 55-70, 61-70, 65-70, 30-75, 35-75, 40-75, 45-75, 50-75, 55-75, 60-75, 65-75, 70-75, 30-79, 35-79, 40-79, 45-79, 50-79, 55-79, 60-79, 65-79, 70-79, 75-79, 81-99, 85-99, 90-99, 95-99, 101-109, 105-109, 111-119, 115-119, 105-119, 109-119, 115-119, 122-125, 127-129 or 133-140;
(2) the number of A nucleotide of at least one R element is in a range of 5-20, 8-20, 10-20, 15-20, 5-30, 8-30, 10-30, 20-30, 5-40, 8-40, 10-40, 20-40, 30-40, 5-50, 8-50, 10-50, 20-50, 30-50, 40-50, 5-60, 8-60, 10-60, 20-60, 30-60, 40-60, 50-60, 5-70, 8-70, 10-70, 20-70, 30-70, 40-70, 50-70, 60-70, 5-80, 8-80, 10-80, 20-80, 30-80, 40-80, 50-80, 60-80, 70-80, 5-90, 8-90, 10-90, 20-90, 30-90, 40-90, 50-90, 60-90, 70-90, 80-90, 5-100, 8-100, 10-100, 20-100, 30-100, 40-100, 50-100, 60-100, 70-100, 80-100, 90-100, 5-110, 8-110, 10-110, 20-110, 30-110, 40-110, 50-110, 60-110, 70-110, 80-110, 90-110 or 100-110;
   preferably, the number of A nucleotide of at least one R element is in a range of 5-19, 8-19, 10-19, 11-19, 13-19, 16-19, 5-18, 8-18, 10-18, 11-18, 13-18, 16-18, 21-29, 31-39, 41-44, 46-49, 51-54, 56-59, 61-64, 66-69, 71-79, 81-99 or greater than 100;
(3) the number of the nucleotides of at least one S element is at least 2, 5, 10, 15 or 20, the number of the nucleotides of at least one S element is no more than 15, no more than 20, no more than 30, no more than 35 or no more than 40; preferably, the number of the nucleotides of at least one S element is in a range of 2-10, 2-15, 2-20, 2-25, 3-10, 3-15, 3-20 or 3-25;
(4) the number of the S element is 1, at least 2, at least 5, at least 8 or at least 10, preferably, the number of the S element is 1, 2, 3 or 4.

In some embodiments, the number of nucleotides of the modified poly(A) is 71-79, and the modified poly(A) further satisfies at least one of the following conditions:
(1) the number of A nucleotide of at least one R element is less than 35, 36-39, 41-54, 56-59, 61-64, or greater than 65; and
(2) the number of A nucleotide of at least one R element is not an integer between 35 and 65.

In some embodiments, the modified poly(A) comprises at least three R elements or at least four R elements.

In some embodiments, the modified poly(A) comprises a nucleotide sequence having at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one of the nucleotide sequences of SEQ ID NOs: 2-100, wherein the R element remains unchanged.

In some embodiments, the nucleotide sequence of the modified poly(A) has at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one of the nucleotide sequences of SEQ ID NOs: 2-100, wherein the R element remains unchanged.

In some embodiments, the modified poly(A) comprises any of the nucleotide sequences of SEQ ID NOs: 2-100.

In some embodiments, the nucleotide sequence of the modified poly(A) is set forth in any one of SEQ ID NOs: 2-100.

In some embodiments, the polynucleotide further comprises a nucleic acid encoding at least one of 5'-UTR, a polypeptide or protein of interest, and 3'-UTR that are located upstream of the modified poly(A);

In some embodiments, the polynucleotide can be transcribed in vitro to RNA.

In some embodiments, the polynucleotide is DNA.

In a second aspect, the invention further provides a vector comprising any one of the above polynucleotides.

In some embodiments, the vector is an expression vector; further, the vector further comprises at least one of a promoter, 5'-UTR and 3'-UTR that are located upstream of the modified poly(A); further, the modified poly(A), promoter, 5'-UTR and 3'-UTR are operably linked to each other;

Further, the vector further comprises a nucleic acid encoding a polypeptide or protein of interest, and the nucleic acid encoding a polypeptide or protein of interest is located between the 5'-UTR and the 3'-UTR.

In a third aspect, the invention provides a host cell comprising any one of the above polynucleotides or any one of the above vectors;

Further, the host cell is an antigen-presenting cell; furthermore, the host cell is dendritic cell, monocyte, or macrophage.

In a fourth aspect, the invention provides a method of preparing RNA, comprising a step of performing transcription using any one of the above polynucleotides as coding strand.

In a fifth aspect, the invention provides an RNA obtained by the method of preparing RNA described above;
preferably, the RNA is mRNA.

In a sixth aspect, the invention provides a method of preparing a polynucleotide, comprising a step of introducing any of the polynucleotides described above into a host cell and culturing the host cell containing the polynucleotide; further, the host cell comprises

### Escherichia coli.

In a seventh aspect, the invention provides a method of preparing a polypeptide or protein, comprising:
transcribing any of the polynucleotides described above into mRNA; and
translating the mRNA into a polypeptide or protein.

In an eighth aspect, the invention provides a lipid nanoparticle (LNP) comprising any one of the above polynucleotides or any of the above RNAs.

In a ninth aspect, the invention provides a pharmaceutical composition, comprising any one of the above polynucleotides, any one of the above vectors, any one of the above host cells, any one of the above RNAs, any one of the above polypeptides or proteins prepared by the method of preparing a polypeptide or protein, or any one of the above lipid nanoparticles, and a pharmaceutically acceptable excipient.

In a tenth aspect, the invention provides use of any one of the above polynucleotides, any one of the above vectors, any one of the above host cells, any one of the above RNAs, any one of the polypeptides or proteins preparation the method of preparing a polypeptide or protein, any one of the above lipid nanoparticles, or any one of the above pharmaceutical compositions in the preparation of a medicament;

Preferably, the medicament is used in gene therapy, gene vaccination or protein replacement therapy;

Further, the medicament is used in the treatment and/or prevention of a disease; preferably, the medicament is a vaccine;
preferably, the disease is selected from the group consisting of rare diseases, infectious diseases, cancers, hereditary diseases, autoimmune diseases, diabetes, neurodegenerative diseases, cardiovascular diseases, renal vascular diseases and metabolic diseases;
preferably, the cancers comprise one or more of lung cancer, gastric cancer, liver cancer, esophageal cancer, colon cancer, pancreatic cancer, brain cancer, lymphoma, leukemia and prostate cancer; the hereditary diseases comprise one or more of hemophilia, thalassemia and Gaucher's disease; the rare diseases comprise one or more of Brittle Bone Disease, Wilson disease, spinal muscular atrophy, Huntington's disease, Rett syndrome, amyotrophic lateral sclerosis, Duchenne Type Muscular Dystrophy, Friedrichs ataxia, Methylmalonic Acidemia, cystic fibrosis, glycogen storage disease 1a, glycogen storage disease III, Crigler-Najjar syndrome, ornithine transcarbamylase deficiency, Propionic Acidemia, phenylketonuria, hemophilia A, hemophilia B, β-thalassemia, Lafora disease, Dravet syndrome, Alexander disease, Leber's congenital amaurosis, myelodysplastic syndrome, and Homocystinuria due to CBS deficiency;
preferably, the medicament is a nucleic acid drug, wherein the nucleic acid comprises at least one of RNA, messenger RNA (mRNA), DNA, plasmid, ribosomal RNA (rRNA), single guide RNA (sgRNA) and cas9 mRNA.

### Brief Description of Drawings

FIG. 1 is a flow chart of plasmid construction;
FIG. 2 is a plasmid map of luciferase-pcDNA3;
FIG. 3 is a plasmid map of the plasmid B of the invention;
FIG. 4 is a plasmid map of the plasmid C of the invention;
FIG. 5 is a plasmid map of the plasmid D of the invention;
FIG. 6 is a plasmid map of the plasmid F of the invention;
FIG. 7 is a plasmid map of the plasmid G of the invention (taking the insertion of polyA-V0 as an example);
FIG. 8 is a plasmid map of the plasmid H of the invention (taking the insertion of polyA-V0 as an example);
FIGs. 9A, 9B and 9C are liver in-vivo imaging results of mice injected with mRNAs comprising different poly(A);
FIG. 10 is the comparison of in vitro synthesis yields of mRNAs comprising different poly(A); and
FIG. 11 shows the results of stability via sequencing the poly(A) in plasmids comprising different poly(A).

### Detailed description of the invention

### I. Definitions

All patents, patent applications, scientific publications, manufacturers' instructions and guidelines, etc. cited herein are hereby incorporated by reference in their entirety, irrespective of the foregoing and following context. Nothing herein is to be construed as an admission that the invention is not entitled to precede such disclosure.

Unless otherwise specified, scientific and technical terms used herein have the meanings commonly understood by those skilled in the art. Moreover, the protein and nucleic acid chemistry, molecular biology, cell and tissue culture, microbiology related terms used herein are all widely used in the respective fields (see, e.g., Molecular Cloning: A Laboratory Manual, 2nd Edition, J. Sambrook et al. eds., Cold Spring Harbor Laboratory Press, Cold Spring Harbor 1989). Meanwhile, in order to better understand the invention, definitions and explanations of related terms are provided below.

As used herein, the expressions "comprise", "include", "contain" and "have" are openended, meaning that the listed elements, steps or components are included but other unrecited elements, steps or components are not excluded. The expression "consist of' excludes any element, step, or component not specified. The expression "consist essentially of' means that the range is limited to the specified elements, steps, or components, plus optional elements, steps, or components that do not significantly affect the basic and novel properties of the claimed subject matter. It will be understood that the expressions "consist essentially of" and "consist of" are encompassed within the meaning of the expression "comprise".

As used herein, the singular form "a", "an", "this" or "the" and similar referents in the context of describing the invention (especially in the context of the claims) are to be construed to cover both the singular and the plural, unless clearly indicated otherwise from the context. The terms "one or more" or "at least one" encompass 1, 2, 3, 4, 5, 6, 7, 8, 9 or more.

The numerical ranges recited herein are understood to encompass any and all sub-ranges subsumed therein. For example, a range of "1 to 10" should be understood to include not only the explicitly recited values 1 and 10, but also any single value in the range of 1 to 10 (e.g., 2, 3, 4, 5, 6, 7, 8 and 9) and sub-ranges (e.g., 1 to 2, 1.5 to 2.5, 1 to 3, 1.5 to 3.5, 2.5 to 4, 3 to 4.5, etc.). This principle also applies to ranges that use only one value as the minimal or maximal value.

All methods described herein can be performed in any suitable order unless otherwise indicated.

As used herein, the terms "and/or," "any combination thereof," and grammatical equivalents thereof are used interchangeably. These terms are used to express any combination specifically referenced. For example, the following phrases "A, B and/or C" or "A, B, C or any combination thereof" may refer to A alone; B alone; C alone; A and B; B and C; A and C; and A, B and C.

As used herein, the term "wild-type" means that the sequence is naturally occurring and not artificially modified, including naturally occurring mutants.

As used herein, the term "% identity" with respect to sequence refers to the percentage of nucleotides or amino acids that are the same in the optimal alignment between sequences to be compared. The difference between two sequences may be distributed over a local region (segment) or the entire length of the sequence to be compared. The identity between two sequences is typically determined after the optimal alignment over the segment or "comparison window". Optimal alignment may be performed manually, or by means of algorithms known in the art, including, but not limited to, the local homology algorithm described by Smith and Waterman, 1981, Ads App. Math. 2, 482 and Needleman and Wunsch, 1970, J. Mol. Biol. 48, 443, the similarity search method described by Pearson and Lipman, 1988, Proc. Natl Acad. Sci. USA 88, 2444, or using a computer program such as GAP, BESTFIT, FASTA, BLASTP, BLASTN, and TFASTA in Wisconsin Genetics Software Package, Genetics Computer Group, 575 Science Drive, Madison, Wis. For example, the percentage identity of two sequences may be determined using BLASTN or BLASTP algorithm publicly available in the National Center for Biotechnology Information (NCBI) website.

The "% identity" may be obtained by dividing the number of identical positions corresponding to the sequence to be compared by the number of positions to be compared (e.g., the number of locations in the reference sequence) and multiplying by 100 to obtain % identity. In some embodiments, at least about 50%, at least about 55%, at least about 60%, at least about 65%, at least about 70%, at least about 75%, at least about 80%, at least about 85%, at least about 90%, at least about 95%, or about 100% of the area gives the degree of identity. In some embodiments, the degree of identity is given over the full length of the reference sequence. The alignments of the sequence identity may be determined using tools known in the art, preferably with optimal sequence alignments, e.g., Align, with standard settings, preferably EMBOSS::needle, Matrix:Blosum62, Gap Open 10.0, Gap Extend 0.5.

As used herein, "nucleotide" includes deoxyribonucleotides, ribonucleotides, derivatives of deoxyribonucleotides, and derivatives of ribonucleotides. As used herein, "ribonucleotide" is a constituent of ribonucleic acid (RNA), consisting of a molecular base, a molecular pentose and a molecular phosphate, which refers to a nucleotide having a hydroxyl group at 2' position of β-D-ribofuranosyl group. "Deoxyribonucleotide" is a constituent of deoxyribonucleic acid (DNA), also consisting of a molecular base, a molecular pentose and a molecular phosphate, which refers to a nucleotide with hydrogen substituting the hydroxyl at 2' position of β-D-ribofuranosyl group, and is the main chemical component of the chromosome. "nucleotide" is generally referred to by a single letter representing the base thereof: "A" or "A nucleotide" refers to adenine deoxyribonucleotide or adenine ribonucleotide containing adenine, "C" or "C nucleotide" refers to cytosine deoxyribonucleotide or cytosine ribonucleotide containing cytosine, "G" or "G nucleotide" refers to guanine deoxyribonucleotide or guanine ribonucleotide containing guanine, "U" or "U nucleotide" refers to uracil ribonucleotide containing uracil, "T" or "T nucleotide" refers to thymine deoxyribonucleotide containing thymine. As used herein, the term "nucleotide other than A nucleotide" refers to a nucleotide that is not adenine deoxyribonucleotide or adenine ribonucleotide. For example, a nucleotide other than A nucleotide is C, G or T.

As used herein, the term "polynucleotide" generally is any compound that comprises a polymer of deoxyribonucleotides (deoxyribonucleic acid, DNA), a polymer of ribonucleotides (ribonucleic acid, RNA), or a combination thereof. In addition, polynucleotides herein also include derivatives of polynucleotides. The term "derivatives of polynucleotides" includes chemical derivatization of polynucleotides on bases, sugar or phosphate of nucleotides, and polynucleotides containing non-natural nucleotides and nucleotide analogs. Further, herein, a polynucleotide can be in a form of single-stranded or double-stranded linear or covalently closed cyclic molecule. Those skilled in the art should understand that DNA coding strand (sense strand) and the RNA encoded thereby can be regarded as having the same nucleotide sequence, and the thymine deoxyribonucleotide in the DNA coding strand sequence corresponds to the uracil ribonucleotide in the RNA sequence encoded thereby.

A polynucleotide may comprise one or more segments (nucleic acid fragments) (e.g., 1, 2, 3, 4, 5, 6, 7, 8 segments). A polynucleotide may comprise a segment encoding a polypeptide (e.g., a polypeptide or a polypeptide antigen as described herein) or protein of interest. In particular embodiments, a polynucleotide may comprise a segment encoding a polypeptide or protein of interest and a regulatory segment (including but not limited to segments for transcription and translation regulation). In some embodiments, the regulatory segment comprises one or more of a promoter, a 5' untranslated region (5'-UTR), a 3' untranslated region (3'-UTR) and a poly(A).

The term "promoter" refers to a polynucleotide located upstream of the 5' end of the coding region of a gene, which contains a conserved sequence required for specific binding and transcription initiation of RNA polymerase, and can activate RNA polymerase, so that RNA polymerase can accurately bind to template DNA and have the specificity of transcription initiation. Promoter may be derived from viruses, bacteria, fungi, plants, insects and animals. Representative examples of promoter include bacteriophage T7 promoter, bacteriophage T3 promoter, SP6 promoter, lac operon promoter, tac promoter, SV40 late promoter, SV40 early promoter, RSV-LTR promoter, CMV IE promoter, SV40 early promoter, or SV 40 late promoter and CMV IE promoter.

As used herein, the term "5' untranslated region" or "5'-UTR" may be an RNA sequence located upstream of coding sequence in mRNA and is not translated to a protein. The 5'-UTR in a gene typically starts from the transcription start site, and ends at the nucleotide upstream of the translation start codon of coding sequence. 5'-UTR may contain elements that control gene expression, such as ribosome binding site, 5'-terminal oligopyrimidine tract, and translation initiation signal such as Kozak sequence. mRNA can be modified by adding a 5' cap after transcription. Therefore, 5'-UTR in mature mRNA may also refer to the RNA sequence between the 5' cap and the start codon.

As used herein, the term "3' untranslated region" or "3'-UTR" may be a polynucleotide (RNA) located downstream of coding sequence in an mRNA and is not translated to a protein. The 3'-UTR in mRNA is located between the stop codon of the coding sequence and poly(A), e.g., from the nucleotide downstream of the stop codon to the end of the nucleotide upstream of poly(A).

As used herein, the terms "polyadenylic acid", "poly(A) sequence", "poly(A)" and "poly(A) tail" are used interchangeably and naturally occurring poly(A) generally consists of adenine ribonucleotides. In accordance with the invention, the term "modified poly(A)" refers to a poly(A) comprising a nucleotide or a nucleotide segment other than adenine ribonucleotide. poly(A) is typically located at the 3' end of mRNA, e.g., 3' end (downstream) of 3'-UTR.

As used herein, the term "5' cap structure": 5' cap structure is typically located at 5' end of a mature mRNA. In some embodiments, 5' cap structure is attached to 5'-end of mRNA by a 5'-5'-triphosphate linkage. 5' cap structure is typically formed from modified (e.g. methylated) ribonucleotides (especially from guanine nucleotide derivatives). For example, m7GpppN (cap0, or "cap0", is a cap structure formed by a 5',5'-phosphodiester bond formed by reaction of the 5' phosphate group of hnRNA with the 5'-phosphate group of m7GTP under the action of guanylyl transferase), where N is the terminal 5' nucleotide of a nucleic acid carrying 5' cap structure. In some embodiments, the 5' cap structure includes but is not limited to cap 0, cap 1 (a cap structure formed by further methylation of 2'-OH on sugar of the first nucleotide of hnRNA on the basis of cap 0, or known as "cap1", or "cap1 type cap"), cap 2 (a cap structure formed by further methylation of 2'-OH on sugar of the second nucleotide of hnRNA on the basis of cap 1, or known as "cap2", or "cap2 type cap"), cap 4, cap 0 analogs, cap 1 analogs, cap 2 analogs, or cap 4 analogs.

As used herein, the term "expression" includes transcription and/or translation of a nucleic acid. Thus, expression may involves the production of transcripts and/or polypeptides. The term "transcription" involves the process of transcribing a genetic code in a DNA sequence into RNA (transcript). The term "in vitro transcription" refers to the in vitro synthesis of RNA, in particular mRNA, in a cell free system (see, e.g., Pardi N., Muramatsu H., Weissman D., Karikó K. (2013). In: Rabinovich P. (eds) Synthetic Messenger RNA and Cell Metabolism Modulation. Methods in Molecular Biology (Methods and Protocols), vol 969. Humana Press, Totowa, NJ.). A vector that may be used to produce transcripts is also known as a "transcription vector," which contains regulatory sequences required for transcription. The term "transcription" encompasses "in vitro transcription."

As used herein, the term "polypeptide" refers to a polymer comprising more than two amino acids covalently linked by peptide bond. A "protein" may comprise one or more polypeptides wherein the polypeptides interact covalently or non-covalently. Unless otherwise specified, "polypeptide" and "protein" may be used interchangeably.

As used herein, the term "host cell" refers to a cell for receiving, holding, replicating, expressing a polynucleotide or vector. The term "host cell" encompasses prokaryotic cells (e.g., *E. coli*) or eukaryotic cells (e.g., yeast cells and insect cells), e.g. cells from humans, mice, hamsters, pigs, goats, primates. The cells may be derived from a variety of tissue types and include primary cells and cell lines. Some specific examples include keratinocytes, peripheral blood leukocytes, bone marrow stem cells, and embryonic stem cells. In other embodiments, the host cell is an antigen presenting cell, in particular dendritic cell, monocyte, or macrophage. Nucleic acids may be present in the host cell in a single copy or in multiple copies. In some embodiments, the host cell may be a cell in which the polypeptide of the invention is expressed.

As used herein, the term "recombinant" refers to homologous recombination in which a nucleotide sequence exchange occurs between two highly similar or identical nucleotide sequences contained in, for example, DNA molecules such as DNA plasmids and/or chromosomes. The term "recombinant" encompasses intra-molecular homologous recombination events, i.e., a recombination of two highly similar or identical nucleotide sequences contained in the same DNA molecule. In the context of the invention, this specifically refers to the recombination of a nucleotide sequence encoding poly(A) (e.g., modified or native poly(A)) in a plasmid with another highly similar or identical nucleotide sequence contained in the same DNA plasmid during the plasmid amplification, and/or a recombination of the two end portions of such nucleotide sequence. The term "recombinant" also encompasses an intermolecular homologous recombination event between a nucleotide sequence encoding poly(A) (e.g., modified or native poly(A)) contained in a DNA plasmid with a highly similar or identical nucleotide sequences contained in another DNA molecule (e.g., another DNA plasmid contained in the same cell).

As used herein, the term "plasmid" generally refers to a cyclic DNA molecule, and may also encompass linearized DNA molecules. In particular, the term "plasmid" also encompasses a molecule obtained by linearizing a cyclic plasmid by digesting the cyclic plasmid, e.g., with a restriction enzyme, thereby converting the cyclic plasmid molecule into a linear molecule. Plasmid can be replicated, i.e., amplified in cells independently of genetic information stored as chromosomal DNA, and can be used for cloning, i.e., for amplification of genetic information in bacterial cells. Preferably, the DNA plasmid is a medium-copy or a high-copy plasmid, more preferably a high-copy plasmid. Examples of such high-copy plasmid include, for example, pUC and pTZ plasmid or any other plasmids containing an origin of replication supporting high copy number of plasmid (e.g. pMB1, pCoIE1).

As used herein, the term "treatment" or the like is used herein to generally mean obtaining a desired pharmacological and/or physiological effect. Thus, the treatment of the invention may involve the treatment of a state of a certain disease, but may also involve prophylactic treatment in terms of complete or partial prevention of a disease or a symptom thereof. Preferably, the term "treatment" is understood to be therapeutic in partial or complete cure of a disease and/or an adverse effect and/or a symptom resulted from the disease. The treatment may also be a prophylactic or preventive treatment, i.e., a measure taken to prevent a disease, e.g., to prevent infection and/or the onset of disease.

Certain elements of the invention will be described herein. These elements and specific embodiments are listed together, however, it should be understood that they can be combined in any manner and in any number to generate additional embodiments. The variously described examples and preferred embodiments should not be construed to limit the invention to only the embodiments explicitly described. The description should be understood to support and include embodiments in which the explicitly described embodiments are combined with any number of the disclosed and/or preferred elements. Moreover, any arrangement and combination of all of the described elements in this disclosure should be considered as disclosed in the present description, unless indicated otherwise from the context. For example, if, in one embodiment, the number of A nucleotide of at least one R element of a modified poly(A) is less than 35, and if, in another embodiment, at least one S element of a modified poly(A) is G nucleotide, then the following embodiment is also an embodiment of the claimed invention: the number of A nucleotide of at least one R element of a modified poly(A) is less than 35 and at least one S element is G nucleotide.

### II. Polynucleotide

The invention provides a polynucleotide comprising a modified poly(A), wherein the number of nucleotides forming the modified poly(A) is at least 30, and the modified poly(A) comprises:
i) at least two R elements, the R element being defined as a polynucleotide consisting of A nucleotide; and
ii) at least one S element, wherein the S element:
   a) is a nucleotide other than A nucleotide, or
   b) consisting of at least two nucleotides, wherein the nucleotides at both ends are not A nucleotide;
wherein the total number of the S element is one less than the total number of the R element, the S element is connected to the R element, and each of the S elements is flanked at both sides by the R element.

In some embodiments, the number of the nucleotides of the modified poly(A) is less than 300. In some embodiments, the number of the nucleotides of the modified poly(A) is less than 100, less than 110, less than 120, less than 130, less than 150, less than 200, less than 250, or less than 280. Herein, "the number of the nucleotides of the modified poly(A) is less than 100, less than 110, less than 120, less than 130, less than 150, less than 200, less than 250, or less than 280" is the abbreviation of "the number of the nucleotides of the modified poly(A) is less than 100, the number of the nucleotides of the modified poly(A) is less than 110, the number of the nucleotides of the modified poly(A) is less than 120, the number of the nucleotides of the modified poly(A) is less than 130, the number of the nucleotides of the modified poly(A) is less than 150, the number of the nucleotides of the modified poly(A) is less than 200, the number of the nucleotides of the modified poly(A) is less than 250, or the number of the nucleotides of the modified poly(A) is less than 280 ", and the like. For example, "the number of the nucleotides of the modified poly(A) is less than 35, 36-39, 41-54, 56-59, 61-64, or greater than 65" is the abbreviation of "the number of the nucleotides of the modified poly(A) is less than 35, the number of the nucleotides of the modified poly(A) is 36-39, the number of the nucleotides of the modified poly(A) is 41-54, the number of the nucleotides of the modified poly(A) is 56-59, the number of the nucleotides of the modified poly(A) is 61-64 or the number of the nucleotides of the modified poly(A) is greater than 65".

In some embodiments, the number of the nucleotides of the modified poly(A) is less than 50, less than 60, less than 70, less than 80, or less than 90.

In some embodiments, the number of the nucleotides of the modified poly(A) is at least 30, 40, 50, or 60. In some embodiments, the number of the nucleotides of the modified poly(A) is at least 30, 40, 50, or 60, and less than 71, less than 80, less than 85, less than 90, less than 100, less than 110, less than 120, less than 130, or less than 140.

In some embodiments, the number of the nucleotides of the modified poly(A) is 30-70, 30-80, 30-90, 30-100, 30-110, 30-120, 30-130, 30-140, 40-70, 40-80, 40-90, 40-100, 40-110, 40-120, 40-130, 40-140, 50-70, 50-80, 50-90, 50-100, 50-110, 50-120, 50-130, 50-140, 60-70, 60-80, 60-90, 60-100, 60-110, 60-120, 60-130, 60-140, 70-80, 70-90, 70-100, 70-110, 70-120, 70-130, 70-140, 80-90, 80-100, 80-110, 80-120, 80-130, 80-140, 90-100, 90-110, 90-120, 90-130 or 90-140.

In some embodiments, the number of the nucleotides of the modified poly(A) is 31-70, 35-70, 41-70, 45-70, 51-70, 55-70, 61-70, 65-70, 30-75, 35-75, 40-75, 45-75, 50-75, 55-75, 60-75, 65-75, 70-75, 30-79, 35-79, 40-79, 45-79, 50-79, 55-79, 60-79, 65-79, 70-79, 75-79, 81-99, 85-99, 90-99, 95-99, 101-109, 105-109, 111-119, 115-119, 105-119, 109-119, 115-119, 122-125, 127-129 or 133-140.

In some embodiments, the number of the nucleotides of the modified poly(A) is 30-70, 73-75, 77-79, 81-89, 93-95, 97-99, 103-105, 107-109, 113-115, 117-119, 123-125, 127-129, 133-135, 137-139, 141-149, 151-199, 201-242, 244-245, 247-257, 259-269 or greater than 270.

In some embodiments, the number of the nucleotides of the modified poly(A) is 31-70, 35-70, 41-70, 45-70, 51-70, 55-70, 58-70, 61-70, 64-70, 65-70, 73-75, 77-79, 81-89, 93-95, 97-99, 103-105, 107-109, 113-115, 117-119, 123-125 or 127-129.

In some embodiments, the number of the nucleotides of the modified poly(A) is 41-70, 45-70, 51-70, 55-70, 58-70, or 61-70.

In some embodiments, the number of the nucleotides of the modified poly(A) is 73-75 or 77-79.

In some embodiments, the number of the nucleotides of the modified poly(A) is 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129 or 130.

In some embodiments, the number of the nucleotides of the modified poly(A) is 30-70, 40-70, 50-70, 60-70, 30-80, 40-80, 50-80, 60-80, or 70-80.

In some embodiments, the number of the nucleotides of the modified poly(A) is 31-70, 35-70, 41-70, 45-70, 51-70, 55-70, 61-70, 65-70, 30-75, 35-75, 40-75, 45-75, 50-75, 55-75, 60-75, 65-75, 70-75, 30-79, 35-79, 40-79, 45-79, 50-79, 55-79, 60-79, 65-79 or 70-79.

In some embodiments, the number of the nucleotides of the modified poly(A) is 30-70, 35-70, 40-70, 45-70, 51-70, 55-70, 61-70, 64-70, 65-70, 69-70, 73-75 or 77-79.

In some embodiments, the number of A nucleotide of at least one R element of the modified poly(A) is no more than 20, no more than 30, no more than 40, no more than 60, no more than 70, no more than 80, no more than 90, or no more than 120.

In some embodiments, the number of A nucleotide of at least one R element is at least 5, 8, or 10. In some embodiments, the number of A nucleotide of at least one R element is at least 5, 8, or 10 and the number of A nucleotide of at least one R element is less than 80, less than 85, less than 90, or less than 120.

In some embodiments, the number of A nucleotide of at least one R element of the modified poly(A) is 5-20, 8-20, 10-20, 15-20, 5-30, 8-30, 10-30, 20-30, 5-40, 8-40, 10-40, 20-40, 30-40, 5-50, 8-50, 10-50, 20-50, 30-50, 40-50, 5-60, 8-60, 10-60, 20-60, 30-60, 40-60, 50-60, 5-70, 8-70, 10-70, 20-70, 30-70, 40-70, 50-70, 60-70, 5-80, 8-80, 10-80, 20-80, 30-80, 40-80, 50-80, 60-80, 70-80, 5-90, 8-90, 10-90, 20-90, 30-90, 40-90, 50-90, 60-90, 70-90, 80-90, 5-100, 8-100, 10-100, 20-100, 30-100, 40-100, 50-100, 60-100, 70-100, 80-100, 90-100, 5-110, 8-110, 10-110, 20-110, 30-110, 40-110, 50-110, 60-110, 70-110, 80-110, 90-110 or 100-110.

In some embodiments, the number of A nucleotide of at least one R element of the modified poly(A) is 5-20, 8-20, 10-20, 15-20, 5-30, 8-30, 10-30, 20-30, 5-40, 8-40, 10-40, 20-40, 30-40, 5-50, 8-50, 10-50, 20-50, 30-50, 40-50, 5-60, 8-60, 10-60, 20-60, 30-60, 40-60 or 50-60.

In some embodiments, the number of A nucleotide of at least one R element of the modified poly(A) is less than 20, 21-29, 31-39, 41-44, 46-49, 51-54, 56-59, 61-64, 66-69, 71-79, 81-99, or greater than 100.

In some embodiments, the number of A nucleotide of at least one R element of the modified poly(A) is less than 20, 21-29, 31-34, 36-39, 41-44, 46-49, 51-54, 56-59, 61-64, 66-69, 71-79, 81-99, or greater than 100.

In some embodiments, the number of A nucleotide of at least one R element of the modified poly(A) is 5-19, 8-19, 10-19, 11-19, 13-19, 16-19, 5-18, 8-18, 10-18, 11-18, 13-18, 16-18, 21-29, 31-34, 36-39, 41-44, 46-49, 51-54, 56-59, 61-64, 66-69, 71-79, 81-99 or greater than 100.

In some embodiments, the number of A nucleotide of at least one R element of the modified poly(A) is 5-19, 8-19, 10-19, 11-19, 13-19, 16-19, 5-18, 8-18, 10-18, 11-18, 13-18, or 16-18.

In some embodiments, the number of A nucleotide of at least one R element of the modified poly(A) is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119 or 120.

In some embodiments, the number of A nucleotide of at least one R element of the modified poly(A) is 11, 12, 13, 14, 15, 16, 17, or 18.

In some embodiments, the number of A nucleotide of at least one R element of the modified poly(A) is less than 20. Preferably, the number of A nucleotide of at least one R element is 5-19. In some embodiments, the number of A nucleotide of at least one R element is 8-18 or 12-18.

In some embodiments, the number of A nucleotide of at least one R element of the modified poly(A) is 8-18, and the number of the nucleotides of the modified poly(A) is less than 79.

In some embodiments, the number of A nucleotide of at least two R elements of the modified poly(A) is less than 20. In some embodiments, the number of A nucleotide of at least one R element of the modified poly(A) is 8-18, and the number of A nucleotide of at least one R element is 12-18. Preferably, the modified poly(A) satisfies at least two of the followings: (1) the number of A nucleotide of at least one R element is 8-18, (2) the number of A nucleotide of at least one R element is 12-18, (3) the number of A nucleotide of at least one R element is 11, and (4) the number of A nucleotide of at least one R element is 18. In some embodiments, the number of A nucleotide of the R element at 5' end of the modified poly(A) is 8-18. Herein, the R element at 5' end of the modified poly(A) refers to the first R element in the 5' → 3' direction. In some embodiments, the number of A nucleotide of at least three, four, five, six or seven R elements of the modified poly(A) is less than 20.

In some embodiments, the number of A nucleotide of at least one R element of the modified poly(A) is 21-29. In some embodiments, the number of A nucleotide of at least one R element of the modified poly(A) is 25-29 or 22-29.

In some embodiments, the number of the nucleotides of the modified poly(A) is 104-106, the number of A nucleotide of at least one R element is 25-28, and the number of the nucleotides of at least one S element is 5-10. In other embodiments, the number of A nucleotide of at least one R element of the modified poly(A) is 22-29 and the number of A nucleotide of at least one R element is 60-69. Further, the modified poly(A) also comprises at least one R element of 10-20 A nucleotides. In an optional specific example, the number of A nucleotide of one R element of the modified poly(A) is 12, the number of A nucleotide of one R element is 28, and the number of A nucleotide of one R element is 60.

In some embodiments, the number of A nucleotide of at least one R element of the modified poly(A) is 31-34. Preferably, the modified poly(A) further comprises at least one R element of 8-12 A nucleotides and at least one R element of 55-65 A nucleotides. In an optional specific example, the number of A nucleotide of one R element of the modified poly(A) is 32, the number of A nucleotide of one R element is 8, and the number of A nucleotide of one R element is 60.

In some embodiments, the number of A nucleotide of at least one R element of the modified poly(A) is 36-39. Preferably, the number of A nucleotide of the R element at 3' end of the modified poly(A) is 36-39. Herein, the R element at 3' end of the modified poly(A) refers to the last R element in the 5' → 3' direction. Further, the modified poly(A) further comprises at least one R element of 30 A nucleotides. Further, the number of the nucleotides of the modified poly(A) is 70-77.

In some embodiments, the number of A nucleotide of at least one R element of the modified poly(A) is 56-59. Preferably, the number of A nucleotide of the R element at 3' end of the modified poly(A) is 56-59. Further, the modified poly(A) also comprises at least one R element of 8-35 A nucleotides, and at least one R element of 5-15 A nucleotides. In an optional specific example, the number of A nucleotide of one R element of the modified poly(A) is 30, the number of A nucleotide of one R element is 12, and the number of A nucleotide of one R element is 57.

In some embodiments, the number of A nucleotide of at least one R element of the modified poly(A) is 61-64. Preferably, the number of A nucleotide of the R element at 3' end of the modified poly(A) is 61-69 (for example, 61-64). More preferably, the modified poly(A) further comprises at least one R element of 8-35A nucleotides and at least one S element of less than 17 nucleotides. In an optional specific example, the number of A nucleotide of one R element of the modified poly(A) is 30, the number of A nucleotide of one R element is 61-64, and the nucleotide sequence of at least one S element is GCATATGACTAAAGAT (SEQ ID NO: 208).

In some embodiments, the number of A nucleotide of at least one R element of the modified poly(A) is 66-69 or 66-67. Preferably, the number of A nucleotide of the R element at 3' end of the modified poly(A) is 66-69 or 66-67. More preferably, the modified poly(A) also comprises at least one R element of 25-30 A nucleotides. Further, the modified poly(A) comprises at least one S element of 8-10 nucleotides.

In some embodiments, the number of A nucleotide of at least one R element of the modified poly(A) is 71-79 or 71-75. Preferably, the number of A nucleotide of the R element at 3' end of the modified poly(A) is 71-79 or 71-75. More preferably, the modified poly(A) further comprises at least one R element of 25-30 A nucleotides.

In some embodiments, the number of the nucleotides of the modified poly(A) is at least 30, and the number of A nucleotide of at least one R element is less than 20. Preferably, the number of A nucleotide of at least one R element is 8-18 or 12-18.

In some embodiments, the number of the nucleotides of the modified poly(A) is 30-70, 30-80, 30-90, 30-100, 30-110, 30-120, 30-130, 30-140, 40-70, 40-80, 40-90, 40-100, 40-110, 40-120, 40-130, 40-140, 50-70, 50-80, 50-90, 50-100, 50-110, 50-120, 50-130, 50-140, 60-70, 60-80, 60-90, 60-100, 60-110, 60-120, 60-130, 60-140, 70-80, 70-90, 70-100, 70-110, 70-120, 70-130, 70-140, 80-90, 80-100, 80-110, 80-120, 80-130, 80-140, 90-100, 90-110, 90-120, 90-130 or 90-140, the number of A nucleotide of at least one R element of the modified poly(A) is less than 20 (e.g., 5-19, 8-19, 10-19, 11-19, 13-19, 16-19, 5-18, 8-18, 10-18, 11-18, 13-18 or 16-18).

In some embodiments, the number of the nucleotides of the modified poly(A) is 40-70, 40-80, 40-90, 40-100, 40-110, 40-120, 40-130, 40-140, 50-70, 50-80, 50-90, 50-100, 50-110, 50-120, 50-130, 50-140, 60-70, 60-80, 60-90, 60-100, 60-110, 60-120, 60-130, 60-140, 70-80, 70-90, 70-100, 70-110, 70-120, 70-130, 70-140, 80-90, 80-100, 80-110, 80-120, 80-130, 80-140, 90-100, 90-110, 90-120, 90-130 or 90-140, the number of A nucleotide of at least one R element of the modified poly(A) is less than 20 (e.g., 5-19, 8-19, 10-19, 11-19, 13-19, 16-19, 5-18, 8-18, 10-18, 11-18, 13-18 or 16-18).

In some embodiments, the number of the nucleotides of the modified poly(A) is 40-70, 40-80, 40-90, 40-100, 40-110, 40-120, 40-130, 40-140, 50-70, 50-80, 50-90, 50-100, 50-110, 50-120, 50-130, 50-140, 60-70, 60-80, 60-90, 60-100, 60-110, 60-120, 60-130, 60-140, 70-80, 70-90, 70-100, 70-110, 70-120, 70-130, 70-140, 80-90, 80-100, 80-110, 80-120, 80-130, 80-140, 90-100, 90-110, 90-120, 90-130 or 90-140, the number of A nucleotide of at least one R element of the modified poly(A) is 5-19, 8-19, 10-19, 11-19, 13-19, 16-19, 5-18, 8-18, 10-18, 11-18, 13-18 or 16-18, the modified poly(A) also satisfies at least one of the followings: (1) at least one S element consists of 1, 3-19, 3-16, 3-12, or 3-10 nucleotides (e.g., one of G, C, T, GTGT, GCAAATGACT, GGC, GGT, GCG, GAG, GGCT, GAGG, GCTGAT, GGCAAT, GTGTAAAG, GCATATGACT, GCTGAT, GAAAGTGT, GCATATGAAT, GCATATAAAT, GCTGATAAAG, GAAAGCTGAT, GCTGATAAAG, CAAAAGCTGAT, GAAAGCTGAT, GCATATGACTAAAGAT, GCAAATGACTAG, CAAAAGCATATGACT, GAAAAGTGT and GAAAG); and (2) the number of the S element is 1, 2, 3, or 4.

In some embodiments, the number of A nucleotide of at least one R element of the modified poly(A) is 8-18 and the number of the nucleotides of the modified poly(A) is less than 79. In some embodiments, the number of A nucleotide of at least one R element of the modified poly(A) is 16-18 and the number of the nucleotides of the modified poly(A) is 51-79.

In some embodiments, the modified poly(A) satisfies at least one of the followings: (1) the number of A nucleotide of one R element is 8-18, (2) the number of A nucleotide of one R element is 12-18, (3) the number of A nucleotide of one R element is 11, and (4) the number of A nucleotide of one R element is 18.

In some embodiments, the number of the nucleotides of the modified poly(A) is at least 30 (e.g., 30-70, 51-70, 58-70, 51-110, or 58-120), and the number of A nucleotide of at least one R element is less than 20 (e.g., 8-18, 12-18, or 16-18), the modified poly(A) also satisfies at least one of the followings: (1) the number of A nucleotide of at least one R element is 10-45 (e.g., 18, 22, 28, 29, 32, 38, or 42); (2) the number of A nucleotide of at least one R element is 15-75 (e.g., 18, 28, 30, 50, 57, 60, or 70); (3) the number of A nucleotide of at least one R element is 10-35 (e.g., 12, 18, 18, or 30); (4) at least one S element consists of 1, 3-19, 3-16, 3-12 or 3-10 nucleotides (e.g., one of G, C, T, GTGT, GCAAATGACT, GGC, GGT, GCG, GAG, GGCT, GAGG, GCTGAT, GGCAAT, GTGTAAAG, GCATATGACT, GCTGAT, GAAAGTGT, GCATATGAAT, GCATATAAAT, GCTGATAAAG, GAAAGCTGAT, GCTGATAAAG, CAAAAGCTGAT, GAAAGCTGAT, GCATATGACTAAAGAT, GCAAATGACTAG, CAAAAGCATATGACT, GAAAAGTGT and GAAAG); and (5) the number of the S element is 1, 2, 3, or 4.

In some embodiments, the number of the nucleotides of the modified poly(A) is at least 30 (e.g., 30-70, 51-70, 58-70, 51-110, or 58-120), and the number of A nucleotide of at least one R element is less than 20, 8-18 (e.g., 8, 11, 12, 16, or 18), or 12-18, the modified poly(A) also satisfies at least one of the followings: (1) the number of A nucleotide of at least one R element is 13-42 (e.g., 18, 22, 28, 29, 32, 38, or 42); (2) the number of A nucleotide of at least one R element is 18-70 (e.g., 18, 28, 30, 50, 57, 60, or 70); (3) the number of A nucleotide of at least one R element is 12-30 (e.g., 12, 18, 18, or 30); (4) at least one S element consists of 1 or 3-10 nucleotides (e.g., one of G, C, T, GCAAATGACT, GGC, and GAAAG); and (5) the number of S element is 1, 2, or 3.

In some embodiments, the number of the nucleotides of the modified poly(A) is at least 30, and the number of A nucleotide of at least two R elements is less than 20. Further, the number of A nucleotide of at least one R element of the modified poly(A) is 8-18 and the number of A nucleotide of at least one R element is 12-18. Preferably, the modified poly(A) further satisfies at least two of the followings: (1) the number of A nucleotide of one R element is 8-18, (2) the number of A nucleotide of one R element is 12-18, (3) the number of A nucleotide of one R element is 11, and (4) the number of A nucleotide of one R element is 18. Further, the number of A nucleotide of the R element at 5' end of the modified poly(A) is 8-18.

In some embodiments, the number of the nucleotides of the modified poly(A) is at least 30, and the number of A nucleotide of at least three R elements is less than 20. In some embodiments, the number of the nucleotides of the modified poly(A) is at least 30, and the number of A nucleotide of at least three R elements is 8-18 or 12-18. In some embodiments, the number of the nucleotides of the modified poly(A) is at least 30, and the modified poly(A) further satisfies at least two of the followings: (1) the number of A nucleotide of one R element is 8-18, (2) the number of A nucleotide of one R element is 12-18, (3) the number of A nucleotide of one R element is 11, and (4) the number of A nucleotide of one R element is 18. Further, the number of A nucleotide of the R element at 5' end of the modified poly(A) is 8-18.

In some embodiments, the number of the nucleotides of the modified poly(A) is 30-70, and the modified poly(A) further satisfies at least one of the followings: (1) the number of A nucleotide of at least one R element is 15-20, (2) the number of A nucleotide of at least one R element is 10-18, (3) the number of A nucleotide of at least one R element is 5-12, and (4) the number of A nucleotide of at least one R element is 15-40.

In some embodiments, the number of the nucleotides of the modified poly(A) is 30-70, and the modified poly(A) further satisfies at least one of the followings: (1) the number of A nucleotide of at least one R element is 15-30, (2) the number of A nucleotide of at least one R element is 10-18, (3) the number of A nucleotide of at least one R element is 5-12, and (4) the number of A nucleotide of at least one R element is 15-40.

In some embodiments, the number of the nucleotides of the modified poly(A) is 51-70, and the modified poly(A) further satisfies at least one of the followings: (1) the number of A nucleotide of at least one R element is 16-30, (2) the number of A nucleotide of at least one R element is 13-18, (3) the number of A nucleotide of at least one R element is 11, and (4) the number of A nucleotide of at least one R element is 18-37. In some embodiments, the number of the nucleotides of the modified poly(A) is 58-70, and the modified poly(A) further satisfies at least one of the followings: (1) the number of A nucleotide of at least one R element is 16-18, (2) the number of A nucleotide of at least one R element is 13-18, (3) the number of A nucleotide of at least one R element is 11, and (4) the number of A nucleotide of at least one R element is 18-37. Further, the number of A nucleotide of the R element at 3' end of the modified poly(A) is 18-37.

In some embodiments, the number of the nucleotides of the modified poly(A) is 30-70 (e.g., 41-70, 45-70, 51-70, 55-70, 58-70, or 61-70), the modified poly(A) also satisfies at least one of the followings: (1) the number of A nucleotide of at least one R element of the modified poly(A) is 5-20, 8-20, 10-20, 15-20, 5-30, 8-30, 10-30, 20-30, 5-40, 8-40, 10-40, 20-40, 30-40, 5-50, 8-50, 10-50, 20-50, 30-50, 40-50, 5-60, 8-60, 10-60, 20-60, 30-60, 40-60 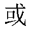 50-60; (2) at least one S element consists of 1, 3-19, 3-16, 3-12, or 3-10 nucleotides (e.g., one of G, C, T, GTGT, GCAAATGACT, GGC, GGT, GCG, GAG, GGCT, GAGG, GCTGAT, GGCAAT, GTGTAAAG, GCATATGACT, GCTGAT, GAAAGTGT, GCATATGAAT, GCATATAAAT, GCTGATAAAG, GAAAGCTGAT, GCTGATAAAG, CAAAAGCTGAT, GAAAGCTGAT, GCATATGACTAAAGAT, GCAAATGACTAG, CAAAAGCATATGACT, GAAAAGTGT and GAAAG); and (3) the number of the S element is 1, 2, 3, or 4.

In some embodiments, the number of the nucleotides of the modified poly(A) is 30-70 (e.g., 41-70, 45-70, 51-70, 55-70, 58-70, or 61-70), the number of A nucleotide of at least one R element of the modified poly(A) is 5-20, 8-20, 10-20, 15-20, 5-30, 8-30, 10-30, 20-30, 5-40, 8-40, 10-40, 20-40, 30-40, 5-50, 8-50, 10-50, 20-50, 30-50, 40-50, 5-60, 8-60, 10-60, 20-60, 30-60, 40-60 or 50-60.

In some embodiments, the number of the nucleotides of the modified poly(A) is 71-79, and the modified poly(A) further satisfies at least one of the followings: (1) the number of A nucleotide of at least one R element is less than 35, 36-39, 41-54, 56-59, 61-64, or more than 65; and (2) the number of A nucleotide of at least one R element is not any integer of 35-65. In some embodiments, the number of the nucleotides of the modified poly(A) is 71-79, 73-75, 73-79 or 77-79, and the number of A nucleotide of at least one R element is less than 35. In some embodiments, the number of the nucleotides of the modified poly(A) is 71-79, 73-75, 73-79 or 77-79, and the number of A nucleotide of at least one R element is 36-39. In some embodiments, the number of the nucleotides of the modified poly(A) is 71-79, wherein the number of A nucleotide of one R element is 30-34, and the number of A nucleotide of one R element is 36-39. Further, the number of A nucleotide of the R element at 5' end of the modified poly(A) is 30-34, and the number of A nucleotide of the R element at 3' end is 36-39.

In some embodiments, the number of the nucleotides of the modified poly(A) is 73-75 or 77-79. In some embodiments, the number of the nucleotides of the modified poly(A) is 77-79 and the number of A nucleotide of at least one R element is more than 30.

In some embodiments, the number of the nucleotides of the modified poly(A) is 81-89, 93-95, 97-99, 103-105, 107-109, 113-115, 117-119, 123-125, 127-129, 133-135, 137-139, 141-149, 151-199, 201-242, 244-245, 247-257, 259-269 or more than 270.

In some embodiments, the number of the nucleotides of the modified poly(A) is 103-105, and the number of A nucleotide of at least one R element is 25-55 and/or the number of A nucleotide of at least one R element is 45-75.

In some embodiments, the number of the nucleotides of the modified poly(A) is 103-105, wherein the number of A nucleotide of one R element is 70 or more. Further, the modified poly(A) further satisfies that the number of A nucleotide of the R element at 3' end is 70 or more, and further, the modified poly(A) further satisfies that the nucleotide sequence of at least one S element is not GGT or GGGC.

In some embodiments, the number of the nucleotides of the modified poly(A) is less than 80, 81-89, 91-99, 101-109, 111-119, 121-129, 131-149, 151-199, or greater than 200. Further, the modified poly(A) further satisfies at least one of the followings: (1) the number of A nucleotide of at least one R element is less than 35, 36-39, 41-54, 56-59, 61-64 or greater than 65; and (2) the number of A nucleotide of at least one R element is not any integer of 35-65.

In some embodiments, the number of the nucleotides of the modified poly(A) is less than 80 and the number of A nucleotide of at least one R element is less than 35. Preferably, the number of A nucleotide of at least one R element is 11-35. Further, the number of A nucleotide of at least one R element of the modified poly(A) is 18-37, and the number of the nucleotides of at least one R element is 12-30 and/or the number of A nucleotide of at least one R element is 13 to 19. Preferably, the number of the nucleotides of the modified poly(A) is 51-79, the number of the nucleotides of at least one R element is 18-30, and the modified poly(A) further satisfies that the number of A nucleotide of at least one R element is 15 to 18 and/or the number of A nucleotide of at least one R element is 18-37.

In some embodiments, the number of the nucleotides of the modified poly(A) is less than 80 (e.g., 31-70, 35-71, 41-70, 45-70, 51-70, 55-70, 58-70, 61-70, 73-75, or 77-79), and the number of A nucleotide of at least one R element is less than 35 (e.g., 5-20, 8-20, 10-20, 15-20, 5-30, 8-30, 10-30, 20-30, or 31-34).

In some embodiments, the number of the nucleotides of the modified poly(A) is less than 80 (e.g., 31-70, 35-71, 41-70, 45-70, 51-70, 55-70, 58-70, 61-70, 73-75, or 77-79), and the number of A nucleotide of at least one R element is less than 20 (e.g., 5-19, 8-19, 10-19, 11-19, 13-19, 16-19, 5-18, 8-18, 10-18, 11-18, 13-18, or 16-18).

In some embodiments, the number of the nucleotides of the modified poly(A) is less than 80 (e.g., 31-70, 35-71, 41-70, 45-70, 51-70, 55-70, 58-70, 61-70, 73-75, or 77-79), the number of A nucleotide of at least one R element is less than 35 (e.g., 5-20, 8-20, 10-20, 15-20, 5-30, 8-30, 10-30, 20-30, or 31-34); the modified poly(A) also satisfies one or more of the followings: (1) the number of A nucleotide of at least one R element is 12-45 (e.g., 12, 13, 18, 22, 28, 29, 30, 32, 38, or 42); (2) the number of A nucleotide of at least one R element is less than 20 (e.g., 8, 10, or 11); (3) the number of A nucleotide of at least one R element is 15-75 (e.g., 18, 28, 30, or 70); (4) the number of A nucleotide of at least one R element is 8-30 (e.g., 8, 12, 18, 29, or 30); (5) the number of S element is 1, 2, 3, or 4; and (6) at least one S element consists of 1, 3-19, 3-16, 3-12, or 3-10 nucleotides (e.g., one of G, C, T, GTGT, GCAAATGACT, GGC, GGT, GCG, GAG, GGCT, GAGG, GCTGAT, GGCAAT, GTGTAAAG, GCATATGACT, GCTGAT, GAAAGTGT, GCATATGAAT, GCATATAAAT, GCTGATAAAG, GAAAGCTGAT, GCTGATAAAG, CAAAAGCTGAT, GAAAGCTGAT, GCATATGACTAAAGAT, GCAAATGACTAG, CAAAAGCATATGACT, GAAAAGTGT and GAAAG).

In some embodiments, the number of the nucleotides of the modified poly(A) is less than 80 (e.g., 41-70, 45-70, 51-70, 55-70, 58-70, 61-70, 73-75, or 77-79), the number of A nucleotide of at least one R element is less than 35 (e.g., 5-20, 8-20, 10-20, 15-20, 5-30, 8-30, 10-30, 20-30 or 31-34) or less than 20 (e.g., 5-19, 8-19, 10-19, 11-19, 13-19, 16-19, 5-18, 8-18, 10-18, 11-18, 13-18 or 16-18); the modified poly(A) also satisfies one or more of the followings: (1) the number of A nucleotide of at least one R element is 12-42 (e.g., 12, 13, 18, 22, 28, 29, 30, 32, 38 or 42); (2) the number of A nucleotide of at least one R element is less than 20 (e.g., 8, 10, or 11); (3) the number of A nucleotide of at least one R element is 18-71 (e.g., 18, 28, 30, or 70); (4) the number of A nucleotide of at least one R element is 8-30 (e.g., 8, 12, 18, 29, or 30); (5) the number of S element is 1, 2, or 3; and (6) at least one S element consists of 1, 3-19, 3-16, 3-12, or 3-10 nucleotides (e.g., one of G, C, T, GCTGAT, GGCT, GGCAAT, GTGT, GCAAATGACT, GGC, GAAAAGTGT and GAAAG).

In some embodiments, the number of the nucleotides of the modified poly(A) is 91-99 or 93-96, and the number of A nucleotide of at least one R element is less than 35. In some embodiments, the number of the nucleotides of the modified poly(A) is 91-99 or 93-96, the number of A nucleotide of at least one R is 30-34, and the number of the nucleotides of at least one S element is greater than 3.

In some embodiments, the number of the nucleotides of the modified poly(A) is 101-109 and the number of A nucleotide of at least one R element is less than 35. In some embodiments, the number of the nucleotides of the modified poly(A) is 101-109, the number of A nucleotide of at least one R element is 8-12, and the modified poly(A) further comprises at least one R element of 60-69 or less than 20 A nucleotides.

In some embodiments, the number of the nucleotides of the modified poly(A) is 111-119 or 111-115, and the number of A nucleotide of at least one R element is less than 35. Preferably, the number of A nucleotide of at least one R element is 15 to 34. Further, the modified poly(A) further comprises at least one R element of more than 65 A nucleotides.

In some embodiments, the number of the nucleotides of the modified poly(A) is less than 80 or 101-109, and the number of A nucleotide of at least one R element is 36-39. In some embodiments, the number of the nucleotides of the modified poly(A) is 70-77 and the number of A nucleotide of at least one R element is 36-39. In some embodiments, the number of the nucleotides of the modified poly(A) is 81-89 or 101-109, and the number of A nucleotide of at least one R element is 41-54. In some embodiments, the number of the nucleotides of the modified poly(A) is 103-106, the number of A nucleotide of at least one R element is 45-50, and the number of the nucleotides of at least one S element is greater than 5. Further, the modified poly(A) further includes at least one R element of 8-55 A nucleotides and/or at least one R element of 42-38 A nucleotides. In some embodiments, the number of the nucleotides of the modified poly(A) is 101-109 and the number of A nucleotide of at least one R element is greater than 65. In some embodiments, the number of the nucleotides of the modified poly(A) is 102-106 and the number of A nucleotide of at least one R element is 66 to 70. In some embodiments, the number of the nucleotides of the modified poly(A) is 104-106, the number of A nucleotide of at least one R element is 66 to 70, the number of A nucleotide of at least one R element is 30 and the number of the nucleotides of at least one S element is 6-9.

In other embodiments, the number of the nucleotides of the modified poly(A) is 102-106, the number of A nucleotide of at least one R element is 66 to 70, the number of A nucleotide of at least one R element is 25, and the number of the nucleotides of at least one S element is 9-11 and is not GAAAAGCTGAT (SEQ ID NO: 209).

In some embodiments, the number of A nucleotide of at least one R element of the modified poly(A) is less than 20, 21-29, 31-39, 41-44, 46-49, 51-54, 56 to 59, 61-69, 71-79, 81-99, or more than 100, and/or the number of A nucleotide of at least one R element is not any integer of 35-65. Further, the modified poly(A) further satisfies at least one of the followings: a. the number of the nucleotides of the modified poly(A) is less than 71, 73-75, 77-79, 81-90, 93-95, 97-99, 103-105, 107-109, 113-115, 117-119, 123-125, 127-129, 133-135, 137-139, 141-242, 244-245, 247-257, 259-269, or more than 270; and b. the number of A nucleotide of at least one R element is not any integer of 35-65.

In some embodiments, the number of A nucleotide of at least one R element of the modified poly(A) is less than 20 and the number of the nucleotides of the modified poly(A) is less than 71. In some embodiments, the number of A nucleotide of at least one R element of the modified poly(A) is 13-18, and the number of the nucleotides of the modified poly(A) is 51-71. Preferably, the modified poly(A) further satisfies at least one of the followings: the number of A nucleotide of at least one R element is 16-30, the number of A nucleotide of at least one R element is 16-18, and the number of the nucleotides of the modified poly(A) is 63-70. Preferably, the modified poly(A) further comprises at least one R element of 11 A nucleotides.

In some embodiments, the number of A nucleotide of at least one R element of the modified poly(A) is less than 20 (e.g., 5-19, 8-19, 10-19, 11-19, 13-19, 16-19, 5-18, 8-18, 10-18, 11-18, 13-18, or 16-18) and the number of the nucleotides of the modified poly(A) is less than 71 (e.g., 41-70, 45-70, 51-70, 55-70, 58-70, or 61-70).

In some embodiments, the number of A nucleotide of at least one R element of the modified poly(A) is less than 20 (e.g., 5-19, 8-19, 10-19, 11-19, 13-19, 16-19, 5-18, 8-18, 10-18, 11-18, 13-18, or 16-18) and the number of the nucleotides of the modified poly(A) is less than 71 (e.g., 41-70, 45-70, 51-70, 55-70, 58-70, or 61-70), the modified poly(A) also satisfies one or more of the followings: (1) the number of A nucleotide of at least one R element is 16-30; (2) the number of A nucleotide of at least one R element is 8-18, 13-18, or 16-18; (3) the number of S element is 1, 2, 3, or 4; and (4) at least one S element consists of 1, 3-19, 3-16, 3-12, or 3-10 nucleotides (e.g., one of G, C, T, GTGT, GCAAATGACT, GGC, GGT, GCG, GAG, GGCT, GAGG, GCTGAT, GGCAAT, GTGTAAAG, GCATATGACT, GCTGAT, GAAAGTGT, GCATATGAAT, GCATATAAAT, GCTGATAAAG, GAAAGCTGAT, GCTGATAAAG, CAAAAGCTGAT, GAAAGCTGAT, GCATATGACTAAAGAT, GCAAATGACTAG, CAAAAGCATATGACT, GAAAAGTGTand GAAAG).

In some embodiments, the number of A nucleotide of at least one R element of the modified poly(A) is less than 20 (e.g., 5-19, 8-19, 10-19, 11-19, 13-19, 16-19, 5-18, 8-18, 10-18, 11-18, 13-18, or 16-18) and the number of the nucleotides of the modified poly(A) is less than 71 (e.g., 41-70, 45-70, 51-70, 55-70, 58-70, or 61-70), the modified poly(A) also satisfies one or more of the followings: (1) the number of A nucleotide of at least one R element is 16-30; (2) the number of A nucleotide of at least one R element is 13-18; (3) the number of S element is 1, 2, or 3; and (4) at least one S element consists of 1 or 3-10 nucleotides (e.g., one of G, C, T, GCTGAT, GGCT, GGCAAT, GTGT, GCAAATGACT, and GGC).

In some embodiments, the number of A nucleotide of at least one R element of the modified poly(A) is 31-39 and the number of the nucleotides of the modified poly(A) is less than 71 or 77-79. Preferably, the number of the nucleotides of the modified poly(A) is 61-70 or 77-79. Further, the number of A nucleotide of the R element at 3' end of the modified poly(A) is 31-39. Preferably, the modified poly(A) further comprises at least one R element of 25-30 A nucleotides.

In some embodiments, the number of A nucleotide of at least one R element of the modified poly(A) is 31-39 and the number of the nucleotides of the modified poly(A) is 77-79, the modified poly(A) also satisfies one or more of the followings: (1) the number of A nucleotide of at least one R element is less than 35 (e.g., 25, 30 or 35); (2) the number of S element is 1, 2, 3, or 4; and (3) at least one S element consists of 1, 3-19, 3-16, 3-12, or 3-10 nucleotides (e.g., one of G, C, T, GTGT, GCAAATGACT, GGC, GGT, GCG, GAG, GGCT, GAGG, GCTGAT, GGCAAT, GTGTAAAG, GCATATGACT, GCTGAT, GAAAGTGT, GCATATGAAT, GCATATAAAT, GCTGATAAAG, GAAAGCTGAT, GCTGATAAAG, CAAAAGCTGAT, GAAAGCTGAT, GCATATGACTAAAGAT, GCAAATGACTAG, CAAAAGCATATGACT, GAAAAGTGT and GAAAG).

In some embodiments, the number of A nucleotide of at least one R element of the modified poly(A) is 31-39 and the number of the nucleotides of the modified poly(A) is 77-79, and the modified poly(A) further satisfies: (1) the number of A nucleotide of at least one R element is 25-35 (e.g., 30), (2) the number of S element is 1, 2, or 3; and (3) at least one S element consists of 1 or 3-10 nucleotides (e.g., one of GTGT, GCAAATGACT, and GAAAG).

In some embodiments, the number of A nucleotide of at least one R element of the modified poly(A) is 21-29 and the number of the nucleotides of the modified poly(A) is 103-105. Preferably, the modified poly(A) further comprises at least one R element of more than 66 A nucleotides and/or at least one S element of less than 13 nucleotides. Preferably, the modified poly(A) further comprises at least one S element of 5-9 nucleotides.

In some embodiments, the number of A nucleotide of at least one R element of the modified poly(A) is 61-69, and the number of the nucleotides of the modified poly(A) is 103-105 or 103-104. Preferably, the modified poly(A) further comprises at least one R element of 25-30 A nucleotides. In some embodiments, the number of A nucleotide of at least one R element of the modified poly(A) is 66-67, the number of A nucleotide of at least one R element is 30, and the number of the nucleotides of the modified poly(A) is 103-104. In some embodiments, the modified poly(A) comprises at least three R elements. Further, the modified poly(A) further satisfies at least one of the followings: (1) the number of A nucleotide of at least one R element is 8-20 or 12-18; or the number of A nucleotide of at least one R element is 8-20, and the number of A nucleotide of at least one R element is 12-18; or the number of A nucleotide of at least one R element is 12-30, the number of A nucleotide of at least one R element is 12-18 and the number of A nucleotide of at least one R element is 18-37; or the number of A nucleotide of at least one R element is 18-30, the number of A nucleotide of at least one R element is 12-18 and the number of A nucleotide of at least one R element is 18-37; and (2) at least one S element consists of one nucleotide that is not A nucleotide or consists of 2-23 nucleotides and the nucleotides at both ends are not A nucleotide, and/or at least one S element consists of one nucleotide that is not A nucleotide or consists of 2-5 nucleotides and the nucleotides at both ends are not A nucleotide. Further, at least one S element consists of one nucleotide that is not A nucleotide or consists of 3-19 nucleotides and the nucleotides at both ends are not A nucleotide, and/or at least one S element consists of one nucleotide that is not A nucleotide or consists of 2-5 nucleotides and the nucleotides at both ends are not A nucleotide. Further, the S element consists of one nucleotide that is not A nucleotide or consists of 3-16 nucleotides and the nucleotides at both ends are not A nucleotide, and/or at least one S element consists of one nucleotide that is not A nucleotide or consists of 2-5 nucleotides and the nucleotides at both ends are not A nucleotide. Further, the S element consists of one nucleotide that is not A nucleotide or consists of 3-10 nucleotides and the nucleotides at both ends are not A nucleotide, and/or at least one S element consists of one nucleotide that is not A nucleotide or consists of 3 nucleotides and the nucleotides at both ends are not A nucleotide.

In some embodiments, the modified poly(A) comprises at least four R elements. Further, the modified poly(A) further satisfies at least one of the followings: (i) at least one S element consists of one nucleotide that is not A nucleotide; and (ii) the number of A nucleotide of at least one R element is at most 20, further, the number of A nucleotide of at least one R element is 15-20; and/or the number of A nucleotide of at least one R element is 8-11.

In some embodiments, at least one R element consists of 11-50 A nucleotides. In some embodiments, at least one R element consists of 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, or 45 A nucleotides.

In some embodiments, the number of the nucleotides of at least one S element is at least 1, 2, 5, 10, 15, or 20. For example, the number of the nucleotides of at least one S element is 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, or 70.

In some embodiments, the number of the nucleotides of at least one S element is at least 1.

In some embodiments, the number of the nucleotides of at least one S element is no more than 15, no more than 20, no more than 30, no more than 35, or no more than 40. In some embodiments, the number of the nucleotides of at least one S element is 2-10, 2-15, 2-20, 2-25, 3-10, 3-15, 3-20, or 3-25.

In some embodiments, the number of the nucleotides of at least one S element is 1. In some embodiments, the number of S element is 1, at least 2, at least 5, at least 8, or at least 10. In some embodiments, the number of S element is 1, 2, 3, or 4.

In some embodiments, the S element is one, the S element is one of G, C, T, GTGT, GCAAATGACT (i.e. SEQ ID NO: 207), GGC, GGT, GCG, GAG, GGCT, GAGG, GCTGAT, GGCAAT, GTGTAAAG, GCATATGACT, GCTGAT, GAAAGTGT, GCATATGAAT, GCATATAAAT, GCTGATAAAG, GAAAGCTGAT, GCTGATAAAG, CAAAAGCTGAT, GAAAGCTGAT, GCATATGACTAAAGAT, GCAAATGACTAG, CAAAAGCATATGACT, GAAAAGTGT and GAAAG.

In some embodiments, there are a plurality of S elements, each S element independently being one of G, C, T, GTGT, GCAAATGACT (i.e. SEQ ID NO: 207), GGC, GGT, GCG, GAG, GGCT, GAGG, GCTGAT, GGCAAT, GTGTAAAG, GCATATGACT, GCTGAT, GAAAGTGT, GCATATGAAT, GCATATAAAT, GCTGATAAAG, GAAAGCTGAT, GCTGATAAAG, CAAAAGCTGAT, GAAAGCTGAT, GCATATGACTAAAGAT, GCAAATGACTAG, CAAAAGCATATGACT, GAAAAGTGT and GAAAG.

In some embodiments, at least one S element satisfies at least one of the followings: (a) consisting of 2-23 nucleotides; further, 3-19, 3-16, 3-12, or 3-10 nucleotides; (b) containing up to 10 A nucleotides; further, up to 4 or 8 A nucleotides; and/or up to 4 consecutive A nucleotides; (c) having a GC content of 20%-100%; further, a GC content of 40%-100%; further, a GC content of 42%-100%, 50%-100%, 60%-100%, or 70%-100%; (d) comprising one of G, C, T, GTGT, GCAAATGACT (i.e. SEQ ID NO: 207), GGC, GAAAAGTGT, and GAAAG.

In some embodiments, there are at least two S elements, each of which independently satisfies at least one of the followings: (a) consisting of 2-23 nucleotides; further, 3-19, 3-16, 3-12, or 3-10 nucleotides; (b) containing up to 10 A nucleotides; further, up to 4 or 8 A nucleotides; and/or up to 4 consecutive A nucleotides; (c) having a GC content of 20% to 100%; further, a GC content of 40% to 100%; further, a GC content of 42%-100%, 50%-100%, 60%-100%, or 70%-100%; and (d) comprising one of G, C, T, GTGT, GCAAATGACT (i.e., SEQ ID NO: 207), GGC, GAAAAGTGT, and GAAAG.

In some embodiments, the S element consists of 3-19 nucleotides, e.g., consisting of 3, 4, 5, 6, 7, 8, 9, 10, 11, 12, 13, 14, 15, 16, 17, 18 or 19 nucleotides. In some embodiments, the S element contains up to 10 A nucleotides.

In some embodiments, the poly(A) comprises up to 79 nucleotides. In some embodiments, the poly(A) comprises at least three R elements, wherein at least one R element comprises up to 20 A nucleotides.

In some embodiments, the modified poly(A) comprises a nucleotide sequence having at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one nucleotide sequence of SEQ ID NOs: 2-100 and 212-247.

In some embodiments, the modified poly(A) comprises a nucleotide sequence having at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one nucleotide sequence of SEQ ID NOs: 2-100 and 212-247, wherein the R element remains unchanged.

In some embodiments, the nucleotide sequence of the modified poly(A) has at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one nucleotide sequence of SEQ ID NOs: 2-100 and 212-247, wherein the R element remains unchanged.

In some embodiments, the modified poly(A) comprises any one of the nucleotide sequences of SEQ ID NOs: 2-100 and 212-247.

In an optional specific example, the nucleotide sequence of the modified poly(A) is se forth in any one of SEQ ID NOs: 2-100 and 212-247.

In some embodiments, the polynucleotide of the invention further comprises at least one of 5'-UTR, a nucleic acid encoding a polypeptide or protein of interest and 3'-UTR that are located upstream of said modified poly(A). In an optional specific example, the polynucleotide of the invention further comprises a nucleic acid encoding a polypeptide or protein of interest located upstream of said modified poly(A). In some embodiments, the polynucleotides of the invention further comprise 5'-UTR and a nucleic acid encoding a polypeptide or protein of interest that are located upstream of said modified poly(A). Alternatively, the polynucleotide of the invention also comprises a nucleic acid encoding a polypeptide or protein of interest and 3'-UTR that are located upstream of said modified poly(A). In some embodiments, the polynucleotide of the invention further comprises 5'-UTR, a nucleic acid encoding a polypeptide or protein of interest, and 3'-UTR that are located upstream of said modified poly(A).

It should be noted that the 5'-UTR, the nucleic acid encoding a polypeptide or protein of interest, and the 3'-UTR are not particularly limited. In some embodiments, the 5'-UTR comprises the nucleotide sequence set forth in SEQ ID NO: 202, and/or, the 3'-UTR comprises the nucleotide sequence set forth in SEQ ID NO: 203. In some embodiments, the nucleotide sequence of 5'-UTR is set forth in SEQ ID NO: 202, and/or, the nucleotide sequence of 3'-UTR is set forth in SEQ ID NO: 203.

In some embodiments, the polynucleotide of the invention comprises one or more modified nucleotides. In some embodiments, the modified nucleotide in the polynucleotide has a base modification and/or a sugar modification. In some embodiments, the modified nucleotide in the polynucleotide also has a phosphate group modification. In some embodiments, the modified nucleotide in the polynucleotide has a base modification.

The polynucleotide of the invention comprises one or more modified nucleosides. In some embodiments, the polynucleotide of the invention comprises at least one of modified uridine, modified cytidine, modified adenosine, modified guanosine, and modified thymidine. In some embodiments, the polynucleotide of the invention comprises at least one of modified uridine, modified cytidine, modified adenosine, and modified guanosine.

In some embodiments, the modified nucleoside in the polynucleotide is a modified uridine. In some embodiments, 0.1%-100% of uridines in the polynucleotide of the invention are modified. For example, at least 0.1%, at least 0.5%, at least 1%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 88%, at least 90%, at least 92%, at least 95%, at least 97%, at least 99% or 100% uridines are modified. In some embodiments, 80%-100% of uridines are modified. In some embodiments, 100% of uridines are modified. Exemplary modified uridine may refer to the description about the RNA below, which will not be repeated here.

In some embodiments, the modified uridines in the polynucleotide are of the same kind. For example, the polynucleotide comprises a plurality of modified uridines, all of which are N1-methylpseudouridine. In other embodiments, the modified uridines in the polynucleotide are of various kinds. For example, the polynucleotide comprises a plurality of modified uridines that are at least two selected from the exemplary modified uridines (e.g. pseudouridine and N1-methylpseudouridine).

In some embodiments, the modified nucleoside in the polynucleotide is a modified cytidine. In some embodiments, 0.1%-100% of cytidines in the polynucleotide of the invention are modified. For example, at least 0.1%, at least 0.5%, at least 1%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 88%, at least 90%, at least 92%, at least 95%, at least 97%, at least 99% or 100% of cytidines are modified. In some embodiments, 80%-100% of cytidines are modified. In some embodiments, 100% of cytidines are modified. Exemplary modified cytidine may refer to the description about the RNA below, which will not be repeated here.

In some embodiments, the modified cytidines in the polynucleotide are of the same kind. For example, the polynucleotide comprises a plurality of modified cytidines, all of which are 5-aza-cytidine. In other embodiments, the modified cytidines of the polynucleotide are of various kinds. For example, the polynucleotide comprises a plurality of modified cytidines that are at least two selected from the exemplary modified cytidines (e.g., 5-aza-cytidine and 6-aza-cytidine).

In some embodiments, the modified nucleoside in the polynucleotide is a modified adenosine. In some embodiments, 0.1%-100% of adenosines in the polynucleotide of the invention are modified. For example, at least 0.1%, at least 0.5%, at least 1%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 88%, at least 90%, at least 92%, at least 95%, at least 97%, at least 99% or 100% of adenosines are modified. In some embodiments, 80%-100% of adenosines are modified. In some embodiments, 100% of adenosines are modified. Exemplary modified adenosine may refer to the description about the RNA below, which will not be repeated here.

In some embodiments, the modified adenosines in the polynucleotide are of the same kind. For example, the polynucleotide comprises a plurality of modified adenosines, all of which are 2-amino-purine. In other embodiments, the modified adenosines of the polynucleotide are of various kinds. For example, the polynucleotide comprises a plurality of modified adenosines that are at least two selected from the exemplary modified adenosines (e.g. 2-amino-purine and 2,6-diaminopurine).

In some embodiments, the modified nucleoside in the polynucleotide is a modified guanosine. In some embodiments, 0.1%-100% of guanosines in the polynucleotide of the invention are modified. For example, at least 0.1%, at least 0.5%, at least 1%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 88%, at least 90%, at least 92%, at least 95%, at least 97%, at least 99% or 100% of guanosines are modified. In some embodiments, 80%-100% of guanosines are modified. In some embodiments, 100% of guanosines are modified. For example, the modified guanosine may refer to the description about the RNA below, which will not be repeated here.

In some embodiments, the modified guanosines in the polynucleotide are of the same kind. For example, the polynucleotide comprises a plurality of modified guanosines, all of which are inosine. In other embodiments, the modified guanosines of the polynucleotide are of various kinds. For example, the polynucleotide comprises a plurality of modified guanosines that are at least two selected from the exemplary modified guanosines (e.g. inosine and 1-methyl-inosine).

In addition, the modified nucleotides of the polynucleotides of the invention include nucleotides containing isotopes. The term "isotope" refers to an element having the same number of protons but different number of neutrons resulting in different mass number.

In some embodiments, the polynucleotide of the invention comprises a nucleotide comprising an isotope. In some embodiments, the polynucleotides of the invention comprises a nucleotide comprising an isotope of hydrogen. The isotope of hydrogen is not limited to deuterium, tritium. In addition, in some embodiments, the polynucleotide of the invention also comprises or contains an isotope of other element than hydrogen, wherein the element includes, but not limited to, carbon, oxygen, nitrogen, and phosphorus.

In some embodiments, the polynucleotide of the invention is RNA (e.g., mRNA).

It can be understood that the solution that the polynucleotide of the invention is an RNA includes the embodiments of replacing T nucleotide in the polynucleotide described above with U nucleotide.

In some embodiments, the polynucleotide of the invention is an RNA (e.g., mRNA) comprising any of the modified nucleosides described above.

In some embodiments, the modified poly(A) in the RNA comprises a nucleotide sequence having at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one nucleotide sequence of SEQ ID NOs: 101-200 and 248-283.

In some embodiments, the modified poly(A) in the RNA comprises a nucleotide sequence having at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one nucleotide sequence of SEQ ID NOs: 102-200 and 248-283, wherein the R element remains unchanged.

In some embodiments, the nucleotide sequence of the modified poly(A) in the RNA has at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one nucleotide sequence of SEQ ID NOs: 102-200 and 248-283, wherein the R element remains unchanged.

In some embodiments, the modified poly(A) in the RNA comprises any one of the nucleotide sequences of SEQ ID NOs: 102-200 and 248-283. In an optional specific example, the nucleotide sequence of the modified poly(A) in the RNA is set forth in any one of SEQ ID NOs: 102-200 and 248-283.

In some embodiments, the polynucleotide of the invention is DNA. In some embodiments, the stability of the polynucleotide of the invention is improved by at least about 5%, at least about 10%, at least about 20%, at least about 25%, at least about 30%, at least about 40%, at least about 50%, at least about 60%, at least about 70%, at least about 80%, at least about 90%, at least about 100%, at least about 150%, at least about 200% or more, as compared to a polynucleotide that does not comprise the modified poly(A) of the invention.

In some embodiments, a polynucleotide in the form of DNA of the invention may be transcribed in vitro to RNA. In some embodiments, the RNA is mRNA. In some embodiments, the yield of mRNA produced by the polynucleotide of the invention is increased by at least about 10%, at least about 20%, at least about 30%, at least about 40%, at least about 50%, at least about 60%, at least about 70%, at least about 80%, at least about 90%, at least about 100%, at least about 150%, at least about 200%, at least about 300%, at least about 400%, at least about 500% or more, as compared to a polynucleotide that does not comprise the modified poly(A) of the invention.

In some embodiments, the in vivo expression level of the polynucleotide of the invention is increased by at least about 10%, at least about 20%, at least about 30%, at least about 40%, at least about 50%, at least about 60%, at least about 70%, at least about 80%, at least about 90%, at least about 100%, at least about 150%, at least about 200%, at least about 300%, at least about 400%, at least about 500%, at least about 600%, at least about 700%, at least about 800%, at least about 900%, at least about 1000% or more, as compared to a polynucleotide that does not comprise the modified poly(A) of the invention.

### III. Vector

The invention also provides a vector comprising the modified poly(A) according to any one of the above embodiments of the invention or the polynucleotide according to any one of the above embodiments.

In some embodiments, the vector is an expression vector. In some embodiments, the vector is a plasmid, cosmid, virus, bacteriophage, or another vector conventionally used in genetic engineering. In some embodiments, the vector further comprises a promoter, 5'-UTR, and 3'-UTR that are located upstream of the modified poly(A) of the invention, wherein the modified poly(A), promoter, 5'-UTR, and 3'-UTR are operably linked to each other. In some embodiments, the vector further comprises a nucleic acid encoding a polypeptide of interest, wherein the nucleic acid encoding a polypeptide of interest is located between the 5'-UTR and the 3'-UTR.

In some embodiments, the vector is a plasmid. The plasmid of the invention preferably comprises at least an origin of replication (ORI), a marker gene or fragment thereof, and/or a reporter gene or fragment thereof, and a restriction site that allows insertion of a DNA element. Restriction sites in the form of multiple cloning sites (MCSs) are preferred.

In some embodiments, the stability of poly(A) in the vector (e.g., plasmid) of the invention is improved by at least about 5%, at least about 10%, at least about 20%, at least about 25%, at least about 30%, at least about 40%, at least about 50%, at least about 60%, at least about 70%, at least about 80%, at least about 90%, at least about 100%, at least about 150%, at least about 200% or more, as compared to others.

In some embodiments, the yield of mRNA by transcription of the vector (e.g., plasmid) of the invention is increased by at least about 10%, at least about 20%, at least about 30%, at least about 40%, at least about 50%, at least about 60%, at least about 70%, at least about 80%, at least about 90%, at least about 100%, at least about 150%, at least about 200%, at least about 300%, at least about 400%, at least about 500% or more, as compared to that of other vectors.

### IV. Preparation Method of Host Cell and Polynucleotide

The invention also provides a method of preparing a polynucleotide, comprising a step of introducing the modified poly(A) or the polynucleotide according to any one of the above embodiments of the invention into a host cell (such as E. coli) and then culturing the host cell containing the polynucleotide or poly(A).

The invention also provides a method of preparing a polynucleotide, comprising a step of preparing the polynucleotide by chemical synthesis based on the nucleotide sequence of the polynucleotide according to any one of the above embodiments. It can be understood that the specific method of chemical synthesis can be a method known in the art, such as solid-phase phosphoramidite method.

In addition, the invention also provides a host cell comprising the modified poly(A), the polynucleotide or the vector according to any one of the above embodiments of the invention.

In some embodiments, the host cell is bacterial cell. In other embodiments, the host cell is an antigen-presenting cell. In some embodiments, the host cell is dendritic cell, monocyte, or macrophage.

In some embodiments, the vector (e.g., plasmid) of the invention is stored and/or amplified in the host cell.

The host cell of the invention can be prepared by transforming a competent host cell with the vector of the invention. Competent host cell is a cell that has ability to take up free extracellular genetic material (e.g., DNA plasmid) independent of sequence. A variety of bacterial cells known to those skilled in the art are naturally capable of taking up exogenous DNA from the environment and thus can act as bacterial host cell according to the invention. Furthermore, it is known to those skilled in the art that competent bacterial host cell can be obtained from natural non-competent bacterial cell using, for example, electroporation or chemicals (e.g., treated with calcium ions with high temperature exposure). After uptake, the DNA plasmid is preferably neither degraded nor integrated into the genomic information of the bacterial host cell. Bacterial host cell includes E. coli cell well known to those skilled in the art.

### V. RNA and RNA Preparation Method, Polypeptide or Protein Preparation Method

The invention provides a method of preparing RNA, comprising a step of transcribing the polynucleotide or vector according to any one of the above embodiments of the invention.

In some embodiments, the method is an in vitro method.

In some embodiments, the method comprises contacting the vector (e.g., plasmid) of the invention with an RNA polymerase. In some embodiments, the method of the invention further comprises a step of linearizing the plasmid. In some embodiments, prior to linearization, the plasmid has a Superhelical Density of at least about 85% (e.g., 85%, 86%, 87%, 88%, 89%, 90%, 91%, 92%, 93%, etc.). In some embodiments, the method of the invention further comprises a step of purifying the linearized plasmid. In some embodiments, the method of the invention further comprises a step of purifying the RNA.

The invention also provides another method of preparing RNA, comprising a step of preparing the RNA by chemical synthesis according to the nucleotide sequence of the RNA according to any one of the above embodiments. It can be understood that the specific method of chemical synthesis can be a method known in the art, such as solid-phase phosphoramidite method.

In some embodiments, any method of preparing RNA of the invention further comprises a step of capping and optionally purifying the capped product. In some embodiments, the cap is Cap1 type cap. The structure of the Cap1 type cap is as follows:
cap G¹G² = m⁷G-5'-ppp-5'-Gm2'-3'-p-[m7 = 7-CH₃; m2' = 2'-O-CH₃; -ppp- = -PO₂H-O-PO₂H-O-PO₂H)-; -p- = -PO₂H-].

The capping reaction is shown below:
pppN1(p)Nx-OH(3')→ ppN1(pN)x-OH(3')+Pi
ppN1(pN)x-OH(3')+GTP → G(5')ppp(5')N1(pN)x-OH(3')+PPi
G(5')ppp(5')N1(pN)x-OH(3')+AdoMet → m7G(5')ppp(5')N1(pN)x-OH(3')+AdoHyc
m7GpppN1(pN)x-OH(3')+AdoMet → m7Gppp[m2'-O]N1(pN)x-OH(3')+AdoHyc.

In some embodiments, the RNA is mRNA.

The invention also provides an RNA prepared by the method of preparing RNA according to any one of the above embodiments.

In some embodiments, the modified poly(A) in the RNA comprises a nucleotide sequence having at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one nucleotide sequence of SEQ ID NOs: 101-200 and 248-283.

In some embodiments, the modified poly(A) in the RNA comprises a nucleotide sequence having at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one nucleotide sequence of SEQ ID NOs: 102-200 and 248-283, wherein the R element remains unchanged.

In some embodiments, the nucleotide sequence of the modified poly(A) in the RNA is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identical to one nucleotide sequence of SEQ ID NOs: 102-200 and 248-283, wherein the R element remains unchanged.

In some embodiments, the modified poly(A) in the RNA comprises any nucleotide sequence of SEQ ID NOs: 102-200 and 248-283. In an optional specific example, the nucleotide sequence of the modified poly(A) in the RNA is set forth in any one of SEQ ID NOs: 102-200 and 248-283.

In some embodiments, the RNA further comprises at least one of 5' cap, 5'-UTR, and 3'-UTR and a nucleic acid encoding a polypeptide or protein of interest that are located upstream of the modified poly(A). In an optional specific example, the RNA further comprises 5'-UTR and a nucleic acid encoding a polypeptide or protein of interest that are located upstream of the modified poly(A). Alternatively, the RNA further comprises 3'-UTR and a nucleic acid encoding a polypeptide or protein of interest that are located upstream of the modified poly(A). In some embodiments, the RNA comprises 5' cap, 5'-UTR, a nucleic acid encoding a polypeptide or protein of interest, and 3'-UTR that are located upstream of the modified poly(A).

It should be noted that the 5' cap, the 5'-UTR, the nucleic acid encoding a polypeptide or protein of interest, and the 3'-UTR are not particularly limited. In some embodiments, the 5'-UTR comprises a nucleotide sequence as set forth in SEQ ID NO: 210, and/or, the 3'-UTR comprises a nucleotide sequence as set forth in SEQ ID NO: 211. In some embodiments, the nucleotide sequence of 5'-UTR is set forth in SEQ ID NO: 210, and/or, the nucleotide sequence of 3'-UTR is set forth in SEQ ID NO: 211.

In some embodiments, the RNA comprises one or more modified nucleotides. In some embodiments, the modified nucleotide of the RNA has a base modification and/or a sugar modification. In some embodiments, the modified nucleotide of the RNA also has a phosphate group modification. In some embodiments, the modified nucleotide of the RNA has a base modification.

In some embodiments, the RNA comprises one or more modified nucleosides. In some embodiments, the RNA comprises at least one of a modified uridine, a modified cytidine, a modified adenosine, and a modified guanosine.

In some embodiments, the modified nucleoside in the RNA is a modified uridine. In some embodiments, 0.1%-100% of uridines in the RNA are modified. For example, at least 0.1%, at least 0.5%, at least 1%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 88%, at least 90%, at least 92%, at least 95%, at least 97%, at least 99%, or 100% of uridines are modified. In some embodiments, 80%-100% of uridines are modified. In some embodiments, 100% of uridines are modified. Exemplary modified uridine comprises pseudouridine (ψ), N1-methylpseudouridine, pyridin-4-one ribonucleoside, 5-aza-uridine, 6-aza-uridine, 2-thio-5-aza-uridine, 2-thiouridine (s2U), 4-thiouridine (s4U), 4-thiopseudouridine, 2-thiopseudouridine, 5-hydroxyuridine (ho5U), 5-aminoallyluridine, 5-halouridine (e.g., 5-iodouridine or 5-bromouridine), 3-methyluridine (m3U), 5-methoxyuridine (mo5U), uridine-5-oxyacetic acid (cmo5U), methyl uridine-5-oxyacetate (mcmo5U), 5-carboxymethyluridine (cm5U), 1-carboxymethylpseudouridine, 5-carboxyhydroxymethyluridine (chm5U), 5-carboxyhydroxymethyluridine methyl ester (mchm5U), 5-methoxycarbonylmethyluridine (mcm5U), 5-methoxycarbonylmethyl-2-thiouridine (mcm5s2U), 5-aminomethyl-2-thiouridine (nm5s2U), 5-methylaminomethyluridine (mnm5U), 5-methylaminomethyl-2-thiouridine (mnm5s2U), 5-methylaminomethyl-2-selenouridine (mnm5se2U), 5-carbamoylmethyluridine (ncm5U), 5-carboxymethylaminomethyluridine (cmnm5U), 5-carboxymethylaminomethyl-2-thiouridine (cmnm5s2U), 5-propynyluridine, 1-propynylpseudouridine, 5-taurinomethyl-uridine (τm5U), 1-taurinomethylpseudouridine, 5-taurinomethyl-2-thiouridine (τm5s2U), 1-taurinomethyl-4-thiopseudouridine, 5-methyluridine (m5U, i.e., with the nucleobase deoxythymidine), 1-methylpseudouridine (m1ψ), 5-methyl-2-thiouridine (m5s2U), 1-methyl-4-thiopseudouridine (m1s4ψ), 4-thio-1-methylpseudouridine, 3-methylpseudouridine (m3ψ), 2-thio-1-methylpseudouridine, 1-methyl-1-deazapseudouridine, 2-thio-1-methyl-1-deazapseudouridine, dihydrouridine (D), dihydropseudouridine, 5,6-dihydrouridine, 5-methyldihydrouridine (m5D), 2-thiodihydrouridine, 2-thiodihydropseudouridine, 2-methoxyuridine, 2-methoxy-4-thiouridine, 4-methoxypseudouridine, 4-methoxy-2-thiopseudouridine, N1-methylpseudouridine, 3-(3-amino-3-carboxypropyl) uridine (acp3U), 1-methyl-3-(3-amino-3-carboxypropyl) pseudouridine (acp3ψ), 5-(isopentenylaminomethyl) uridine (inm5U), 5-(isopentenylaminomethyl)-2-thiouridine (inm5s2U), α-thiouridine, 2'-O-methyluridine (Um), 5,2'-O-dimethyluridine (m5Um), 2'-O-methylpseudouridine (ψm), 2-thio-2'-O-methyluridine (s2Um), 5-methoxycarbonylmethyl-2'-O-methyluridine (mcm5Um), 5-carbamoylmethyl-2'-O-methyluridine (ncm5Um), 5-carboxymethylaminomethyl-2'-O-methyluridine (cmnm5Um), 3,2'-O-dimethyluridine (m3Um), and 5-(isopentenylaminomethyl)-2'-O-methyluridine (inm5Um), 1-thiouridine, deoxythymidine, 2'-F-arabino-uridine (2'-F-ara-uridine), 2'-F-uridine, 2'-OH-arabino-uridine, 5-(2-carbomethoxyvinyl)uridine and 5-[3-(1-E-propenylamino)] uridine.

In some embodiments, the modified uridines in the RNA are of the same kind. For example, the RNA comprises a plurality of modified uridines, all of which are N1-methylpseudouridine. In other embodiments, the modified uridines in the RNA are of various kinds. For example, the RNA comprises a plurality of modified uridines that are at least two selected from the above exemplary modified uridines (e.g. pseudouridine and N1-methylpseudouridine).

In some embodiments, the modified nucleoside in the RNA is a modified cytidine. In some embodiments, 0.1%-100% of cytidines in the RNA of the invention are modified. For example, at least 0.1%, at least 0.5%, at least 1%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 88%, at least 90%, at least 92%, at least 95%, at least 97%, at least 99%, or 100% of cytidines are modified. In some embodiments, 80%-100% of cytidines are modified. In some embodiments, 100% of cytidines are modified. Exemplary modified cytidine comprises 5-aza-cytidine, 6-aza-cytidine, pseudoisocytidine, 3-methylcytidine (m3C), N4-acetylcytidine (ac4C), 5-formylcytidine (f5C), N4-methylcytidine (m4C), 5-methylcytidine (m5C), 5-halocytidine (e.g., 5-iodocytidine), 5-hydroxymethylcytidine (hm5C), 1-methylpseudoisocytidine, pyrrolo-cytidine, pyrrolo-pseudoisocytidine, 2-thiocytidine (s2C), 2-thio-5-methylcytidine, 4-thiopseudoisocytidine, 4-thio-1-methylpseudoisocytidine, 4-thio-1-methyl-1-deazapseudoisocytidine, 1-methyl-1-deazapseudoisocytidine, zebularine, 5-aza-zebularine, 5-methylzebularine, 5-aza-2-thio-zebularine, 2-thio-zebularine, 2-methoxycytidine, 2-methoxy-5-methylcytidine, 4-methoxypseudoisocytidine, 4-methoxy-1-methylpseudoisocytidine, lysidine (k2C), α-thiocytidine, 2'-O-methylcytidine (Cm), 5,2'-O-dimethylcytidine (m5Cm), N4-acetyl-2'-O-methylcytidine (ac4Cm), N4,2'-O-dimethylcytidine (m4Cm), 5-formyl-2'-O-methylcytidine (f5Cm), N4,N4,2'-O-trimethylcytidine (m42Cm), 1-thiocytidine, 2'-F-arabino-cytidine, 2'-F-cytidine and 2'-OH-arabino-cytidine.

In some embodiments, the modified cytidines in the RNA are of the same kind. For example, the RNA comprises a plurality of modified cytidines, all of which are 5-aza-cytidine. In other embodiments, the modified cytidines of the RNA are of various kinds. For example, the RNA comprises a plurality of modified cytidines that are at least two selected from the above exemplary modified cytidines (e.g., 5-aza-cytidine and 6-aza-cytidine).

In some embodiments, the modified nucleoside in the RNA is a modified adenosine. In some embodiments, 0.1%-100% of adenosines in the RNA of the invention are modified. For example, at least 0.1%, at least 0.5%, at least 1%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 88%, at least 90%, at least 92%, at least 95%, at least 97%, at least 99%, or 100% of adenosines are modified. In some embodiments, 80%-100% of adenosines are modified. In some embodiments, 100% of adenosines are modified. Exemplary modified adenosine comprises 2-amino-purine, 2,6-diaminopurine, 2-amino-6-halo-purine (e.g., 2-amino-6-chloro-purine), 6-halo-purine (e.g., 6-chloro-purine), 2-amino-6-methyl-purine, 8-azido-adenosine, 7-deaza-adenine, 7-deaza-8-aza-adenine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m1A), 2-methyl-adenine (m2A), N6-methyl-adenosine (m6A), 2-methylthio-N6-methyl-adenosine (ms2m6A), N6-isopentenyl-adenosine (i6A), 2-methylthio-N6-isopentenyl-adenosine (ms2i6A), N6-(cis-hydroxyisopentenyl) adenosine (io6A), 2-methylthio-N6-(cis-hydroxyisopentenyl) adenosine (ms2io6A), N6-glycylcarbamoyl-adenosine (g6A), N6-threonylcarbamoyl-adenosine (t6A), N6-methyl-N6-threonylcarbamoyl-adenosine (m6t6A), 2-methylthio-N6-threonylcarbamoyl-adenosine (ms2g6A), N6,N6-dimethyl-adenosine (m62A), N6-hydroxynorvalylcarbamoyl-adenosine (hn6A), 2-methylthio-N6-hydroxynorvalylcarbamoyl-adenosine (ms2hn6A), N6-acetyl-adenosine (ac6A), 7-methyl-adenine, 2-methylthio-adenine, 2-methoxy-adenine, α-thio-adenosine, 2'-O-methyl-adenosine (Am), N6,2'-O-dimethyl-adenosine (m6Am), N6,N6,2'-O-trimethyl-adenosine (m62Am), 1,2'-O-dimethyl-adenosine (m1Am), 2'-O-ribosyladenosine (phosphate) (Ar(p)), 2-amino-N6-methyl-purine, 1-thio-adenosine, 8-azido-adenosine, 2'-F-arabino-adenosine, 2'-F-adenosine, 2'-OH-arabino-adenosine and N6-(19-amino-pentaoxanonadecyl)-adenosine.

In some embodiments, the modified adenosines in the RNA are of the same kind. For example, the RNA comprises a plurality of modified adenosines, all of which are 2-amino-purine. In other embodiments, the RNA modified adenosines are of various kinds. For example, the RNA comprises a plurality of modified adenosines that are at least two selected from the above exemplary modified adenosines (e.g. 2-amino-purine and 2,6 -diaminopurine).

In some embodiments, the modified nucleoside in the RNA is a modified guanosine. In some embodiments, 0.1%-100% of guanosines in the RNA of the invention are modified. For example, at least 0.1%, at least 0.5%, at least 1%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 88%, at least 90%, at least 92%, at least 95%, at least 97%, at least 99%, or 100% of guanosines are modified. In some embodiments, 80%-100% of guanosines are modified. In some embodiments, 100% of guanosines are modified. Exemplary modified guanosine comprises inosine (I), 1-methyl-inosine (m1I), wyosine (imG), methylwyosine (mimG), 4-demethyl-wyosine (imG-14), isowyosine (imG2), wybutosine (yW), peroxywybutosine (o2yW), hydroxywybutosine (OHyW), undermodified hydroxywybutosine (OHyW*), 7-deaza-guanosine, queuosine (Q), epoxyqueuosine (oQ), galactosyl-queuosine (galQ), mannosyl-queuosine (manQ), 7-cyano-7-deaza-guanosine (preQ0), 7-aminomethyl-7-deaza-guanosine (preQ1), archaeosine (G+), 7-deaza-8-aza-guanosine, 6-thio-guanosine, 6-thio-7-deaza-guanosine, 6-thio-7-deaza-8-aza-guanosine, 7-methyl-guanosine (m7G), 6-thio-7-methyl-guanosine, 7-methyl-inosine, 6-methoxy-guanosine, 1-methyl-guanosine (m1G), N2-methyl-guanosine (m2G), N2,N2-dimethyl-guanosine (m22G), N2,7-dimethyl-guanosine (m2,7G), N2,N2,7-trimethyl-guanosine (m2,2,7G), 8-oxo-guanosine, 7-methyl-8-oxo-guanosine, 1-methyl-6-thio-guanosine, N2-methyl-6-thio-guanosine, N2,N2-dimethyl-6-thio-guanosine, α-thio-guanosine, 2'-O-methyl-guanosine (Gm), N2-methyl-2'-O-methyl-guanosine (m2Gm), N2,N2-dimethyl-2'-O-methyl-guanosine (m22Gm), 1-methyl-2'-O-methyl-guanosine (m1Gm), N2,7-dimethyl-2'-O-methyl-guanosine (M2,7Gm), 2'-O-methyl-inosine (Im), 1,2'-O-dimethyl-inosine (m1Im), 2'-O-ribosylguanosine (phosphate) (Gr(p)), 1-thio-guanosine, O6-methyl-guanosine, 2'-F-arabino-guanosine and 2'-F-guanosine.

In some embodiments, the modified guanosines in the RNA are of the same kind. For example, the RNA comprises a plurality of modified guanosines, all of which are inosine. In other embodiments, the modified guanosines of the RNA are of various kinds. For example, the RNA comprises a plurality of modified guanosines that are at least two selected from the above exemplary modified guanosines (e.g. inosine and 1-methyl-inosine).

In addition, the modified nucleotide in the RNA comprises an isotope-containing nucleotide.

In some embodiments, the RNA comprises a nucleotide comprising an isotope of hydrogen. The isotope of hydrogen is not limited to deuterium, tritium. In addition, in some embodiments, the RNA further comprises or contains a nucleotide containing an isotope of other element than hydrogen, wherein the element includes, but not limited to, carbon, oxygen, nitrogen, and phosphorus.

In some embodiments, the RNA is mRNA. In addition, the invention further provides a method of preparing a polypeptide or protein, comprising: transcribing a polynucleotide encoding a polypeptide or protein of interest and the modified poly(A) of the invention into an mRNA; and translating the mRNA into a polypeptide or protein. In some embodiments, the method of preparing a polypeptide or protein is carried out in vitro or in vivo.

### VI. Lipid Nanoparticle and Pharmaceutical Composition

The invention provides a lipid nanoparticle (LNP) comprising the modified poly(A), polynucleotide, RNA, or vector according to any of the above embodiments of the invention.

In some embodiments, the RNA is mRNA.

In some embodiments, the lipid nanoparticle comprises a protonatable cationic lipid.

In some embodiments, the lipid nanoparticle further comprises one or more of a helper lipid, a structural lipid, and a PEG-lipid (polyethylene glycol-lipid). In some further embodiments, the lipid nanoparticle further comprises the helper lipid, the structural lipid, and the PEG-lipid.

In some embodiments as described above, the helper lipid is a phospholipid. The phospholipid are typically semi-synthetic or naturally derived or chemically modified. The phospholipid includes, but not limited to, DSPC (distearoyl phosphatidylcholine), DOPE (dioleoylphosphatidylethanolamine), DOPC (dioleoylphosphatidylcholine), DOPS (dioleoylphosphatidylserine), DSPG (1,2-distearoyl-sn-glycero-3-phospho-(1'-rac-glycerol)), DPPG (dipalmitoylphosphatidylglycerol), DPPC (dipalmitoylphosphatidylcholine), DGTS (1,2-dipalmitoyl-sn-glycerol-3-O-4'-(N,N,N-trimethyl)homoserine), lysophospholipids and the like. In some embodiments, the helper lipid is one or more selected from the group consisting of DSPC, DOPE, DOPC, and DOPS. In some embodiments, the helper lipid is DSPC and/or DOPE.

In some embodiments, the structural lipid is sterol, including but not limited to cholesterol, cholesteryl ester, steroid hormone, sterol vitamin, bile acid, cholesterol, ergosterol, β-sitosterol, and oxidized cholesterol derivatives, and the like. In some embodiments, the structural lipid is at least one selected from the group consisting of cholesterol, cholesterol esters, steroid hormones, sterol vitamins, and bile acids. In some embodiments, the structural lipid is cholesterol, preferably cholesterol of high purity, particularly injection grade high purity cholesterol, e.g., CHO-HP (produced by AVT).

As used herein, the term PEG-lipid (polyethylene glycol-lipid) is a conjugate of polyethylene glycol and lipid structure. In some embodiments, the PEG-lipid is selected from PEG-DMG and PEG-distearoylphosphatidylethanolamine (PEG-DSPE), preferably PEG-DMG. In some embodiments, the PEG-DMG is a polyethylene glycol (PEG) derivative of 1,2 -dimyristoyl glycerol. In some embodiments, the PEG has an average molecular weight of about 2000 to 5000, preferably about 2000.

In some further embodiments, the lipid nanoparticle further comprises the helper lipid, the structural lipid, and the PEG-lipid. In some embodiments, based on the total of the protonatable cationic lipid, the helper lipid, the structural lipid and the PEG-lipid, the lipid nanoparticle comprises the protonatable cationic lipid in the following amount (mole percentage): about 25.0% to 75.0%, for example, about 25.0% to 28.0%, 28.0% to 32.0%, 32.0% to 35.0%, 35.0% to 40.0%, 40.0% to 42.0%, 42.0% to 45.0%, 45.0% to 46.3%, 46.3% to 48.0%, 48.0% to 49.5%, 49.5% to 50.0%, 50.0% to 55.0%, 55.0% to 60.0%, 60.0% to 65.0%, or 65.0% to 75.0%.

In some embodiments, based on the total of the cationic lipid, helper lipid, structural lipid and PEG-lipid, the lipid nanoparticle described above comprises the following amount (mole percent) of helper lipid: 5% to 45%, for example, 5% to 9%, 9% to 9.4%, 9.4% to 10%, 10% to 10.5%, 10.5% to 11%, 11% to 15%, 15% to 16%, 16% to 18%, 18% to 20%, 20% to 25%, 25% to 33.5%, 33.5% to 37%, 37% to 40%, 40% to 42%, or 42% to 45%.

In some embodiments, based on the total of the cationic lipid, helper lipid, structural lipid and PEG-lipid, the lipid nanoparticle described above comprises the following amounts (mole percent) of structural lipid: 0% to 50%, for example, 0% to 10%, 10% to 15.5%, 15.5% to 18.5%, 18.5% to 22.5%, 22.5% to 23.5%, 23.5% to 28.5%, 28.5% to 33.5%, 33.5% to 35%, 35% to 36.5%, 36.5% to 38%, 38% to 38.5%, 38.5% to 39%, 39% to 39.5%, 39.5% to 40.5%, 40.5% to 41.5%, 41.5% to 42.5%, 42.5% to 42.7%, 42.7% to 43%, 43% to 43.5%, 43.5% to 45%, 45% to 46.5%, 46.5% to 48.5%, or 46.5% to 50%.

In some embodiments, based on the total of the cationic lipid, helper lipid, structural lipid and PEG-lipid, the lipid nanoparticle described above comprises the following amount (mole percent) of PEG-lipid: 0.5% to 5%, for example, 0.5% to 1%, 1% to 1.5%, 1.5% to 1.6%, 1.6% to 2%, 2% to 2.5%, 2.5% to 3%, 3% to 3.5%, 3.5% to 4%, 4% to 4.5%, or 4.5% to 5%.

The invention provides a cationic liposome comprising the modified poly(A), polynucleotide, RNA or vector of the invention.

The invention provides a cationic protein comprising the modified poly(A), polynucleotide, RNA or vector of the invention.

The invention provides a cationic polymer (lipopolyplex, LPP) comprising the modified poly(A), polynucleotide, RNA or vector of the invention.

The invention provides a pharmaceutical composition comprising the modified poly(A), polynucleotide, RNA, vector, host cell, cationic liposome, cationic protein, cationic polymer or lipid nanoparticle of the invention, and a pharmaceutically acceptable excipient.

As used herein, the term "pharmaceutically acceptable" means approved for use in animals and/or humans by regulatory agencies, such as the China Food and Drug Administration (CFDA), the Food and Drug Administration (FDA), or recognized pharmacopoeia (e.g., Chinese Pharmacopoeia, European Pharmacopeia). The term "excipient" refers to a substance that can be administered with the modified poly(A), polynucleotide, vector, host cell, cationic liposome, cationic protein, cationic polymer, or lipid nanoparticle of the invention, including but not limited to diluents, adjuvants, carriers, vehicles, glidants, sweeteners, preservatives, dyes/colorants, flavoring agents, surfactants, wetting agents, dispersing agents, suspending agents, stabilizers, isotonic agents, solvents, or emulsifiers.

VII. Use of the modified poly(A), polynucleotide, RNA, vector, host cell, cationic liposome, cationic protein, cationic polymer, lipid nanoparticle or pharmaceutical composition in the preparation of a medicament.

The invention provides use of the modified poly(A), polynucleotide, RNA, vector, host cell, cationic liposome, cationic protein, cationic polymer, lipid nanoparticle or pharmaceutical composition in the preparation of a medicament.

In some embodiments, the RNA is mRNA.

In some embodiments, the medicament is used in gene therapy, gene vaccination, protein replacement therapy, antisense therapy, or treatment by interfering RNA.

In some embodiments, the medicament is a nucleic acid drug. wherein the nucleic acid comprises at least one of RNA, messenger RNA (mRNA), antisense oligonucleotide, DNA, plasmid, ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), and cas9 mRNA.

In some embodiments, the medicament is used in the treatment and/or prevention of a disease. In some embodiments, the disease is selected from the group consisting of rare diseases, infectious diseases, cancers, hereditary diseases, autoimmune diseases, diabetes, neurodegenerative diseases, cardiovascular diseases, renal vascular diseases and metabolic diseases; in some embodiments, the cancers include one or more of lung cancer, gastric cancer, liver cancer, esophageal cancer, colon cancer, pancreatic cancer, brain cancer, lymphoma, leukemia, and prostate cancer; the hereditary diseases include one or more of hemophilia, thalassemia, and Gaucher's disease. Rare diseases include one or more of Brittle Bone Disease, Wilson Disease, Spinal Muscular Atrophy (SMA), Huntingdon's Disease, Rett Syndrome, Amyotropic Lateral Sclerosis (ALS), Duchenne Type Muscular dystrophy, Friedrichs Ataxia, methylmalonic acidemia (MMA), Cystic Fibrosis (CF), glycogen storage disease 1a (GSD1a), glycogen storage disease III (GSD III), Crigler-Najjar syndrome, ornithine transcarbamylase deficiency (OTCD), propionic acidemia (PA), phenylketonuria (PKU), hemophilia A, hemophilia B, β-thalassemia, Lafora disease, Dravet syndrome (DS), Alexander disease, Leber's congenital amaurosis (LCA), myelodysplastic syndromes (MDS), Homocystinuria due to CBS deficiency. In addition, the rare diseases in www.orpha.net/consor/cgi-bin/Disease Search List.php and rarediseases.info.nih.gov/diseases are also incorporated herein.

In some embodiments, the medicament is a vaccine. In some embodiments, the modified poly(A), polynucleotide, RNA, vector, host cell, cationic liposome, cationic protein, cationic polymer, lipid nanoparticle or pharmaceutical composition is used to generate an antigen of a pathogen or a portion thereof.

In some embodiments, the medicament is a gene therapy agent. In some embodiments, the modified poly(A), polynucleotide, RNA, vector, host cell, cationic liposome, cationic protein, cationic polymer, lipid nanoparticle or pharmaceutical composition is used to produce a genetic disease related protein.

In some embodiments, the modified poly(A), polynucleotide, RNA, vector, host cell, cationic liposome, cationic protein, cationic polymer, lipid nanoparticle or pharmaceutical composition is used to generate an antibody, such as scFV or nanobody.

In addition, the invention also provides a method of preventing or treating a disease, comprising administering to a subject in need thereof the polynucleotide, RNA, cationic liposome, cationic protein, cationic polymer, lipid nanoparticle or pharmaceutical composition of the invention.

In some embodiments, the disease or condition can be referenced to the above description regarding the use of the modified poly(A), polynucleotide, RNA, cationic liposome, cationic protein, cationic polymer, lipid nanoparticle or pharmaceutical composition of the invention in the preparation of a medicament.

In some embodiments, the polynucleotide, RNA, cationic liposome, cationic protein, cationic polymer, lipid nanoparticle or pharmaceutical composition is used as a vaccine for preventing a disease. In some embodiments, the polynucleotide, RNA, cationic liposome, cationic protein, cationic polymer, lipid nanoparticle or pharmaceutical composition is used to generate an antigen of a pathogen or portion thereof.

In some embodiments, the polynucleotide, RNA, cationic liposome, cationic protein, cationic polymer, lipid nanoparticle or pharmaceutical composition is used to generate a genetic disease related protein.

In some embodiments, the polynucleotide, RNA, cationic liposome, cationic protein, cationic polymer, lipid nanoparticle or pharmaceutical composition is used to generate an antibody, such as scFV or nanobody.

VIII. The significant advantages and effects of the invention:
The inventors have designed a series of poly(A)s, which can significantly improve the expression level of mRNA, have good stability, and can improve the yield of mRNA vaccines, which are valuable in the development of mRNA vaccines.

Specifically:
(1) Compared with a polynucleotide (e.g., DNA plasmid) comprising a poly(A) without the S element, the recombination rate of polynucleotide (e.g., DNA plasmid) comprising the modified poly(A) of the invention when expanded in bacterial cells is reduced.
(2) Compared with a polynucleotide (e.g., mRNA) comprising a poly(A) without the S element, a polynucleotide (e.g., mRNA) containing the modified poly(A) of the invention can increase the stability and/or translation efficiency of the nucleic acid encoding a polypeptide or protein of interest comprised therein, thereby increasing the expression level of the polypeptide or protein of interest.
(3) The inventors also unexpectedly found that when the number of a polynucleotide containing the modified poly(A) is lower than 80, compared with a polynucleotide (such as mRNA) containing a poly(A) without the S element, the polynucleotide containing the modified poly(A) of the invention can also reduce its recombination rate when it is amplified in bacterial cells, and at the same time can improve the stability and/or translation efficiency of the nucleic acid encoding a polypeptide or protein of interest comprised therein, thereby increasing the expression level of the polypeptide or protein of interest. For example, the number of the polynucleotide comprising the modified poly(A) is 30-79, 40-79, 30-70, 40-70, 50-70, or 60-70.

### Examples

To make the objectives, technical solutions, and advantages of the invention clearer, the following describes the technical solutions of the invention in detail. Apparently, the described examples are merely a part rather than all the embodiments of the invention. All other embodiments obtained by those of ordinary skill in the art based on the examples of the invention without creative efforts shall fall within the protection scope of the invention.

### Example 1: Design of Poly(A)

Poly(A) as shown in Table 1 was designed and synthesized by IDT (Integrated DNA Technologies) Co., Ltd., and the sequence was consistent with the theoretical design sequence according to the quality analysis report provided by the IDT.

**Table 1: Poly(A) List of the Invention**

| poly(A) numbering | DNA sequence (SEQ ID NO:) | RNA sequence (SEQ ID NO:) |
|---|---|---|
| polyA-V0 | AAAAAAAAAAAAAAAAAAAA AAAAAAAAAGCATATGACTAAA AAAAAAAAAAAAAAAAAAAA AAAAAAAAAAAAAAAAAAAA AAAAAAAAAAAAAAAAAAAA AAAA (1) | AAAAAAAAAAAAAAAAAAAAAAAA AAAAAAAGCAUAUGACUAAAAAA AAAAAAAAAAAAAAAAAAAAAAAA AAAAAAAAAAAAAAAAAAAAAAAA AAAAAAAAAAAAAAAAAA (101) |
| polyA-V1 | AAAAAAAAAAAAAAAAAAAAA AAAAAAAAAGGCAAAAAAAAA AAAAAAAAAAAAAAAAAAAAA AAAAAAAAAAAAAAAAAAAAA AAAAAAAAAAAAAAAAAAAA (2) | AAAAAAAAAAAAAAAAAAAAAAA AAAAAAAGGCAAAAAAAAAAAA AAAAAAAAAAAAAAAAAAAAAAA AAAAAAAAAAAAAAAAAAAAAAA AAAAAAAAAAA (102) |
| polyA-V2 | AAAAAAAAAAAAAAAAAAAA AAAAAAAAAGGTAAAAAAAA AAAAAAAAAAAAAAAAAAAAA AAAAAAAAAAAAAAAAAAAAA AAAAAAAAAAAAAAAAAA (3) | AAAAAAAAAAAAAAAAAAAAAAA AAAAAAAGGUAAAAAAAAAAAAA AAAAAAAAAAAAAAAAAAAAAAA AAAAAAAAAAAAAAAAAAAAAAA AAAAAAAAAAA (103) |
| polyA-V3 | AAAAAAAAAAAAAAAAAAAAA AAAAAAAAAGAGAAAAAAAAA AAAAAAAAAAAAAAAAAAAAA AAAAAAAAAAAAAAAAAAAAA AAAAAAAAAAAAAAAAAA (4) | AAAAAAAAAAAAAAAAAAAAAAA AAAAAAAGAGAAAAAAAAAAAAA AAAAAAAAAAAAAAAAAAAAAAA AAAAAAAAAAAAAAAAAAAAAAA AAAAAAAAAAA (104) |
| polyA-V4 | AAAAAAAAAAAAAAAAAAAAA AAAAAAAAAGCGAAAAAAAAA AAAAAAAAAAAAAAAAAAAAA AAAAAAAAAAAAAAAAAAAAA AAAAAAAAAAAAAAAAAAAA (5) | AAAAAAAAAAAAAAAAAAAAAAA AAAAAAAGCGAAAAAAAAAAAAA AAAAAAAAAAAAAAAAAAAAAAA AAAAAAAAAAAAAAAAAAAAAAA AAAAAAAAAA (105) |
| polyA-V5 | AAAAAAAAAAAAAAAAAAAAA AAAAAAAAAGGCTAAAAAAAAA AAAAAAAAAAAAAAAAAAAAAA AAAAAAAAAAAAAAAAAAAAAA AAAAAAAAAAAAAAAAAAAAA (6) | AAAAAAAAAAAAAAAAAAAAAAAAA AAAAAAAGGCUAAAAAAAAAAAAA AAAAAAAAAAAAAAAAAAAAAAAAA AAAAAAAAAAAAAAAAAAAAAAAAA AAAAAAAAAAAAA (106) |
| polyA-V6 | AAAAAAAAAAAAAAAAAAAAA AAAAAAAAAGTGTAAAAAAAAA AAAAAAAAAAAAAAAAAAAAAA AAAAAAAAAAAAAAAAAAAAAA AAAAAAAAAAAAAAAAAAAAA (7) | AAAAAAAAAAAAAAAAAAAAAAAA AAAAAAAGUGUAAAAAAAAAAAAA AAAAAAAAAAAAAAAAAAAAAAAA AAAAAAAAAAAAAAAAAAAAAAAA AAAAAAAAAAAA (107) |
| polyA-V7 | AAAAAAAAAAAAAAAAAAAAA AAAAAAAAAGCATATGACTAAA AAAAAAAAAAAAAAAAAAAAA AAAAAAGAAAAAAAAAAAAAA AAAAAAAAAAAAAAAAAAA (8) | AAAAAAAAAAAAAAAAAAAAAAA AAAAAAAGCAUAUGACUAAAAAA AAAAAAAAAAAAAAAAAAAAAAA AGAAAAAAAAAAAAAAAAAAAAA AAAAAAAAAAAA (108) |
| polyA-V8 | AAAAAAAAAAAAAAAAAAAAA AAAAAAAAAGCATATGACTAAA AAAAAAAAAAAAAAAAAAAAA AAAAAAGCAAAAAAAAAAAAA AAAAAAAAAAAAAAAAAA (9) | AAAAAAAAAAAAAAAAAAAAAAA AAAAAAAGCAUAUGACUAAAAAA AAAAAAAAAAAAAAAAAAAAAAA AGCAAAAAAAAAAAAAAAAAAAA AAAAAAAAAA (109) |
| polyA-V9 | AAAAAAAAAAAAAAAAAAAAA AAAAAAAAAGGGCAAAAAAAA AAAAAAAAAAAAAAAAAAAAA | AAAAAAAAAAAAAAAAAAAAAAA AAAAAAAGGGCAAAAAAAAAAAA AAAAAAAAAAAAAAAAAAAAAAA |
| | AAAAAAAAAAAAAAAAAAAA AAAAAAAAAAAAAAAAAAA (10) | AAAAAAAAAAAAAAAAAAAAAAA AAAAAAAAAAAAA (110) |
| polyA-V10 | AAAAAAAAAAAAAAAAAAAAA AAAAAAAAAGGGTAAAAAAAA AAAAAAAAAAAAAAAAAAAAA AAAAAAAAAAAAAAAAAAAAA AAAAAAAAAAAAAAAAAAAA (11) | AAAAAAAAAAAAAAAAAAAAAAAAA AAAAAAAGGGUAAAAAAAAAAAAAA AAAAAAAAAAAAAAAAAAAAAAAAA AAAAAAAAAAAAAAAAAAAAAAAAA AAAAAAAAAAAAA (111) |
| polyA-V11 | AAAAAAAAAAAAAAAAAAAAA AAAAAAAAAGAGGAAAAAAAA AAAAAAAAAAAAAAAAAAAAA AAAAAAAAAAAAAAAAAAAAA AAAAAAAAAAAAAAAAAAAA (12) | AAAAAAAAAAAAAAAAAAAAAAAAA AAAAAAAGAGGAAAAAAAAAAAAAA AAAAAAAAAAAAAAAAAAAAAAAAA AAAAAAAAAAAAAAAAAAAAAAAAA AAAAAAAAAAAA (112) |
| Pol-yA-V12 | AAAAAAAAAAAAAAAAAAAAA AAAAAAAAAGGGAAAAAAAAA AAAAAAAAAAAAAAAAAAAAA AAAAAAAAAAAAAAAAAAAAA AAAAAAAAAAAAAAAAAAA (13) | AAAAAAAAAAAAAAAAAAAAAAAAA AAAAAAAGGGAAAAAAAAAAAAAA AAAAAAAAAAAAAAAAAAAAAAAAA AAAAAAAAAAAAAAAAAAAAAAAAA AAAAAAAAAAA (113) |
| polyA-V13 | AAAAAAAAAAAAAAAAAAAA AAAAAAAAAGCTGATAAAAAA AAAAAAAAAAAAAAAAAAAAA AAAAAAAAAAAAAAAAAAAAA AAAAAAAAAAAAAAAAAAAAA (14) | AAAAAAAAAAAAAAAAAAAAAAAA AAAAAAAGCUGAUAAAAAAAAAAA AAAAAAAAAAAAAAAAAAAAAAAA AAAAAAAAAAAAAAAAAAAAAAAA AAAAAAAAAAAAAAAA (114) |
| polyA-V14 | AAAAAAAAAAAAAAAAAAAAA AAAAAAAAAGGCAAAAAAAAA AAAAAAAAAAAAAAAAAAAAA AAAAAAAAAAAAAAAAAAAAA AAAAAAAAAAAA (212) | AAAAAAAAAAAAAAAAAAAAAAA AAAAAAAGGCAAAAAAAAAAAAA AAAAAAAAAAAAAAAAAAAAAAA AAAAAAAAAAAAAAAAAAAAAAA A (248) |
| polyA-V15 | AAAAAAAAAAAAAAAAAAAA AAAAAAAAAGCTGATAAAAAA AAAAAAAAAAAAAAAAAAAA AAAAAAAAAAAAAAAAAAAAA AAAAAAAAAAAAA (15) | AAAAAAAAAAAAAAAAAAAAAAAAA AAAAAAAGCUGAUAAAAAAAAAAA AAAAAAAAAAAAAAAAAAAAAAAAA AAAAAAAAAAAAAAAAAAAAAAAAA AAAA (115) |
| polyA-V16 | AAAAAAAAAAAAAAAAAAAA AAAAAAAAAAAAAAAAAAAAGG CAAAAAAAAAAAAAAAAAAAAA AAAAAAAAAAAAAAAAAAAAAA AAAAAAAAAAAAAAAAAAAA (16) | AAAAAAAAAAAAAAAAAAAAAAA AAAAAAAAAAAAAAAAAGGCAAA AAAAAAAAAAAAAAAAAAAAAAA AAAAAAAAAAAAAAAAAAAAAAA AAAAAAAAAAAA (116) |
| polyA-V17 | AAAAAAAAAAAAAAAAAAAA AAAAAAAAAAAAAAAAAAAGCT GATAAAAAAAAAAAAAAAAAA AAAAAAAAAAAAAAAAAAAAAA AAAAAAAAAAAAAAAAAAAAAA (17) | AAAAAAAAAAAAAAAAAAAAAA AAAAAAAAAAAAAAAAGCUGAU AAAAAAAAAAAAAAAAAAAAAAA AAAAAAAAAAAAAAAAAAAAAAA AAAAAAAAAAAAAAA (117) |
| polyA-V18 | AAAAAAAAAAAAAAAAAAAAAA AAAAAAAAAAAAAAAAAAAAAA AAAAAAAAAAAAAAGGCAAAAA AAAAAAAAAAAAAAAAAAAAAA AAAAAAAAAAAAAAAAAAAA (18) | AAAAAAAAAAAAAAAAAAAAAAA AAAAAAAAAAAAAAAAAAAAAAA AAAAAAAAAGGCAAAAAAAAAAA AAAAAAAAAAAAAAAAAAAAAAA AAAAAAAAAAA (118) |
| polyA-V19 | AAAAAAAAAAAAAAAAAAAAA AAAAAAAAAAAAAAAAAAAAA AAAAAAAAAAAAAGCTGATAAA AAAAAAAAAAAAAAAAAAAAA | AAAAAAAAAAAAAAAAAAAAAAAA AAAAAAAAAAAAAAAAAAAAAAAA AAAAAAAAAGCUGAUAAAAAAAA AAAAAAAAAAAAAAAAAAAAAAAA |
| | AAAAAAAAAAAAAAAAAAA (19) | AAAAAAAAAAAAAA (119) |
| polyA-V20 | AAAAAAAAAAAAAAAAAAAAAA AAAAGAAAAGCATATGACTAAA AAAAAAAAAAAAAAAAAAAAAA AAAAAAAAAAAAAAAAAAAAAA AAAAAAAAAAAAAAAAAAAAAA AAAA (20) | AAAAAAAAAAAAAAAAAAAAAAAAA AAGAAAAGCAUAUGACUAAAAAA AAAAAAAAAAAAAAAAAAAAAAAAAA AAAAAAAAAAAAAAAAAAAAAAAAAA AAAAAAAAAAAAAAAAAAAA (120) |
| polyA-V21 | AAAAAAAAAAAAAAAAAAAAA AAAACAAAAGCATATGACTAAA AAAAAAAAAAAAAAAAAAAAA AAAAAAAAAAAAAAAAAAAAA AAAAAAAAAAAAAAAAAAAAA AAAA (21) | AAAAAAAAAAAAAAAAAAAAAAA AACAAAAGCAUAUGACUAAAAAA AAAAAAAAAAAAAAAAAAAAAAAAA AAAAAAAAAAAAAAAAAAAAAAAAA AAAAAAAAAAAAAAAAAAAAA (121) |
| polyA-V22 | AAAAAAAAAAAAAAAAAAAAA AAAATAAAAGCATATGACTAAA AAAAAAAAAAAAAAAAAAAAAA AAAAAAAAAAAAAAAAAAAAAA AAAAAAAAAAAAAAAAAAAAAA AAAA (22) | AAAAAAAAAAAAAAAAAAAAAA AAUAAAGCAUAUGACUAAAAAA AAAAAAAAAAAAAAAAAAAAAA AAAAAAAAAAAAAAAAAAAAAA AAAAAAAAAAAAAAAAAA (122) |
| polyA-V24 | AAAAAAAAAAAAAAAAAA AAAAAAAAAGCATATGACTAAA ACAAAAAAAAAAAAAAAAA AAAAAAAAAAAAAAAAAAAAAA AAAAAAAAAAAAAAAAAAAAAA AAAA (23) | AAAAAAAAAAAAAAAAAAAAAA AAAAAAAGCAUAUGACUAAAACA AAAAAAAAAAAAAAAAAAAAAAA AAAAAAAAAAAAAAAAAAAAAAA AAAAAAAAAAAAAAAAAAA (123) |
| polyA-V25 | AAAAAAAAAAAAAAAAAAAAAAA AAAAAAAAAGCATATGACTAAA ATAAAAAAAAAAAAAAAAAAAAA AAAAAAAAAAAAAAAAAAAAAAA AAAAAAAAAAAAAAAAAAAAAAA AAAA (24) | AAAAAAAAAAAAAAAAAAAAAAAA AAAAAAAGCAUAUGACUAAAUA AAAAAAAAAAAAAAAAAAAAAAAA AAAAAAAAAAAAAAAAAAAAAAAA AAAAAAAAAAAAAAAAAAA (124) |
| polyA-V26 | AAAAAAAAAAAAAAAAAAAAAA AAAAAAAAAAAAAAAAGAAAAGC ATATGACTAAAAAAAAAAAAAAA AAAAAAAAAAAAAAAAAAAAAAA AAAAAAAAAAAAAAAAAAAAAAA AAAA (25) | AAAAAAAAAAAAAAAAAAAAAA AAAAAAAAAAAAAGAAAAGCAUAU GACUAAAAAAAAAAAAAAAAAAA AAAAAAAAAAAAAAAAAAAAAA AAAAAAAAAAAAAAAAAA (125) |
| polyA-V27 | AAAAAAAAAAAAAAAAAAAAAA AAAAAAAAAAAAAAAACAAAAGC ATATGACTAAAAAAAAAAAAAAA AAAAAAAAAAAAAAAAAAAAAAA AAAAAAAAAAAAAAAAAAAAAAA AAAA (26) | AAAAAAAAAAAAAAAAAAAAAAAAA AAAAAAAAAAAAAACAAAAGCAUAU GACUAAAAAAAAAAAAAAAAAAAAA AAAAAAAAAAAAAAAAAAAAAAAAA AAAAAAAAAAAAAAAAAAA (126) |
| polyA-V28 | AAAAAAAAAAAAAAAAAAAA AAAAAAAAAAAAATAAAAGCA TATGACTAAAAAAAAAAAAAA AAAAAAAAAAAAAAAAAAAAA AAAAAAAAAAAAAAAAAAAAA AAA (27) | AAAAAAAAAAAAAAAAAAAAAAAA AAAAAAAAAAAAAUAAAAGCAUAU GACUAAAAAAAAAAAAAAAAAAAA AAAAAAAAAAAAAAAAAAAAAAAA AAAAAAAAAAAAAAAAAAA (127) |
| polyA-V29 | AAAAAAAAAAAAAAAAAAAA AAAAAAAAAAAAAAAAAAAAGC ATATGACTAAAAGAAAAAAAAA AAAAAAAAAAAAAAAAAAAAAA AAAAAAAAAAAAAAAAAAAAAA AAAA (28) | AAAAAAAAAAAAAAAAAAAAAAAAA AAAAAAAAAAAAAAAAAAAAAGCAUAU GACUAAAAGAAAAAAAAAAAAAAAAA AAAAAAAAAAAAAAAAAAAAAAAAAAA AAAAAAAAAAAAAAAAAAAAAAA (128) |
| polyA-V30 | AAAAAAAAAAAAAAAAAAAA AAAAAAAAAAAAAAAAAAGC ATATGACTAAAACAAAAAAA | AAAAAAAAAAAAAAAAAAAAAA AAAAAAAAAAAAAAAAGCAUAU GACUAAAACAAAAAAAAAAAAA |
| | AAAAAAAAAAAAAAAAAAAAA AAAAAAAAAAAAAAAAAAAAA AAAA (29) | AAAAAAAAAAAAAAAAAAAAAAAA AAAAAAAAAAAAAAAAAAAAA (129) |
| polyA-V31 | AAAAAAAAAAAAAAAAAAAAAA AAAAAAAAAAAAAAAAAAAAGC ATATGACTAAAATAAAAAAAAA AAAAAAAAAAAAAAAAAAAAAA AAAAAAAAAAAAAAAAAAAAAA AAAA (30) | AAAAAAAAAAAAAAAAAAAAAAAAA AAAAAAAAAAAAAAAAAAAGCAUAU GACUAAAAUAAAAAAAAAAAAAAAA AAAAAAAAAAAAAAAAAAAAAAAAA AAAAAAAAAAAAAAAAAAAA (130) |
| polyA-V32 | AAAAAAAAAAAAAAAAAAAAAA AAAAAAAAAGAATATGACTAAA AAAAAAAAAAAAAAAAAAAAAA AAAAAAAAAAAAAAAAAAAAAA AAAAAAAAAAAAAAAAAAAAAA AAAA (31) | AAAAAAAAAAAAAAAAAAAAAA AAAAAAAGAAUAUGACUAAAAAA AAAAAAAAAAAAAAAAAAAAAAAA AAAAAAAAAAAAAAAAAAAAAAAA AAAAAAAAAAAAAAAAAAA (131) |
| polyA-V33 | AAAAAAAAAAAAAAAAAAAA AAAAAAAAAGCATATAACTAAA AAAAAAAAAAAAAAAAAAAAA AAAAAAAAAAAAAAAAAAAAA AAAAAAAAAAAAAAAAAAAAA AAAA (32) | AAAAAAAAAAAAAAAAAAAAAAA AAAAAAAGCAUAUAACUAAAAAA AAAAAAAAAAAAAAAAAAAAAAA AAAAAAAAAAAAAAAAAAAAAAA AAAAAAAAAAAAAAAAA (132) |
| polyA-V34 | AAAAAAAAAAAAAAAAAAAAA AAAAAAAAAGCATATGAATAAA AAAAAAAAAAAAAAAAAAAAAA AAAAAAAAAAAAAAAAAAAAAA AAAAAAAAAAAAAAAAAAAAAA AAAA (33) | AAAAAAAAAAAAAAAAAAAAAA AAAAAAAGCAUAUGAAUAAAAA AAAAAAAAAAAAAAAAAAAAAAA AAAAAAAAAAAAAAAAAAAAAAA AAAAAAAAAAAAAAAA (133) |
| polyA-V35 | AAAAAAAAAAAAAAAAAAAAA AAAAAAAAAGCATATAAATAAA AAAAAAAAAAAAAAAAAAAAAA AAAAAAAAAAAAAAAAAAAAAA AAAAAAAAAAAAAAAAAAAAAA AAAA (34) | AAAAAAAAAAAAAAAAAAAAAAAAAAAA AAAAAAAAGCAUAUAAAUAAAAAAAAAA AAAAAAAAAAAAAAAAAAAAAAAAAAAA AAAAAAAAAAAAAAAAAAAAAAAAAAAA AAAAAAAAAAAAAAAAAAAAAA (134) |
| polyA-V36 | AAAAAAAAAAAAAAAAAAAAA AAAAAAAAAGCAAATGACTAAA AAAAAAAAAAAAAAAAAAAAA AAAAAAAAAAAAAAAAAAAAA AAAAAAAAAAAAAAAAAAAAA AAAA (35) | AAAAAAAAAAAAAAAAAAAAAAA AAAAAAAGCAAAUGACUAAAAAA AAAAAAAAAAAAAAAAAAAAAAA AAAAAAAAAAAAAAAAAAAAAAA AAAAAAAAAAAAAAAAAA (135) |
| polyA-V37 | AAAAAAAAAAAAAAAAAAAA AAAAAAAAAGCAAATGACTAGA AAAAAAAAAAAAAAAAAAAAAA AAAAAAAAAAAAAAAAAAAAAA AAAAAAAAAAAAAAAAAAAAAA AAAAAA (36) | AAAAAAAAAAAAAAAAAAAAAAA AAAAAAAGCAAAUGACUAGAAAA AAAAAAAAAAAAAAAAAAAAAAAA AAAAAAAAAAAAAAAAAAAAAAAA AAAAAAAAAAAAAAAAAAA (136) |
| polyA-V38 | AAAAAAAAAAAAAAAAAAAA AAAAAAAAGAAAGAAAAAAAA AAAAAAAAAAAAAAAAAAAAA AGAAAGAAAAAAAAAAAAAAA AAAAAAAAAAAAAAAAAAAAA AAAA (37) | AAAAAAAAAAAAAAAAAAAAAAAAAAAAA AAAAAAAGAAAGAAAAAAAAAAAAAAAA AAAAAAAAAAAAAAAAAAAAAAAGAAAG AAAAAAAAAAAAAAAAAAAAAAAAAAAAA AAAAAAAAAAAAAAAAAAAAAAA (137) |
| polyA-V39 | AAAAAAAAAAAAAAAAAAAAA AAAAAAAAGAAAGAAAAAAAA AAAAAAAAAAAAAAAAAAAAA AAAAAAAAAAAAAAAAAAAAA AAAAAAAAAAAAAAAAAAAA (38) | AAAAAAAAAAAAAAAAAAAAAAAAA AAAAAAGAAAGAAAAAAAAAAAAA AAAAAAAAAAAAAAAAAAAAAAAAA AAAAAAAAAAAAAAAAAAAAAAAAA AAAAAAAAAAAAA (138) |
| polyA-V40 | AAAAAAAAAAAAAAAAAAAAA AAAAAAAAAAAAAAAAAAAGA | AAAAAAAAAAAAAAAAAAAAAAA AAAAAAAAAAAAAAAAAGAAAGA |
| | AAGAAAAAAAAAAAAAAAAA AAAAAAAAAAAAAAAAAAAA AAAAA (39) | AAAAAAAAAAAAAAAAAAAAAAA AAAAAAAAAAAAAAAAAAAA (139) |
| polyA-V41 | AAAAAAAAAAAAAAAAAAAAA AAAAAAAAAAAAAAAAAAAAA AAAAAAAAGAAAGAAAAAAAA AAAAAAAAAAAAAAAAAAAAA AAAAAAAAAAAAAAAAAAAAA (40) | AAAAAAAAAAAAAAAAAAAAAAAAAAAA AAAAAAAAAAAAAAAAAAAAAAAAAAAAA AAAAGAAAGAAAAAAAAAAAAAAAAAAA AAAAAAAAAAAAAAAAAAAAAAAAAAAAA AAAAAAAAAAAAAA (140) |
| polyA-V42 | AAAAAAAAAAAAAGAAAAAA AAAAAAAAAAAAGAAAGAAAAA AAAAAAAAAAAAAAAAAAAAAA AAAAAAAAAAAAAAAAAAAAAA AAAAAAAAAAAAAAAAAAAAAA A (41) | AAAAAAAAAAAAAGAAAAAAAAAAA AAAAAAAAGAAAGAAAAAAAAAAAA AAAAAAAAAAAAAAAAAAAAAAAAA AAAAAAAAAAAAAAAAAAAAAAAAA AAAAAAAAAAAAAAAA (141) |
| polyA-V43 | AAAAAAAAAAAAGAAAAAAAA AAAAAAAAAAAAAAAAAAAAG AAAGAAAAAAAAAAAAAAAAA AAAAAAAAAAAAAAAAAAAAA AAAAAAAAAAAAAAAAAAAAA A (42) | AAAAAAAAAAAAAAGAAAAAAAAAAAA AAAAAAAAAAAAAAAAAAAAAAGAAAG AAAAAAAAAAAAAAAAAAAAAAAAAAA AAAAAAAAAAAAAAAAAAAAAAAAAAA AAAAAAAAAAAAAAAA (142) |
| polyA-V44 | AAAAAAAAAAAAAGAAAAAAAA AAAAAAAAAAAAAAAAAAAAAA AAAAAAAAAAGAAAGAAAAAAA AAAAAAAAAAAAAAAAAAAAAA AAAAAAAAAAAAAAAAAAAAAA A (43) | AAAAAAAAAAAAAGAAAAAAAAAAA AAAAAAAAAAAAAAAAAAAAAAAAA AAAAAGAAAGAAAAAAAAAAAAAAA AAAAAAAAAAAAAAAAAAAAAAAAA AAAAAAAAAAAAAAAA (143) |
| polyA-V45 | AAAAAAAGAAAAAAAAAAAAA AAAAAAAAAAGAAAGAAAAAA AAAAAAAAAAAAAAAAAAAAA AAAAAAAAAAAAAAAAAAAAA AAAAAAAAAAAAAAAAAAAAA A (44) | AAAAAAAGAAAAAAAAAAAAAAAAA AAAAAAAGAAAGAAAAAAAAAAAAA AAAAAAAAAAAAAAAAAAAAAAAAA AAAAAAAAAAAAAAAAAAAAAAAAA AAAAAAAAAAAAAAAA (144) |
| polyA-V46 | AAAAAAAGAAAAAAAAAAAAAA AAAAAAAAAAAAAAAAAAAAAG AAAGAAAAAAAAAAAAAAAAAA AAAAAAAAAAAAAAAAAAAAAA AAAAAAAAAAAAAAAAAAAAAA A (45) | AAAAAAAGAAAAAAAAAAAAAAAA AAAAAAAAAAAAAAAAAAAAGAAAG AAAAAAAAAAAAAAAAAAAAAAAAA AAAAAAAAAAAAAAAAAAAAAAAAA AAAAAAAAAAAAAAAAAA (145) |
| polyA-V47 | AAAAAAAGAAAAAAAAAAAAAA AAAAAAAAAAAAAAAAAAAAAA AAAAAAAAAGAAAGAAAAAAAA AAAAAAAAAAAAAAAAAAAAAA AAAAAAAAAAAAAAAAAAAAAA A (46) | AAAAAAAGAAAAAAAAAAAA AAAAAAAAAAAAAAAAAAAA AAAAAGAAAGAAAAAAAAAA AAAAAAAAAAAAAAAAAAAA AAAAAAAAAAAAA (146) |
| polyA-V48 | AAAAAAAAAAAAAAAAAAAAA AAAAAAAAACAAAGGCTCTTTT CAGAGCCACCAAAAAAAAAAAA AAAAAAAAAAAAAAAAAAAAAA AAAAAAAAAAAAAAAAAAAAAA AAAAAAAAAAAAAAA (47) | AAAAAAAAAAAAAAAAAAAAAAA AAAAAAAACAAAGGCUCUUUUCAG AGCCACCAAAAAAAAAAAAAAAA AAAAAAAAAAAAAAAAAAAAAAAA AAAAAAAAAAAAAAAAAAAAAAAA AAAAA (147) |
| polyA-V49 | AAAAAAAAAAAAAAAAAAAAAA AAAAAAAAAGGCTCAAAACAGA GCCACCAAAAAAAAAAAAAAAA AAAAAAAAAAAAAAAAAAAAAA AAAAAAAAAAAAAAAAAAAAAA AAAAAAAAAAAAAAA (48) | AAAAAAAAAAAAAAAAAAAAAA AAAAAAAGGCUCAAAACAGAGCC ACCAAAAAAAAAAAAAAAAAAA AAAAAAAAAAAAAAAAAAAAAAA AAAAAAAAAAAAAAAAAAAAAAA AAAAA (148) |
| polyA-V50 | AAAAAAAAAAAAAAAAAAAAA AAAAAAAAAGCATATGACTAAA | AAAAAAAAAAAAAAAAAAAAAAA AAAAAAAGCAUAUGACUAAAAGC |
| | AGCAAAAAAAAAAAAAAAAA AAAAAAAAAAAAAAAAAAAA AAAAAAAAAAAAAAAAAAAA AAAA (49) | AAAAAAAAAAAAAAAAAAAAAA AAAAAAAAAAAAAAAAAAAAAA AAAAAAAAAAAAAAAAA (149) |
| polyA-V51 | AAAAAAAAAAAAAAAAAAAAAA AAAAAAAAAGCATATGACTAAA AGTAAAAAAAAAAAAAAAAAAA AAAAAAAAAAAAAAAAAAAAAA AAAAAAAAAAAAAAAAAAAAAA AAAA (50) | AAAAAAAAAAAAAAAAAAAAAAA AAAAAAAGCAUAUGACUAAAAGU AAAAAAAAAAAAAAAAAAAAAAA AAAAAAAAAAAAAAAAAAAAAAAA AAAAAAAAAAAAAAAAAA (150) |
| polyA-V52 | AAAAAAAAAAAAAAAAAAAAAA AAAAAAAAAAGCATATGACTAAA AGTAAAAAAAAAAAAAAAAAAA AAAAAAAAAAAAAAAAAAAAAAA AAAAAAAAAAAAAAAAAAAAAAA AAA (51) | AAAAAAAAAAAAAAAAAAAAAA AAAAAAAGCAUAUGACUAAAAGU UAAAAAAAAAAAAAAAAAAAAA AAAAAAAAAAAAAAAAAAAAAA AAAAAAAAAAAAAAAAA (151) |
| polyA-V53 | AAAAAAAAAAAAAAAAAAAAAA AAAAAAAAAGCATATGACTAAA GATAAAAAAAAAAAAAAAAAAA AAAAAAAAAAAAAAAAAAAAAAA AAAAAAAAAAAAAAAAAAAAAAA AAAA (52) | AAAAAAAAAAAAAAAAAAAAAA AAAAAAAGCAUAUGACUAAAGAU AAAAAAAAAAAAAAAAAAAAAA AAAAAAAAAAAAAAAAAAAAAA AAAAAAAAAAAAAAAAAAA (152) |
| polyA-V54 | AAAAAAAAAAAAAAAAAAAAA AAAAAAAAAGCATATGACTAAA GCATATAAAAAAAAAAAAAAAA AAAAAAAAAAAAAAAAAAAAAA AAAAAAAAAAAAAAAAAAAAAA AAA (53) | AAAAAAAAAAAAAAAAAAAAAAA AAAAAAAGCAUAUGACUAAAGCA UAUAAAAAAAAAAAAAAAAAAAA AAAAAAAAAAAAAAAAAAAAAAA AAAAAAAAAAAAAAAAAA (153) |
| polyA-V55 | AAAAAAAAAAAAAAAAAAAAA AAAAAAAAAGCATATGACTAAA GCATTAAAAAAAAAAAAAAAAA AAAAAAAAAAAAAAAAAAAAAA AAAAAAAAAAAAAAAAAAAAAA AAA (54) | AAAAAAAAAAAAAAAAAAAAAA AAAAAAAGCAUAUGACUAAAGCA UUAAAAAAAAAAAAAAAAAAAAA AAAAAAAAAAAAAAAAAAAAAAA AAAAAAAAAAAAAAAAAA (154) |
| polyA-V56 | AAAAAAAAAAAAAAAAAAAAAAA AAAAAAAAAAGCATATGACTAAA AAAAAAAAAAGAAAAAAAAAAAA AAAAAAAAAAAAAAAAAAAAAAAA AAAAAAAAAAAAAAAAAAAAAAAA AAAA (55) | AAAAAAAAAAAAAAAAAAAAAAA AAAAAAAGCAUAUGACUAAAAA AAAAAAGAAAAAAAAAAAAAAAA AAAAAAAAAAAAAAAAAAAAAAAAAA AAAAAAAAAAAAAAAAAAAAAA (155) |
| polyA-V57 | AAAAAAAAAAAAAAAAAAAAA AAAAAAAAAGCATATGACTAAA AAAAAAAAAAAAAAAAAGAAAA AAAAAAAAAAAAAAAAAAAAAA AAAAAAAAAAAAAAAAAAAAAA AAAA (56) | AAAAAAAAAAAAAAAAAAAAAA AAAAAAAGCAUAUGACUAAAAAA AAAAAAAAAAAAAAGAAAAAAA AAAAAAAAAAAAAAAAAAAAAA AAAAAAAAAAAAAAAAA (156) |
| polyA-V58 | AAAAAAAAAAAAAAAAAAAAA AAAAAAAAAGCATATGACTAAA GAAAAAAAAAAAAAAAAAAAA AAAAAAAAAAAAAAAAAAAAA AAAAAAAAAAAAAAAAAAAAA AAAA (57) | AAAAAAAAAAAAAAAAAAAAAAA AAAAAAAGCAUAUGACUAAAGAA AAAAAAAAAAAAAAAAAAAAAAA AAAAAAAAAAAAAAAAAAAAAAA AAAAAAAAAAAAAAAAAA (157) |
| polyA-V59 | AAAAAAAAAAAAAAAAAAAAA AAAAAAAAAGCATATGACTAAA AGAAAAAAAAAAAAAAAAAAA AAAAAAAAAAAAAAAAAAAAA AAAAAAAAAAAAAAAAAAAAA AAAA (58) | AAAAAAAAAAAAAAAAAAAAAAAAA AAAAAAAGCAUAUGACUAAAAGA AAAAAAAAAAAAAAAAAAAAAAAAA AAAAAAAAAAAAAAAAAAAAAAAAA AAAAAAAAAAAAAAAAAAAA (158) |
| polyA-V60 | AAAAAAAAAAAAAAAAAAAAAAA | AAAAAAAAAAAAAAAAAAAAAAAAA |
| | AAAAAAAAAGCATATGACTAAA AAGAAAAAAAAAAAAAAAAAAA AAAAAAAAAAAAAAAAAAAAAA AAAAAAAAAAAAAAAAAAAAAA AAAAA (59) | AAAAAAGCAUAUGACUAAAAG AAAAAAAAAAAAAAAAAAAAA AAAAAAAAAAAAAAAAAAAAA AAAAAAAAAAAAAAAA (159) |
| polyA-V61 | AAAAAAAAAAAAAAAAAAAA AAAAAAAAAGCATATGACTAAA AAAGAAAAAAAAAAAAAAAAA AAAAAAAAAAAAAAAAAAAAA AAAAAAAAAAAAAAAAAAAAA AAAA (60) | AAAAAAAAAAAAAAAAAAAAAAAA AAAAAAAGCAUAUGACUAAAAAA GAAAAAAAAAAAAAAAAAAAAAAA AAAAAAAAAAAAAAAAAAAAAAAA AAAAAAAAAAAAAAAAAAA (160) |
| polyA-V62 | AAAAAAAAAAAAAAAAAAAAAA AAAAAAAAAGCATATGACTAAA AAAAGAAAAAAAAAAAAAAAAA AAAAAAAAAAAAAAAAAAAAAAA AAAAAAAAAAAAAAAAAAAAAA AAAA (61) | AAAAAAAAAAAAAAAAAAAAAA AAAAAAAGCAUAUGACUAAAAA AGAAAAAAAAAAAAAAAAAAAA AAAAAAAAAAAAAAAAAAAAAA AAAAAAAAAAAAAAAAAA (161) |
| polyA-V63 | AAAAAAAAAAAAAAAAAAAAA AAAAAAAAAGCATATGACTAAA AAAAAGAAAAAAAAAAAAAAA AAAAAAAAAAAAAAAAAAAAAA AAAAAAAAAAAAAAAAAAAAAA AAAA (62) | AAAAAAAAAAAAAAAAAAAAAAA AAAAAAAGCAUAUGACUAAAAAA AAGAAAAAAAAAAAAAAAAAAAA AAAAAAAAAAAAAAAAAAAAAAA AAAAAAAAAAAAAAAAAA (162) |
| polyA-V64 | AAAAAAAAAAAAAAAAAAAAAA AAAAAAAAAGCATATGACTAA AAAAAAGAAAAAAAAAAAAAAA AAAAAAAAAAAAAAAAAAAAAA AAAAAAAAAAAAAAAAAAAAAA AAAA (63) | AAAAAAAAAAAAAAAAAAAAAAAA AAAAAAAGCAUAUGACUAAAAA AAAGAAAAAAAAAAAAAAAAAAAA AAAAAAAAAAAAAAAAAAAAAAAA AAAAAAAAAAAAAAAAAAAA (163) |
| polyA-V66 | AAAAAAAAAAAAAAAAAAAAA AAAAAAAAGAAAGAAAAAAAA AAAAAAAAAAAAAAAAAAAAA GAAAGAAAAAAAAAAAAAAAA AAAAAAAAAAAA (64) | AAAAAAAAAAAAAAAAAAAAAAA AAAAAAGAAAGAAAAAAAAAAAA AAAAAAAAAAAAAAAAAGAAAGA AAAAAAAAAAAAAAAAAAAAAAA AAAA (164) |
| polyA-V67 | AAAAAAAAAAAAAAAAAAAA AAAAAAAAAGGCAAGAAAAAA AAAAAAAAAAAAAAAAAAAAAA AAAAAAAAAAAAAAAAAAAAAA AAAAAAAAAAAAAAAAAAA (65) | AAAAAAAAAAAAAAAAAAAAAAAA AAAAAAAGGCAAGAAAAAAAAAAA AAAAAAAAAAAAAAAAAAAAAAAA AAAAAAAAAAAAAAAAAAAAAAAA AAAAAAAAAAA (165) |
| polyA-V68 | AAAAAAAAAAAAAAAAAAAAA AAAAAAAAAGGCAATAAAAAAA AAAAAAAAAAAAAAAAAAAAAAA AAAAAAAAAAAAAAAAAAAAAAA AAAAAAAAAAAAAAAAAAA (66) | AAAAAAAAAAAAAAAAAAAAAAA AAAAAAAGGCAAUAAAAAAAAAA AAAAAAAAAAAAAAAAAAAAAAA AAAAAAAAAAAAAAAAAAAAAAA AAAAAAAAAA (166) |
| polyA-V69 | AAAAAAAAAAAAAAAAAAAAA AAAAAAAAAGGCAACAAAAAA AAAAAAAAAAAAAAAAAAAAAA AAAAAAAAAAAAAAAAAAAAAA AAAAAAAAAAAAAAAAAAAA (67) | AAAAAAAAAAAAAAAAAAAAAAAAA AAAAAAAGGCAACAAAAAAAAAAAA AAAAAAAAAAAAAAAAAAAAAAAAA AAAAAAAAAAAAAAAAAAAAAAAAA AAAAAAAAAAA (167) |
| polyA-V70 | AAAAAAAAAAAAAAAAAAAAA AAAAAAAAAGGCAAAGAAAAA AAAAAAAAAAAAAAAAAAAAA AAAAAAAAAAAAAAAAAAAAA AAAAAAAAAAAAAAAAAAA (68) | AAAAAAAAAAAAAAAAAAAAAAAA AAAAAAAGGCAAAGAAAAAAAAAA AAAAAAAAAAAAAAAAAAAAAAAAA AAAAAAAAAAAAAAAAAAAAAAAAA AAAAAAAAAAAAAAA (168) |
| polyA-V71 | AAAAAAAAAAAAAAAAAAAAA AAAAAAAAAGGCAAATAAAAAA | AAAAAAAAAAAAAAAAAAAAAAA AAAAAAAGGCAAAUAAAAAAAAA |
| | AAAAAAAAAAAAAAAAAAAAAAAAA AAAAAAAAAAAAAAAAAAAAAAAAA AAAAAAAAAAAAAAAAAAA (69) | AAAAAAAAAAAAAAAAAAAAAAA AAAAAAAAAAAAAAAAAAAAAAA AAAAAAAAAAAAAAA (169) |
| polyA-V72 | AAAAAAAAAAAAAAAAAAAA AAAAAAAAGGCAAACAAAAAA AAAAAAAAAAAAAAAAAAAAA AAAAAAAAAAAAAAAAAAAAA AAAAAAAAAAAAAAAAAA (70) | AAAAAAAAAAAAAAAAAAAAAAA AAAAAAGGCAAACAAAAAAAAAA AAAAAAAAAAAAAAAAAAAAAAA AAAAAAAAAAAAAAAAAAAAAAA AAAAAAAAAA (170) |
| polyA-V73 | AAAAAAAAAAAAAAAAAAA AAAAGAAAAGTGTAAAAAAAA AAAAAAAAAAAAAAAAAAA AAAAAAAAAAAAAAAAAAA AAAAAAAAAAAAAAAAA (71) | AAAAAAAAAAAAAAAAAAAAAAAA AAGAAAAGUGUAAAAAAAAAAAAA AAAAAAAAAAAAAAAAAAAAAAAA AAAAAAAAAAAAAAAAAAAAAAAA AAAAAAAAAAAAAAAAAA (171) |
| polyA-V74 | AAAAAAAAAAAAAAAAAAAA AAAAAAAAAGTGTAAAAGAAA AAAAAAAAAAAAAAAAAAAA AAAAAAAAAAAAAAAAAAAA AAAAAAAAAAAAAAAAAA (72) | AAAAAAAAAAAAAAAAAAAAAA AAAAAAAGUGUAAAGAAAAAAA AAAAAAAAAAAAAAAAAAAAAAA AAAAAAAAAAAAAAAAAAAAAAA AAAAAAAAAAAAA (172) |
| polyA-V75 | AAAAAAAAAAAAAAAAAAAA AAAAAGAAAGTGTAAAAAAA AAAAAAAAAAAAAAAAAAAA AAAAAAAAAAAAAAAAAAAA AAAAAAAAAAAAAAAAAA (73) | AAAAAAAAAAAAAAAAAAAAAAA AAAGAAAGUGUAAAAAAAAAAAA AAAAAAAAAAAAAAAAAAAAAAA AAAAAAAAAAAAAAAAAAAAAAA AAAAAAAAAAAAA (173) |
| polyA-V76 | AAAAAAAAAAAAAAAAAAAAAAA AAAAAAAAAGTGTAAAGAAAAAA AAAAAAAAAAAAAAAAAAAAAAA AAAAAAAAAAAAAAAAAAAAAAA AAAAAAAAAAAAAAAAAAAAA (74) | AAAAAAAAAAAAAAAAAAAAAAAA AAAAAAAGUGUAAAGAAAAAAAAA AAAAAAAAAAAAAAAAAAAAAAAA AAAAAAAAAAAAAAAAAAAAAAAA AAAAAAAAAAAAA (174) |
| polyA-V77 | AAAAAAAAAAAAAAAAAAAAAA AAAACAAAAGTGTAAAGAAAAA AAAAAAAAAAAAAAAAAAAAAA AAAAAAAAAAAAAAAAAAAAAA AAAAAAAAAAAAAAAAAAAA (75) | AAAAAAAAAAAAAAAAAAAAA AACAAAAGUGUAAAGAAAAAAA AAAAAAAAAAAAAAAAAAAAAA AAAAAAAAAAAAAAAAAAAAAA AAAAAAAAAAAA (175) |
| polyA-V78 | AAAAAAAAAAAAAAAAAAAAA AAAAAAAAAGTGTAAAACAAAA AAAAAAAAAAAAAAAAAAAAAA AAAAAAAAAAAAAAAAAAAAAA AAAAAAAAAAAAAAAAAAA (76) | AAAAAAAAAAAAAAAAAAAAAAAA AAAAAAAGUGUAAACAAAAAAA AAAAAAAAAAAAAAAAAAAAAAAA AAAAAAAAAAAAAAAAAAAAAAAA AAAAAAAAAAAAA (176) |
| polyA-V79 | AAAAAAAAAAAAAAAAAAAAA AAAAGAAAAGCTGATAAAAAA AAAAAAAAAAAAAAAAAAAAAA AAAAAAAAAAAAAAAAAAAAAA AAAAAAAAAAAAAAAAAAAAAA (77) | AAAAAAAAAAAAAAAAAAAAAAA AAGAAAAGCUGAUAAAAAAAAAA AAAAAAAAAAAAAAAAAAAAAAA AAAAAAAAAAAAAAAAAAAAAAA AAAAAAAAAAAAAA (177) |
| polyA-V80 | AAAAAAAAAAAAAAAAAAAA AAAAAAAAAGCTGATAAAGAA AAAAAAAAAAAAAAAAAAAAA AAAAAAAAAAAAAAAAAAAAA AAAAAAAAAAAAAAAAAAAAA (78) | AAAAAAAAAAAAAAAAAAAAAAA AAAAAAAGCUGAUAAAGAAAAA AAAAAAAAAAAAAAAAAAAAAAA AAAAAAAAAAAAAAAAAAAAAAA AAAAAAAAAAAAAAA (178) |
| polyA-V81 | AAAAAAAAAAAAAAAAAAAAA AAAAAAAAAGCTGATAAAACAA AAAAAAAAAAAAAAAAAAAAAA | AAAAAAAAAAAAAAAAAAAAAA AAAAAAAGCUGAUAAACAAAAA AAAAAAAAAAAAAAAAAAAAAA |
| | AAAAAAAAAAAAAAAAAAAAA AAAAAAAAAAAAAAAAAAAAA (79) | AAAAAAAAAAAAAAAAAAAAAAA AAAAAAAAAAAAAAAA (179) |
| polyA-V82 | AAAAAAAAAAAAAAAAAAAAAA AAAACAAAAGCTGATAAAAAAA AAAAAAAAAAAAAAAAAAAAAA AAAAAAAAAAAAAAAAAAAAAA AAAAAAAAAAAAAAAAAAAAAA (80) | AAAAAAAAAAAAAAAAAAAAAA AACAAAAGCUGAUAAAAAAAAA AAAAAAAAAAAAAAAAAAAAAAA AAAAAAAAAAAAAAAAAAAAAAA AAAAAAAAAAAAAAA (180) |
| polyA-V83 | AAAAAAAAAAAAAAAAAAAAA AAAAAGAAAGCTGATAAAAAAA AAAAAAAAAAAAAAAAAAAAAA AAAAAAAAAAAAAAAAAAAAAA AAAAAAAAAAAAAAAAAAAAAA (81) | AAAAAAAAAAAAAAAAAAAAAAA AAAGAAAGCUGAUAAAAAAAAAA AAAAAAAAAAAAAAAAAAAAAAA AAAAAAAAAAAAAAAAAAAAAAA AAAAAAAAAAAAAAAA (181) |
| polyA-V84 | AAAAAAAAAAAAAAAAAAAA AAAAAAAAAGCTGATAAAGAAA AAAAAAAAAAAAAAAAAAAAAAA AAAAAAAAAAAAAAAAAAAAAAA AAAAAAAAAAAAAAAAAAAAAAA (82) | AAAAAAAAAAAAAAAAAAAAAA AAAAAAAGCUGAUAAAGAAAAAA AAAAAAAAAAAAAAAAAAAAAAA AAAAAAAAAAAAAAAAAAAAAAA AAAAAAAAAAAAAAA (182) |
| polyA-V85 | AAAAAAAAAAAAAAAAAAAAA AAAAAAAAAGGCAAAAAAAAA AAAAAAAAAAAAAAAAAAAAA (83) | AAAAAAAAAAAAAAAAAAAAAA AAAAAAAGGCAAAAAAAAAAAA AAAAAAAAAAAAAAAAAA (183) |
| polyA-V86 | AAAAAAAAAAAAAAAAAA AAAAAAAAAGGCAAAAAA AAAAAAAAA (84) | AAAAAAAAAAAAAAAAAAAAAAA AAAAAAAGGCAAAAAAAAAAAA AAAAA (184) |
| polyA-V87 | AAAAAAAAAAAAAAAAAAAAA AAAAAAAAAGGCAAAAAAAAA AAAAAAAAAAAAAAAAAAAAA AAAAAAA (85) | AAAAAAAAAAAAAAAAAAAAAAA AAAAAAAGGCAAAAAAAAAAAA AAAAAAAAAAAAAAAAAAAAAAA A (185) |
| polyA-V88 | AAAAAAAAAAAAAAAAAAAA AAAAAAAAAGTGTAAAAAAA AAAAAAAAAAAAAAAAAAAA (86) | AAAAAAAAAAAAAAAAAAAAA AAAAAAAGUGUAAAAAAAAAA AAAAAAAAAAAAAAAA (186) |
| polyA-V89 | AAAAAAAAAAAAAAAAAA AAAAAAAAAGTGTAAAAA AAAAAAAAA (87) | AAAAAAAAAAAAAAAAAAAAAAA AAAAAAAGUGUAAAAAAAAAAAA AAAAAA (187) |
| polyA-V90 | AAAAAAAAAAAAAAAAAAAAA AAAAAAAAAGTGTAAAAAAAAA AAAAAAAAAAAAAAAAAAAAAA AAAAAAA (88) | AAAAAAAAAAAAAAAAAAAAAA AAAAAAAGUGUAAAAAAAAAAA AAAAAAAAAAAAAAAAAAAAAA AA (188) |
| polyA-V91 | AAAAAAAAAAAAAAAAAAA AAAAAAAAAGCAAATGACTAAA AAAAAAAAAAAAAAAAAAAAA AAAAAA (89) | AAAAAAAAAAAAAAAAAAAAAAA AAAAAAAGCAAAUGACUAAAAAA AAAAAAAAAAAAAAAAAAAAAAA A (189) |
| polyA-V92 | AAAAAAAAAAAAAAAAAA AAAAAAAAAGCAAATGACTAAA AAAAAAAAAAAAAAA (90) | AAAAAAAAAAAAAAAAAAAAAAA AAAAAAAGCAAAUGACUAAAAAA AAAAAAAAAAAAA (190) |
| polyA-V93 | AAAAAAAAAAAAAAAAAAAAA AAAAAAAAAGCAAATGACTAAA AAAAAAAAAAAAAAAAAAAAA AAAAAAAAAAAAAAA (91) | AAAAAAAAAAAAAAAAAAAAA AAAAAAAGCAAAUGACUAAAAAA AAAAAAAAAAAAAAAAAAAAA AAAAAAAA (191) |
| polyA-V94 | AAAAAAAAAAAAAAAGAAAA AAAAAAAAAGGCAAAAAAAA AAAAAAAAAAAAAAAAAAAA (92) | AAAAAAAAAAAAAAGAAAAAA AAAAAAAGGCAAAAAAAAAAA AAAAAAAAAAAAAAAAAA (192) |
| polyA-V95 | A A A A A A A A A A A A A A A A C A A A A | AAAAAAAAAAAAAAACAAAAAA |
| | AAAAAAAAGGCAAAAAAAAA AAAAAAAAAAAAAAAAAAAAA (93) | AAAAAAAGGCAAAAAAAAAAAAAA AAAAAAAAAAAAAAAAAAAAA (193) |
| polyA-V96 | AAAAAAAAAAAAAAATAAAA AAAAAAAAAGGCAAAAAAAA AAAAAAAAAAAAAAAAAAAA (94) | AAAAAAAAAAAAAAAAUAAAAAA AAAAAAAGGCAAAAAAAAAAAAA AAAAAAAAAAAAAAAAAAAA (194) |
| polyA-V97 | AAAAAAAAAAAAAAAAAAGAA AAAAAAAAAAAAAAAAGGCAA AAAAAAAAAAAAAAAAAAAAA AAAAAAA (95) | AAAAAAAAAAAAAAAAAAGAAAA AAAAAAAAAAAAAAAGGCAAAAA AAAAAAAAAAAAAAAAAAAAAAA A (195) |
| polyA-V98 | AAAAAAAAAAAAAAAAAACAA AAAAAAAAAAAAAAAAGGCAA AAAAAAAAAAAAAAAAAAAAA AAAAAAA (96) | AAAAAAAAAAAAAAAAAACAAAA AAAAAAAAAAAAAAGGCAAAAAA AAAAAAAAAAAAAAAAAAAAAAA A (196) |
| polyA-V99 | AAAAAAAAAAAAAAAAAATAA AAAAAAAAAAAAAAAAGGCAA AAAAAAAAAAAAAAAAAAAAA AAAAAAA (97) | AAAAAAAAAAAAAAAUAAAAA AAAAAAAAAAAAAGGCAAAAA AAAAAAAAAAAAAAAAAAAAA A (197) |
| polyA-V100 | AAAAAAAAAAAAAAAAAAGAA AAAAAAAAAAAAAAAAGGCAA AAAAAAAAAGAAAAAAAAAAA AAAAAAA (98) | AAAAAAAAAAAAAAAAAAGAAAA AAAAAAAAAAAAAAGGCAAAAAA AAAAAGAAAAAAAAAAAAAAAAA A (198) |
| polyA-V101 | AAAAAAAAAAAAAAAAAAACAA AAAAAAAAAAAAAAAAAGGCAA AAAAAAAAAACAAAAAAAAAAA AAAAAAA (99) | AAAAAAAAAAAAAAAAAAAAAACAAAA AAAAAAAAAAAAAAAAAAGGCAAAAAA AAAAACAAAAAAAAAAAAAAAAAAAAA A (199) |
| polyA-V102 | AAAAAAAAAAAAAAAAAAAAAATAA AAAAAAAAAAAAAAAAAAGGCAA AAAAAAAAAATAAAAAAAAAAAA AAAAAAA (100) | AAAAAAAAAAAAAAAAAAAAAAAAAAUAAAA AAAAAAAAAAAAAAAAAAAAAAGGCAAAAAA AAAAAUAAAAAAAAAAAAAAAAAAAAAAAAA A (200) |
| polyA-V103 | AAAAAAAAAAAAAAAAAAAAAAA AAAAAAAAGTGTAAAAAAAAAAA (213) | AAAAAAAAAAAAAAAAAAAAAAAA AAAAAAGUGUAAAAAAAAAAAA (249) |
| polyA-V104 | AAAAAAAAAAAAAAAAAAAA AAAAAAAAGTGTAAAAAAAAA AAAAAAAAAAAAAA (214) | AAAAAAAAAAAAAAAAAAAAAAAAA AAAAAAGUGUAAAAAAAAAAAAAAA AAAAAAAAAA(250) |
| polyA-V105 | AAAAAAAAAAAAAAAAAAAAAA AAAAAAAAGTGTAAAAAAAAAAA AAAAAAAAAAAAAAAAAAAAAAA (215) | AAAAAAAAAAAAAAAAAAAAAAA AAAAAAGUGUAAAAAAAAAAAAA AAAAAAAAAAAAAAAAAAAA (251) |
| polyA-V106 | AAAAAAAAAAAAAAAAAAAAAA AAAAAAAAGTGTAAAAAAAAAAAA AAAAAAAAAAAAAAAAAAAAAAAA AAAAAA (216) | AAAAAAAAAAAAAAAAAAAAAAAA AAAAAAGUGUAAAAAAAAAAAAAA AAAAAAAAAAAAAAAAAAAAAAAA A (252) |
| polyA-V107 | AAAAAAAAAAAAAAAAAAAAA AAAAAAAAGTGTAAAAAAAAA AAAAAAAAAAAAAAAAAAAAAA AAAAAAAA (217) | AAAAAAAAAAAAAAAAAAAAAAAA AAAAAAGUGUAAAAAAAAAAAAAA AAAAAAAAAAAAAAAAAAAAAAAA AAA (253) |
| polyA-V108 | AAAAAAAAAAAAAAAAAAAAA AAAAAAAAGTGTAAAAAAAAA AAAAAAAAAAAAAAAAAAAAA AAAAAAAAAAA (218) | AAAAAAAAAAAAAAAAAAAAAAAAA AAAAAAGUGUAAAAAAAAAAAAAAA AAAAAAAAAAAAAAAAAAAAAAAAA AAAAAA (254) |
| polyA-V109 | AAAAAAAAAAAAAAAAAAAAA AAAAAAAAGTGTAAAAAAAAAA AAAAAAAAAAAAAAAAAAAAAA AAAAAAAAAAAAAAAA (219) | AAAAAAAAAAAAAAAAAAAAAAAA AAAAAAGUGUAAAAAAAAAAAAAA AAAAAAAAAAAAAAAAAAAAAAAA AAAAAAAA (255) |
| polyA-V110 | AAAAAAAAAAAAAAAAAAAAA AAAAAAAAGTGTAAAAAAAAAA | AAAAAAAAAAAAAAAAAAAAAAAAA AAAAAAGUGUAAAAAAAAAAAAAAA |
| | AAAAAAAAAAAAAAAAAAAAAA AAAAAAAAAAAAAAAAAA (220) | AAAAAAAAAAAAAAAAAAAAAAAAAAA AAAAAAAAAAAA (256) |
| polyA-V111 | AAAAAAAAAAAAAAAAAAAAAAA AAAAAAAAGTGTAAAAAAAAAAAA AAAAAAAAAAAAAAAAAAAAAAAA AAAAAAAAAAAAAAAAAAAA (221) | AAAAAAAAAAAAAAAAAAAAAAA AAAAAAGUGUAAAAAAAAAAAAAA AAAAAAAAAAAAAAAAAAAAAAAA AAAAAAAAAAAAAAAA (257) |
| polyA-V112 | AAAAAAAAAAAAAAAAAAAAAA AAAAAAAAGTGTAAAAAAAAAAA AAAAAAAAAAAAAAAAAAAAAA AAAAAAAAAAAAAAAAAAAAAA (222) | AAAAAAAAAAAAAAAAAAAAAAAAAA AAAAAAGUGUAAAAAAAAAAAAAAA AAAAAAAAAAAAAAAAAAAAAAAAAA AAAAAAAAAAAAAAAAAA (258) |
| polyA-V113 | AAAAAAAAAAAAAAAAAAAAAA AAAAAAAAGTGTAAAAAAAAAAA AAAAAAAAAAAAAAAAAAAAAAA AAAAAAAAAAAAAAAAAAAAAAA AAAA (223) | AAAAAAAAAAAAAAAAAAAAAAAA AAAAAAGUGUAAAAAAAAAAAAAA AAAAAAAAAAAAAAAAAAAAAAAA AAAAAAAAAAAAAAAAAAAA (259) |
| polyA-V114 | AAAAAAAAAAAAAAAAAAAAAAA AAAAAAAAGTGTAAAAAAAAAAAA AAAAAAAAAAAAAAAAAAAAAAAA AAAAAAAAAAAAAAAAAAAAAAAA AAAAAA (224) | AAAAAAAAAAAAAAAAAAAAAAA AAAAAAGUGUAAAAAAAAAAAAA AAAAAAAAAAAAAAAAAAAAAAA AAAAAAAAAAAAAAAAAAAAAA (260) |
| polyA-V115 | AAAAAAAAAAAAAAAAAAAAAAA AAAAAAAAGTGTAAAAAAAAAAAA AAAAAAAAAAAAAAAAAAAAAAAA AAAAAAAAAAAAAAAAAAAAAAAA AAAAAAAA (225) | AAAAAAAAAAAAAAAAAAAAAAAA AAAAAAGUGUAAAAAAAAAAAAAA AAAAAAAAAAAAAAAAAAAAAAAA AAAAAAAAAAAAAAAAAAAAAAAA A (261) |
| polyA-V116 | AAAAAAAAAAAAAAAAAAAAAAA AAAAAAAAGTGTAAAAAAAAAAAA AAAAAAAAAAAAAAAAAAAAAAAA AAAAAAAAAAAAAAAAAAAAAAAA AAAAAAAAAAAAAAAAAAAAAA (226) | AAAAAAAAAAAAAAAAAAAAAAAA AAAAAAGUGUAAAAAAAAAAAAAA AAAAAAAAAAAAAAAAAAAAAAAA AAAAAAAAAAAAAAAAAAAAAAAA AAAAAAAAAAAAAA (262) |
| polyA-V117 | AAAAAAAAAAAAAAAAAAAAAAA AAAAAAAAAGTGTAAAAAAAAAAAA AAAAAAAAAAAAAAAAAAAAAAAA AAAAAAAAAAAAAAAAAAAAAAAA AAAAAAAAAAAAAAAAAAAAAAAA AA (227) | AAAAAAAAAAAAAAAAAAAAAAA AAAAAAGUGUAAAAAAAAAAAAA AAAAAAAAAAAAAAAAAAAAAAA AAAAAAAAAAAAAAAAAAAAAAA AAAAAAAAAAAAAAAAAAA (263) |
| polyA-V118 | AAAAAAAAAAAAAAAAAAAAA AAAAAAAAGTGTAAAAAAAAAA AAAAAAAAAAAAAAAAAAAAA AAAAAAAAAAAAAAAAAAAAA AAAAAAAAAAAAAAAAAAAAA AAAAAAA (228) | AAAAAAAAAAAAAAAAAAAAAAAA AAAAAAGUGUAAAAAAAAAAAAAA AAAAAAAAAAAAAAAAAAAAAAAA AAAAAAAAAAAAAAAAAAAAAAAA AAAAAAAAAAAAAAAAAAAAAAA (264) |
| polyA-V119 | AAAAAAAAAAAAAAAAAAAAAAA AAAAAAAAGTGTAAAAAAAAAAAA AAAAAAAAAAAAAAAAAAAAAAAA AAAAAAAAAAAAAAAAAAAAAAAA AAAAAAAAAAAAAAAAAAAAAAAA AAAAAAAAAAAAA (229) | AAAAAAAAAAAAAAAAAAAAAAAAAA AAAAAAGUGUAAAAAAAAAAAAAAAA AAAAAAAAAAAAAAAAAAAAAAAAAA AAAAAAAAAAAAAAAAAAAAAAAAAA AAAAAAAAAAAAAAAAAAAAAAAAAA AAA (265) |
| polyA-V120 | AAAAAAAAAAAAAAAAAAAAAA AAAAAAAAGTGTAAAAAAAAAAA AAAAAAAAAAAAAAAAAAAAAA AAAAAAAAAAAAAAAAAAAAAA AAAAAAAAAAAAAAAAAAAAAA AAAAAAAAAAAAAAAAAA (230) | AAAAAAAAAAAAAAAAAAAAAAAA AAAAAAGUGUAAAAAAAAAAAAAA AAAAAAAAAAAAAAAAAAAAAAAA AAAAAAAAAAAAAAAAAAAAAAAA AAAAAAAAAAAAAAAAAAAAAAAA AAAAAAAA (266) |
| polyA-V121 | AAAAAAAAAAAAAAAAAAAAAA AAAAAAAAGTGTAAAAAAAAAAA AAAAAAAAAAAAAAAAAAAAAA | AAAAAAAAAAAAAAAAAAAAAAAA AAAAAAGUGUAAAAAAAAAAAAAA AAAAAAAAAAAAAAAAAAAAAAAA |
| | AAAAAAAAAAAAAAAAAAAAAAAA AAAAAAAAAAAAAAAAAAAAAAAA AAAAAAAAAAAAAAAAAAAAAAAA AA (231) | AAAAAAAAAAAAAAAAAAAAAAAAAAA AAAAAAAAAAAAAAAAAAAAAAAAAAA AAAAAAAAAAAAAAAAA (267) |
| polyA-V122 | AAAAAAAAAAAAAAAAAAAA AAAAAAAAGGCAAAAAAAA (232) | AAAAAAAAAAAAAAAAAAAAAAAA AAAAAAGGCAAAAAAAA (268) |
| polyA-V123 | AAAAAAAAAAAAAAAAAAAAAAA AAAAAAAAGGCAAAAAAAAAAAA AAAAAAAAAAAAAAA (233) | AAAAAAAAAAAAAAAAAAAAAAAA AAAAAAGGCAAAAAAAAAAAAAAA AAAAAAAAAA (269) |
| polyA-V124 | AAAAAAAAAAAAAAAAAAAAAA AAAAAAAAGGCAAAAAAAAAAA AAAAAAAAAAAAAAAAAAAAAA A (234) | AAAAAAAAAAAAAAAAAAAAAAAA AAAAAAGGCAAAAAAAAAAAAAAA AAAAAAAAAAAAAAAAAAA (270) |
| polyA-V125 | AAAAAAAAAAAAAAAAAAAAA AAAAAAAAGGCAAAAAAAAAA AAAAAAAAAAAAAAAAAAAAA AAAAAA (235) | AAAAAAAAAAAAAAAAAAAAAAAAAA AAAAAAGGCAAAAAAAAAAAAAAAA AAAAAAAAAAAAAAAAAAAAAAAAAA (271) |
| polyA-V126 | AAAAAAAAAAAAAAAAAAAAA AAAAAAAAGGCAAAAAAAAAA AAAAAAAAAAAAAAAAAAAAA AAAAAAAA (236) | AAAAAAAAAAAAAAAAAAAAAAAAA AAAAAAGGCAAAAAAAAAAAAAAAA AAAAAAAAAAAAAAAAAAAAAAAAA AAA (272) |
| polyA-V127 | AAAAAAAAAAAAAAAAAAAAAA AAAAAAAAGGCAAAAAAAAAAA AAAAAAAAAAAAAAAAAAAAAAA AAAAAAAAAAAA (237) | AAAAAAAAAAAAAAAAAAAAAAA AAAAAAGGCAAAAAAAAAAAAAA AAAAAAAAAAAAAAAAAAAAAAA AAAAA (273) |
| polyA-V128 | AAAAAAAAAAAAAAAAAAAAAAAA AAAAAAAAGGCAAAAAAAAAAAAA AAAAAAAAAAAAAAAAAAAAAAAA AAAAAAAAAAAAAAAA (238) | AAAAAAAAAAAAAAAAAAAAAAAA AAAAAAGGCAAAAAAAAAAAAAAA AAAAAAAAAAAAAAAAAAAAAAAA AAAAAAAAA (274) |
| polyA-V129 | AAAAAAAAAAAAAAAAAAAAAA AAAAAAAAGGCAAAAAAAAAAA AAAAAAAAAAAAAAAAAAAAAAAA AAAAAAAAAAAAAAAAAA (239) | AAAAAAAAAAAAAAAAAAAAAAA AAAAAAGGCAAAAAAAAAAAAAA AAAAAAAAAAAAAAAAAAAAAAAA AAAAAAAAAAAAA (275) |
| polyA-V130 | AAAAAAAAAAAAAAAAAAAAAAA AAAAAAAAGGCAAAAAAAAAAAA AAAAAAAAAAAAAAAAAAAAAAAA AAAAAAAAAAAAAAAAAAAAAAA (240) | AAAAAAAAAAAAAAAAAAAAAAAAA AAAAAAGGCAAAAAAAAAAAAAAAA AAAAAAAAAAAAAAAAAAAAAAAAAA AAAAAAAAAAAAAAAA (276) |
| polyA-V131 | AAAAAAAAAAAAAAAAAAAAAAA AAAAAAAAGGCAAAAAAAAAAAA AAAAAAAAAAAAAAAAAAAAAAA AAAAAAAAAAAAAAAAAAAAAAA A (241) | AAAAAAAAAAAAAAAAAAAAAAAA AAAAAAGGCAAAAAAAAAAAAAAA AAAAAAAAAAAAAAAAAAAAAAAA AAAAAAAAAAAAAAAAAA (277) |
| polyA-V132 | AAAAAAAAAAAAAAAAAAAAAA AAAAAAAAGGCAAAAAAAAAAA AAAAAAAAAAAAAAAAAAAAAA AAAAAAAAAAAAAAAAAAAAAA AAAAAA (242) | AAAAAAAAAAAAAAAAAAAAAAAAA AAAAAAGGCAAAAAAAAAAAAAAAA AAAAAAAAAAAAAAAAAAAAAAAAA AAAAAAAAAAAAAAAAAAAAAAA (278) |
| polyA-V133 | AAAAAAAAAAAAAAAAAAAAAA AAAAAAAAGGCAAAAAAAAAAA AAAAAAAAAAAAAAAAAAAAAA AAAAAAAAAAAAAAAAAAAAAA AAAAAAAAA (243) | AAAAAAAAAAAAAAAAAAAAAAAAA AAAAAAGGCAAAAAAAAAAAAAAAA AAAAAAAAAAAAAAAAAAAAAAAAA AAAAAAAAAAAAAAAAAAAAAAAAA A (279) |
| polyA-V134 | AAAAAAAAAAAAAAAAAAAAAAA AAAAAAAAGGCAAAAAAAAAAAA AAAAAAAAAAAAAAAAAAAAAAA AAAAAAAAAAAAAAAAAAAAAAA AAAAAAAAAAAAAAAAAAAAAAA (244) | AAAAAAAAAAAAAAAAAAAAAAAAAA AAAAAAGGCAAAAAAAAAAAAAAAAA AAAAAAAAAAAAAAAAAAAAAAAAAA AAAAAAAAAAAAAAAAAAAAAAAAAA AAAAAAAAAAAAAAAA (280) |
| polyA-V135 | AAAAAAAAAAAAAAAAAAAAAA AAAAAAAAGGCAAAAAAAAAAA AAAAAAAAAAAAAAAAAAAAAA AAAAAAAAAAAAAAAAAAAAAA AAAAAAAAAAAAAAAAAAAAAA AAAAAAAA (245) | AAAAAAAAAAAAAAAAAAAAAAAAA AAAAAAGGCAAAAAAAAAAAAAAAA AAAAAAAAAAAAAAAAAAAAAAAAA AAAAAAAAAAAAAAAAAAAAAAAAA AAAAAAAAAAAAAAAAAAAAAAA (281) |
| polyA-V136 | AAAAAAAAAAAAAAAAAAAAAA AAAAAAAAGGCAAAAAAAAAAA AAAAAAAAAAAAAAAAAAAAAA AAAAAAAAAAAAAAAAAAAAAA AAAAAAAAAAAAAAAAAAAAAA AAAAAAAAAAAAAAA (246) | AAAAAAAAAAAAAAAAAAAAAAAAAA AAAAAAGGCAAAAAAAAAAAAAAAAA AAAAAAAAAAAAAAAAAAAAAAAAAA AAAAAAAAAAAAAAAAAAAAAAAAAA AAAAAAAAAAAAAAAAAAAAAAAAAA AAAA (282) |
| polyA-V137 | AAAAAAAAAAAAAAAAAAAAAA AAAAAAAAGGCAAAAAAAAAAA AAAAAAAAAAAAAAAAAAAAAA AAAAAAAAAAAAAAAAAAAAAA AAAAAAAAAAAAAAAAAAAAAA AAAAAAAAAAAAAAAAAAAAAA AAA (247) | AAAAAAAAAAAAAAAAAAAAAAAAA AAAAAAGGCAAAAAAAAAAAAAAAA AAAAAAAAAAAAAAAAAAAAAAAAA AAAAAAAAAAAAAAAAAAAAAAAAA AAAAAAAAAAAAAAAAAAAAAAAAA AAAAAAAAAAAAAAAAA (283) |

### Example 2: Construction of Plasmid H

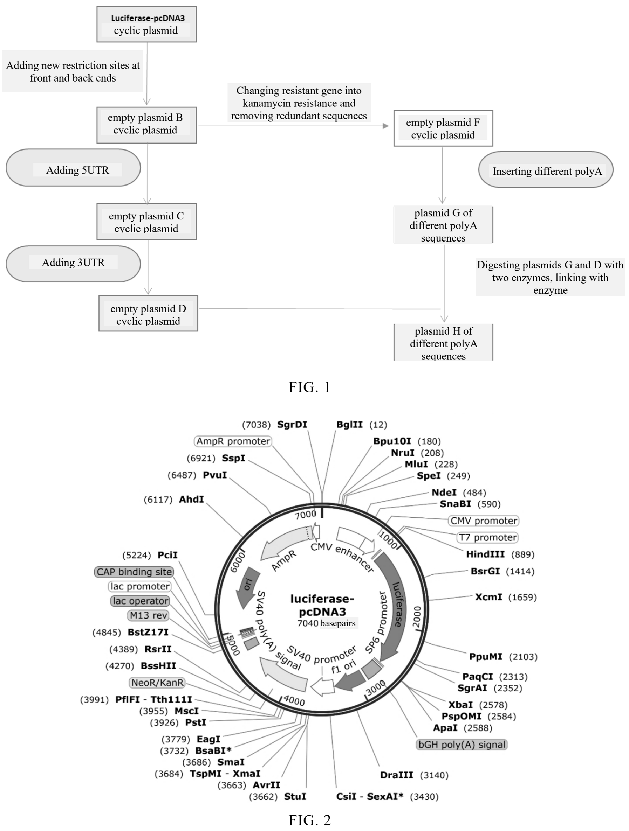

Utilizing In-Fusion cloning technique, using In-Fusion^{®} Snap Assembly Master Mix kit (Takara, catalog number: 638948) and Luciferase-pcDNA3 plasmid as backbone, plasmid B, plasmid C, plasmid D, plasmid F and plasmid G were constructed via homologous recombination, and the specific plasmid construction procedure was shown in FIG. 1.

### 2.1. Construction of Plasmid B

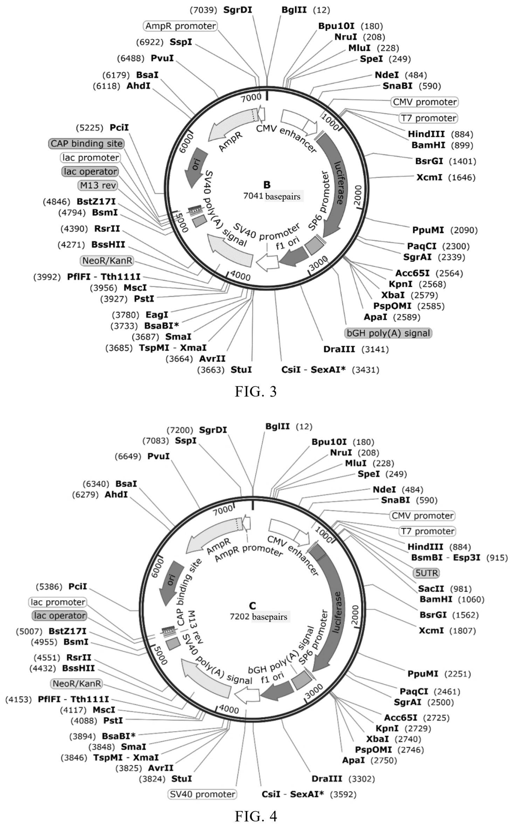

Using Luciferase-pcDNA3 plasmid (purchased from Addgene, plasmid number # 18964) as backbone, a restriction site was introduced to the primers, which was introduced into the Luciferase-pcDNA3 plasmid by homologous recombination using In-Fusion^{®} Snap Assembly Master Mix kit (Takara, Cat. No. 638948). Specifically, new HindIII and BamHI restriction sites were added upstream of the Kozack sequence of the Luciferase-pcDNA3 plasmid, and new KpnI and ApaI restriction sites were added at 3' end of the stop codon of luciferase, resulting in plasmid B. The plasmid map of luciferase-pcDNA3 is shown in FIG. 2, the plasmid map of plasmid B is shown in FIG. 3, and the nucleotide sequence of plasmid B is shown in SEQ ID NO: 201.

### 2.2. Construction of Plasmid C

A 5'-UTR sequence as set forth in SEQ ID NO: 202 was inserted between the restriction sites of HindIII and BamHI of plasmid B to give plasmid C.

Specifically, plasmid B was digested with HindIII (NEB, Cat. No. R3104L) and BamHI (NEB, Cat. No. R3136S), wherein the plasmid B was 2 µg, Cutsmart buff was 2 µL, each enzyme was 1 µL, and the water was 14 µL, and mixed well, and digested at 37°C for 30 min. After separation by agarose gel electrophoresis and using FastPure Gel DNA Extraction Mini Kit & 100 RNX/EA, DC301 purification and recovery kit of Vazyme to purify the vector backbone fragment of about 7 kb, the 5'-UTR sequence shown in SEQ ID NO: 202 was introduced into a homologous arm sequence via PCR, and then linked to the vector backbone by homologous recombination using the In-Fusion^{®} Snap Assembly Master Mix kit (Takara, catalog number: 638948) to generate plasmid C, and the plasmid map of plasmid C is shown in FIG. 4.

### 2.3. Construction of Plasmid D

A 3'-UTR sequence as set forth in SEQ ID NO: 203 was inserted between the restriction sites of KpnI and ApaI of plasmid C to give plasmid D.

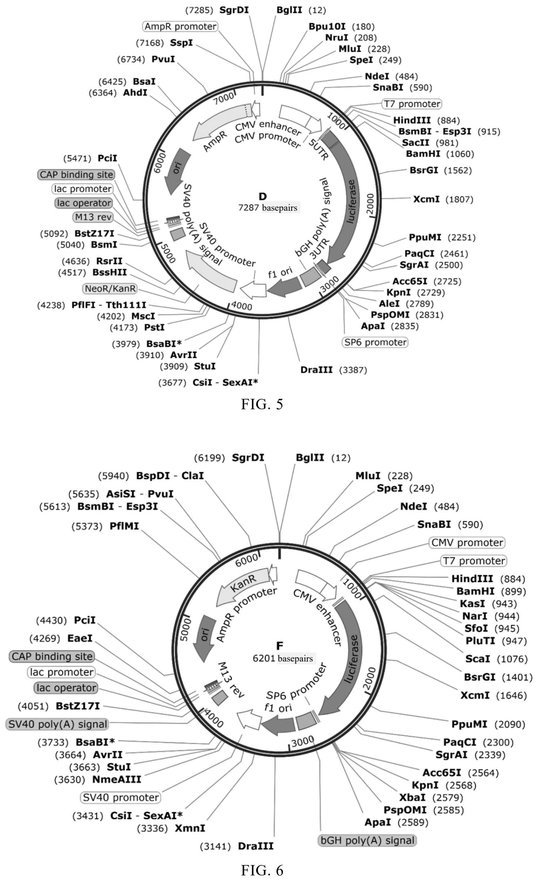

Specifically, the plasmid C was digested with KpnI (NEB Cat. No.: R3142S) and ApaI (NEB Cat. No.: R0114S), wherein the plasmid C was 2 µg, Cutsmart buff was 2 µL, each enzyme was 1 µL, and the water was 14 µL, mixed well, digested at 37°C for 30 min, separated by agarose gel electrophoresis and using the FastPure Gel DNA Extraction Mini Kit & 100 RNX/EA, DC301 purification and recovery kit of Vazyme to purify the vector backbone fragment of about 7 kb, the 3'-UTR sequence shown in SEQ ID NO: 203 was introduced into the homologous arm sequence by PCR, and then linked to the vector backbone via homologous recombination using the In-Fusion^{®} Snap Assembly Master Mix kit (Takara, catalog number: 638948) to generate plasmid D, and the plasmid map of the plasmid D is shown in FIG. 5.

### 2.4. Construction of Plasmid F

Primers were designed to amplify the backbone vector and the Kan resistant gene sequence, the Kan resistant gene sequence carries the homology arm of the plasmid B backbone, homologous recombination was performed by using the In-Fusion^{®} Snap Assembly Master Mix kit (Takara, catalog number: 638948), to replace the whole Amp (ampicillin) resistance gene in the plasmid B with a complete Kan (kanamycin) resistance gene. On this basis, the In-Fusion^{®} Snap Assembly Master Mix kit (Takara, catalog number: 638948) was used to remove the neoR/KanR sequence from 3746 to 4540 by homologous recombination to obtain plasmid F. The plasmid map of the plasmid F is shown in FIG. 6, and the nucleotide sequence of the plasmid F is shown in SEQ ID NO: 204.

### 2.5. Construction of Plasmid G Containing Different Poly(A)

Poly(A)s carrying the homology arm of the plasmid backbone were synthesized by the IDT, and then the different poly(A)s carrying the homology arm of the plasmid backbone were inserted into the plasmid F via homologous recombination, respectively, to obtain plasmid G corresponding to different poly(A)s. Specifically, the preparation of each plasmid G containing different poly(A)s were as follows: the plasmid F was digested with ApaI (NEB Cat. No.: R0114S), wherein the plasmid F was 2 µg, Cutsmart buffer was 2 µL, ApaI was 1 µL, and water was 15 µL, mixed well, digested at 37°C for 30 min, separated by agarose gel electrophoresis, and purified by the FastPure Gel DNA Extraction Mini Kit & 100 RNX/EA (Vazyme Cat. No.: DC301) purification and recovery kit to recover the vector backbone fragment of about 7 kb; then, the purified single enzyme digested product and the different poly(A) carrying the homologous arm of the plasmid backbone synthesized by IDT respectively were subjected to homologous recombination using the In-Fusion^{®} Snap Assembly Master Mix kit (Takara, catalog number: 638948), and the product was transformed into E. coli DH5α competent cells (TransGen), and the positive clones were screened out with LB plates containing kanamycin (50 mg/L) and sequenced to confirm that the correct plasmid was obtained.

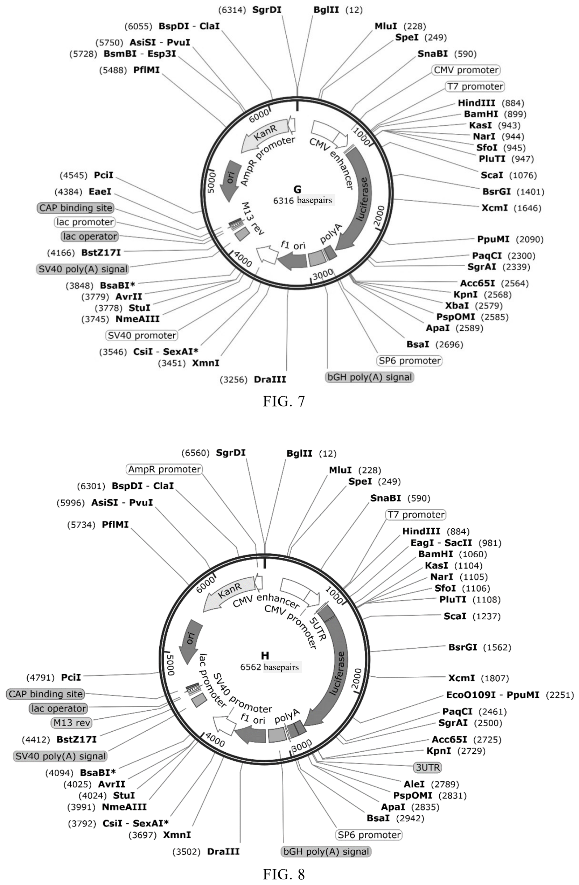

Taking the plasmid G inserted with polyA-V0 as an example, the plasmid map thereof is shown in FIG. 7, and the nucleotide sequence thereof is shown in SEQ ID NO: 205.

The sequencing results showed that plasmid G containing the polyA sequence shown in one of SEQ ID NOs: 1-100 and 212-247, respectively, was obtained.

### 2.6. Construction of Plasmid H Containing Different PolyA Sequences

Taking the construction of the plasmid H comprising polyA-V0 as an example, the construction of other plasmid Hs comprising other polyA sequences refers to that of plasmid H comprising the polyA-V0. Construction of the plasmid H comprising polyA-V0 comprises the following steps: digesting the plasmid G containing polyA-V0 using HindIII (NEB catalog number: R3104L) and ApaI (NEB catalog number: R0114S), wherein the plasmid G containing polyA-V0 was 2 µg, Cutsmart buff was 2 µL, each enzyme was 1 µL, and the water was 14 µL, mixed well and digested at 37°C for 30 min, separated by agarose gel electrophoresis, and purified by the FastPure Gel DNA Extraction Mini Kit & 100 RNX/EA (Vazyme Cat No. DC301) to purify the fragment A of about 4.6 kb. Plasmid D was digested with HindIII (NEB Cat. No.: R3104L) and ApaI (NEB Cat. No.: R0114S), wherein the plasmid D was 2 µg, Cutsmart buff was 2 ul, each enzyme was 1 µL, and the water was 14 µL, mixed well, digested at 37°C for 30 min, separated by agarose gel electrophoresis, and purified by the FastPure Gel DNA Extraction Mini Kit & 100 RNX/EA (Vazyme Cat. No.: DC301) to purify the fragment B of about 1.6 kb. Fragments A and B were ligated with T4 ligase (NEB catalog number: M2200L), wherein 50 ng of A fragment, 60 ng of B fragment, 1 µL of T4 enzyme, and ligation products were transformed into E. coli DH5α competent cells (TransGen), positive clones were screened out with LB plates containing kanamycin (50 mg/L) and sequenced to confirm that the correct H plasmid was obtained. The plasmid map of the plasmid H containing polyA-V0 is shown in FIG. 8, and the nucleotide sequence thereof is shown in SEQ ID NO: 206.

The sequencing results showed that plasmid Hs containing the polyA sequence of one of SEQ ID NOs: 1-100 and 212-247, respectively, were obtained.

### Example 3 Preparation of mRNA Containing the Poly(A) of the invention

In this example, mRNA was prepared by transcription using the plasmid H containing different poly(A)s constructed in Example 2.

### 3.1. Plasmid linearization

Plasmid samples with a Superhelical Density of 90% were used to ensure mRNA quality.

In a 0.2 mL tube, 20 µg of plasmid H was mixed with 1 µL of BsaI enzyme (NEB catalog number: R3733L), 8 µL Buffer and water, in a total volume of 80 µL. After mixing, it was incubated at 37°C for 2 h. 1 µL of the product was taken for agarose gel electrophoresis to verify whether the plasmid was completely linearized. After complete linearization, linearized plasmid G was recovered using Takara recovery kit.

Specifically, the linearized product was mixed with 3 × volume (240 µL) of Buffer DC. The Spin Column in the kit was placed on a Collection Tube. The mixed solution was transferred to the Spin Column, centrifuged at 12000 rpm for 1 minute at room temperature, and the filtrate was discarded. 700 µL of Buffer WB was added to the Spin Column, centrifuged at 12000 rpm for 30 seconds at room temperature, and the filtrate was discarded. repeating the previous step. The tube was then centrifuged for 2 min, and the excess alcohol was spin-dried. Finally, the mixture was eluted with 100 µL of Elution buffer and recovered to a new EP tube.

Phenol Chloroform Extraction (removing RNase, Proteins, etc.)

The plasmid to be extracted was diluted to 300 µL, and an equal volume of phenol chloroform (25:24:1) was added and mixed uniformly, centrifuged at 12000 rpm at room temperature for 10 min, then the supernatant liquid was collected as much as possible, and an equal volume of phenol chloroform was added, centrifuged at 12000 rpm and at 4°C for 10 min (after centrifugation, collecting upper liquid as much as possible (excluding the lower liquid), then 0.1 volume of 5 M NaCl and 0.7 volume of isopropanol were added, ice bath for 15 min, centrifuged at 4°C and 12000 rpm, and the supernatant was discarded.

The precipitate was washed once with 200 µL of 70% (v/v) ethanol (pre-ice bath), centrifuged at 4°C and 12000 rpm for 1 min, the supernatant was discarded, then 12000 rpm for 1 min, and the liquid was fully aspirated. After air drying at room temperature, 60 µL of UltraPure DNase/RNase-Free-Distilled Water was added and mixed uniformly, and the purified sample concentration was determined by OneDrop.

Plasmid linearization was confirmed by electrophoresis of 100 ng of purified digested plasmid and the original plasmid. If the electrophoretic indicated plasmid was completely linearized and there was not non-target band, the linearized plasmid can be used for mRNA synthesis.

### 3.2. In Vitro Transcription of mRNA

Reagents for in vitro transcription (IVT), purification, and identification of mRNA were purchased from Vazyme BioTech Co., Ltd.

A system for the in vitro transcription of mRNA was prepared on ice, comprising 5 µg of linearized plasmid, 10 µL of 10 × Reaction Buffer, 10 µL of ATP (75 mM), 10 µL of GTP (75 mM), 10 µL of CTP (75 mM), 7.5 µL of N1-Me-Pseudoo UTP (100 mM), 10 µL of T7 RNA polymerase, in a total volume of 100 µL, which were mixed by vortex and centrifuged slightly.) incubated at 37°C for 2h.

5 µL of DNase was added and incubated at 37°C for 15 min to remove linearized plasmid.

The IVT product was purified by magnetic beads (Vazyme Cat. No. N412-01). Specifically, the VAHTTS RNA Clean Beads were kept at room temperature to temperature equilibrium, vortexed to make the magnetic beads mixed well. The 1.8 × original RNA solution volume of VAHTS RNA Clean Beads was mixed well with the original RNA solution. Incubating at room temperature for 5 min to bind RNA to the magnetic beads. The mixture was placed on a magnetic rack for 5 min, and after the solution was clear, the supernatant was carefully removed.

The tube holding the bead-RNA sample was always in the magnetic rack, 200 µL of freshly prepared 80% (v/v) ethanol was added to rinse the magnetic beads twice, each for 30s, the supernatant was carefully removed; and then open-lid air-drying of magnetic beads for 5 min.

The tube was taken out of the magnetic rack, Nucleas-free H₂O was added and mixed well, and stood at room temperature for 5 min. Then, the sample was placed in a magnetic rack for 5 min, and after the solution was clear, the supernatant was carefully transferred to a new Nuclease free tube. After 10 times dilution, the concentration was determined by onedrop or Qubit.

Identification: 5 µL of diluted purified sample was mixed with the NorthernMax Formaldehyde Load Dye and incubated at 75°C for 10 min, and then placed on ice for storage. The gel electrophoresis was then performed using a 1% (m/V) agarose gel, showing that the obtained mRNA was correct in size and the band was free of dispersion.

### 3.3. mRNA Cap1 Capping Reaction:

The Cap1 (i.e., cap G¹G²) structure is as follows:
cap G¹G² = m⁷G-5'-ppp-5'-Gm2'-3'-p-, wherein m7 = 7-CH₃; m2' = 2'-O-CH₃; -ppp- = - PO₂H-O-PO₂H-O-PO₂H)-; -p- = -PO₂H-.

An aqueous solution of 67 µL mRNA (containing 25 pmol mRNA) was preheated at 65°C for 5 min and then mixed with a mixture containing 10 µL of 10 × ScriptCap Capping Buffer, 10 µL of 10 mM GTP, 2.5 µL of 20 mM SAM, 2.5 µL of ScriptGuard RNase Inhibitor, 4 µL of ScriptCap 2'-O-Methyltransferase (100U/µL) and 4 µL of ScriptCap Capping Enzyme (10 U/µL), and incubated at 37°C for 1h. Reagents for capping reactions were purchased from Cellscript.

The capped product was purified and determined for its concentration as described in Example 3, 3.2.

It was confirmed by mass spectrometry that luciferase-coding mRNA containing Cap 1 type cap structure and methyl pseudouracil modification was obtained and can be used for subsequent verification experiments in mice.

### Example 4 Effect of poly(A) on in vivo translation of mRNA

In this example, in-vivo imaging (IVIS) data were used to compare the effects of the poly(A) on the expression level of luciferase.

### 4.1 Preparation of LNP-mRNA Preparation:

(1) LNP encapsulation: the liquid volume (the total volume of the aqueous phase of each system and the alcohol phase thereof) was 1.5 mL, wherein the mass ratio of mRNA: lipid was 1:10, each mRNA with different polyA was prepared in Example 3, and the concentration of HAc-NaOAc buffer (0.2M, pH 5.0) in the final aqueous phase was 0.025M. The microfluidic device (MPE-L2) and the chip (SN. 000035) were used to prepare each sample in the aqueous phase: the alcohol phase = 9 mL/min: 3 mL/min, and the two phases were injected into the microfluidic chip according to the interface requirement for mixing. The aqueous phase contained 0.3 mg of mRNA, and the total volume was 1125 µL; the alcohol phase contained lipid (SM-102) dissolved in ethanol: DSPC: CHO-HP: M-DMG2000 (Mol%) = 50:10:38.5:1.5, with a total volume of 375 µL, wherein the chemical structure of SM-102 was as follow: 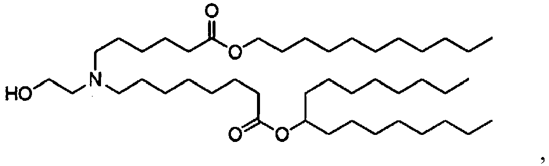 SM102 can be purchased commercially or prepared according to known techniques in the art.
(2) Dialysis to exchange fluid to prepare LNP-mRNA preparation:
   a. Preparation of dialysate
      1× PBS+8% (m/V) sucrose solution: 2 packages of 1 × PBS pre-prepared powder were put into a beaker, mixed uniformly with 2 L of DEPC water, 160g of sucrose was added, and mixed uniformly to obtain 1 × PBS+8% (m/V) sucrose solution.
   b. Dialysis: the solutions of each group were respectively put into a 100 KD dialysis bag, immersed in a beaker containing 1 L of dialysis solution, the beaker was wrapped with aluminum foil paper and dialyzed at 100 rpm for 1 h at room temperature, and the dialysis solution was changed for dialysis for 1 hour.

4.2 Female BALB/C mice were fed under an SPF condition laboratory setting, 5 weeks old (purchased from Vital River). Each LNP-mRNA preparation prepared in 4.1 was administered to a corresponding group of mice (n = 3) by tail vein injection, respectively, and 12 µg of LNP-mRNA preparation was injected to each mouse. After 12 h, mice were anesthetized by inhaling isoflurane and luciferase developed substrate D-Luciferin (150 mg/kg) was injected. The animals were placed in a supine position, and the signal distribution and strength of luciferin in mice were observed with an IVIS in vivo imaging system.

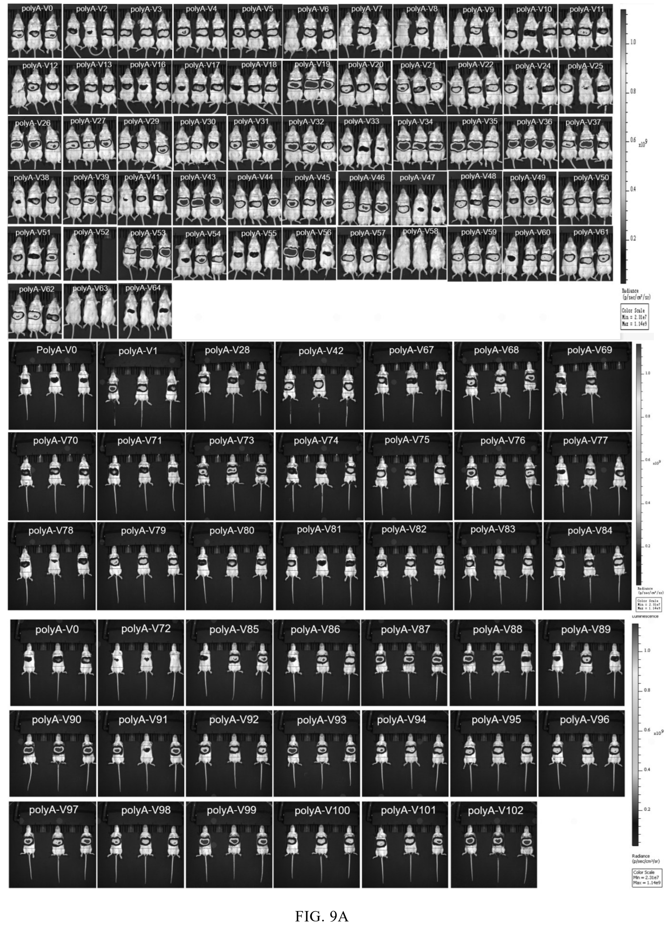

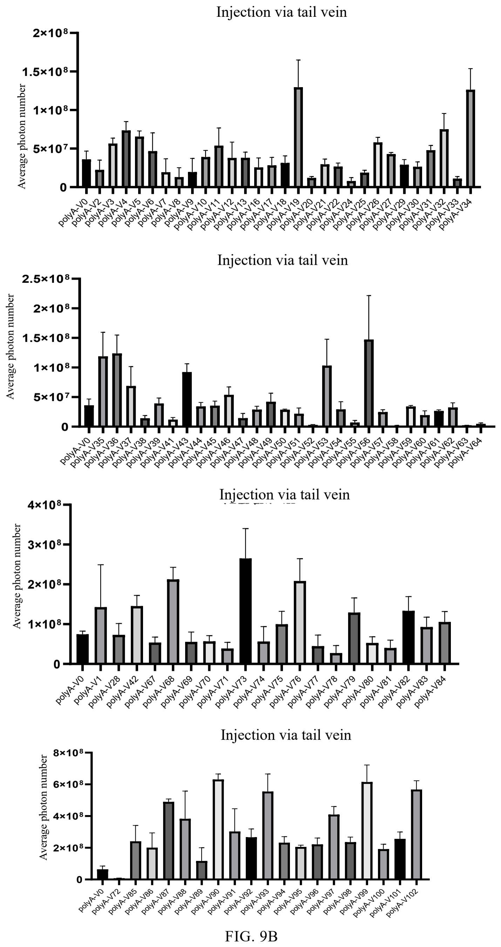

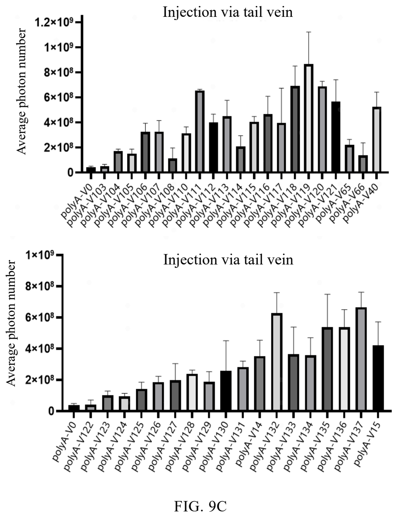

The results are shown in FIG. 9A, FIG. 9B, FIG. 9C and Tables 2-4, FIGs. 9A and 9B are images observed with IVIS, FIG. 9A is the original picture, FIG. 9B and FIG. 9C are comparison diagrams of results after the bar values of photon numbers are unified, and the protein expression ability in the liver is represented by fluorescence values. The unit of the ordinate average photon number in FIG. 9B and FIG. 9C is p/s/cm²/sr.

Experimental results showed that, compared with mice injected with mRNA containing polyA-V0, mice injected with mRNA comprising polyA-V1, polyA-V3, polyA-V4, polyA-V5, polyA-V6, polyA-V10, polyA-V12, polyA-V13, polyA-V19, polyA-V26, polyA-V27, polyA-V31, polyA-V32, polyA-V34, polyA-V35, polyA-V36, polyA-V37, polyA-V38, polyA-V42, polyA-V43, polyA-V46, polyA-V49, polyA-V53, polyA-V56, polyA-V68, polyA-V73, polyA-V75, polyA-V76, polyA-V79, polyA-V82, polyA-V83, polyA-V84, polyA-V85, polyA-V86, polyA-V87, polyA-V88, polyA-V89, polyA-V90, polyA-V91, polyA-V92, polyA-V93, polyA-V94, polyA-V95, polyA-V96, polyA-V97, polyA-V98, polyA-V99, polyA-V100, polyA-V101 or poly A-V102 had higher fluorescence intensity, and showed higher expression levels of luciferase protein.

### Example 5 Effect of poly(A) on mRNA Yield

Plasmid template H containing different poly(A) was linearized as described in 3.1 of Example 3, and the mRNA corresponding to different poly(A) was obtained by in vitro transcription as described in 3.2 of Example 3, and the obtained mRNA was measured by onedrop or Qubit, respectively, to determine the yield of mRNA.

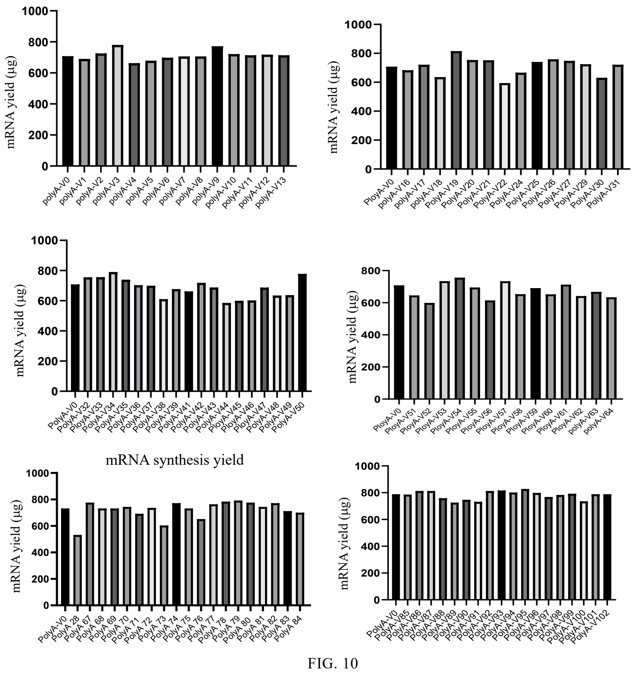

The results of yield of mRNA corresponding to different poly(A) are shown in FIG. 10. It can be seen from the experimental results that compared with the mRNA containing polyA-V0, the mRNA containing polyA-V2, polyA-V3, polyA-V9, polyA-V10, polyA-V11, polyA-V12, polyA-V13, polyA-V17, polyA-V19, polyA-V20, polyA-V21, polyA-V25, polyA-V26, polyA-V27, polyA-V29, polyA-V31, polyA-V32, polyA-V33, polyA-V34, polyA-V35, polyA-V36, polyA-V50, polyA-V53, polyA-V54, polyA-V57, polyA-V67, polyA-V68, polyA-V69, polyA-V70, polyA-V72, polyA-V74, polyA-V75, polyA-V77, polyA-V78, polyA-V79, polyA-V80, polyA-V81, polyA-V82, polyA-V85, polyA-V86, polyA-V87, polyA-V92, polyA-V93, polyA-V94, polyA-V95, polyA-V96, polyA-V97, polyA-V98, polyA-V99, polyA-V101, polyA-V102 had higher yield.

### Example 6 Stability of poly(A)

After the different poly(A) in Example 1 was inserted into the ApaI restriction site of plasmid F, a series of different plasmids in which the sequence of other parts was the same while the poly(A) was different.

The obtained plasmid product was transformed into E. coli DH5α competent cells (TransGen) which were then spread on LB plates containing kanamycin (50 mg/L), and incubated overnight at 37°C. 15 single colonies were picked up for each poly(A), and plasmids were extracted. The extracted 15 plasmid samples were sequenced (Biotech (Shanghai) Co., Ltd.) to determine whether poly(A) in the plasmid was consistent with the designed sequence, thereby evaluating the stability of poly(A).

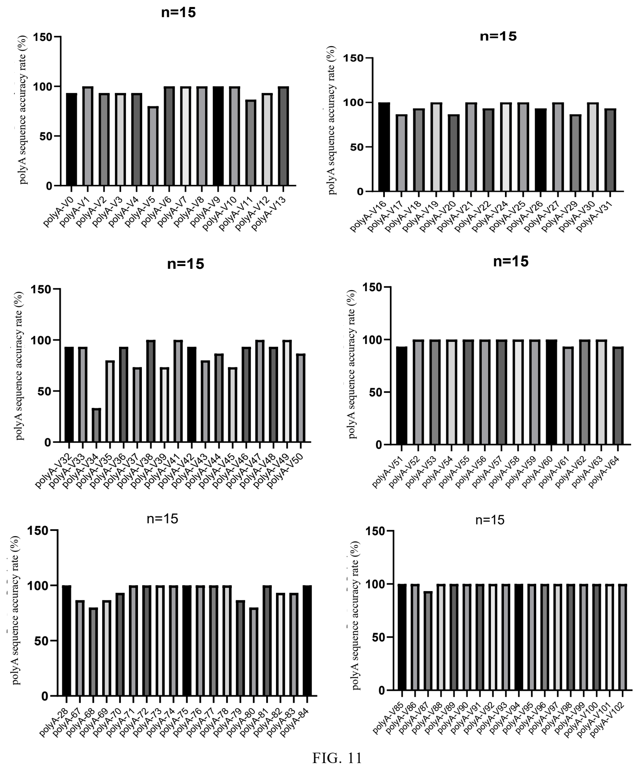

As shown in FIG. 11, it can be seen from FIG. 11 that the sequencing results of the 15 single colonies of each of the following poly(A)s showed that the sequence accuracy is 100%, which is completely consistent with the theoretical sequence: polyA-V1, polyA-V6, polyA-V7, polyA-V8, polyA-V9, polyA-V10, polyA-V13, polyA-V16, polyA-V19, polyA-V21, polyA-V24, polyA-V25, polyA-V27, polyA-V28, polyA-V30, polyA-V38, polyA-V41, polyA-V47, polyA-V49, polyA-V52, polyA-V53, polyA-V54, polyA-V55, polyA-V56, polyA-V57, polyA-V58, polyA-V59, polyA-V60, polyA-V62, polyA-V63, polyA-V71, polyA-V72, polyA-V73, polyA-V74, polyA-V75, polyA-V76, polyA-V77, polyA-V78, polyA-V81, polyA-V84, polyA-V85, polyA-V86, polyA-V88, polyA-V89, polyA-V90, polyA-V91, polyA-V92, polyA-V93, polyA-V94, polyA-V95, polyA-V96, polyA-V97, polyA-V98, polyA-V99, polyA-V100, polyA-V101 and polyA-V102, indicating that each of the above poly(A)s has lower recombination rate in the plasmid and higher stability.

In summary, the poly(A) of the invention not only has high stability, but also has obvious improvement in the yield, stability and/or protein expression level of transcribed mRNA, so that the production of mRNA can be performed quickly and efficiently.

### Sequence listing

5'-UTR (SEO ID NO. 202): 
TAATACGACTCACTATAGGACAGATCGCCTGGAGACGCCATCCACGCTGTTTTGACCTCCATAG
AAGACACCGGGACCGATCCAGCCTCCGCGGCCGGGAACGGTGCATTGGAACGCGGATTCCCC
GTGCCAAGAGTGACGTAAGTACCGCAGTCACCGTCCTTGACACG。
3'-UTR ( SEQ ID NO. 203): 
CGGGTGGCATCCCTGTGACCCCTCCCCAGTGCCTCTCCTGGCCCTGGAAGTTGCCACTCCAGT
GCCCACCAGCCTTGTCCTAATAAAATTAAGTTGCATC。
5'-UTR ( SEQ ID NO. 210, RNA): 
UAAUACGACUCACUAUAGGACAGAUCGCCUGGAGACGCCAUCCACGCUGUUUUGACCUCCA
UAGAAGACACCGGGACCGAUCCAGCCUCCGCGGCCGGGAACGGUGCAUUGGAACGCGGAUU
CCCCGUGCCAAGAGUGACGUAAGUACCGCAGUCACCGUCCUUGACACG。
3'-UTR (SEQ ID NO. 211, RNA): 
CGGGUGGCAUCCCUGUGACCCCUCCCCAGUGCCUCUCCUGGCCCUGGAAGUUGCCACUCCA
GUGCCCACCAGCCUUGUCCUAAUAAAAUUAAGUUGCAUC。
Plasmid B (SEQ ID NO. 201): 
GACGGATCGGGAGATCTCCCGATCCCCTATGGTGCACTCTCAGTACAATCTGCTCTGA
TGCCGCATAGTTAAGCCAGTATCTGCTCCCTGCTTGTGTGTTGGAGGTCGCTGAGTAGTGC
GCGAGCAAAATTTAAGCTACAACAAGGCAAGGCTTGACCGACAATTGCATGAAGAATCT
GCTTAGGGTTAGGCGTTTTGCGCTGCTTCGCGATGTACGGGCCAGATATACGCGTTGACAT
TGATTATTGACTAGTTATTAATAGTAATCAATTACGGGGTCATTAGTTCATAGCCCATATA
TGGAGTTCCGCGTTACATAACTTACGGTAAATGGCCCGCCTGGCTGACCGCCCAACGACC
CCCGCCCATTGACGTCAATAATGACGTATGTTCCCATAGTAACGCCAATAGGGACTTTCC
ATTGACGTCAATGGGTGGAGTATTTACGGTAAACTGCCCACTTGGCAGTACATCAAGTGT
ATCATATGCCAAGTACGCCCCCTATTGACGTCAATGACGGTAAATGGCCCGCCTGGCATT
ATGCCCAGTACATGACCTTATGGGACTTTCCTACTTGGCAGTACATCTACGTATTAGTCAT
CGCTATTACCATGGTGATGCGGTTTTGGCAGTACATCAATGGGCGTGGATAGCGGTTTGA
CTCACGGGGATTTCCAAGTCTCCACCCCATTGACGTCAATGGGAGTTTGTTTTGGCACCAA
AATCAACGGGACTTTCCAAAATGTCGTAACAACTCCGCCCCATTGACGCAAATGGGCGGT
AGGCGTGTACGGTGGGAGGTCTATATAAGCAGAGCTCTCTGGCTAACTAGAGAACCCACT
GCTTACTGGCTTATCGAAATTAATACGACTCACTATAGGGAAAGCTTGATCAGAACGGAT
CCGCCACCATGGAAGACGCCAAAAACATAAAGAAAGGCCCGGCGCCATTCTATCCGCTG
GAAGATGGAACCGCTGGAGAGCAACTGCATAAGGCTATGAAGAGATACGCCCTGGTTCCT
GGAACAATTGCTTTTACAGATGCACATATCGAGGTGGACATCACTTACGCTGAGTACTTC
GAAATGTCCGTTCGGTTGGCAGAAGCTATGAAACGATATGGGCTGAATACAAATCACAGA
ATCGTCGTATGCAGTGAAAACTCTCTTCAATTCTTTATGCCGGTGTTGGGCGCGTTATTTA
TCGGAGTTGCAGTTGCGCCCGCGAACGACATTTATAATGAACGTGAATTGCTCAACAGTA
TGGGCATTTCGCAGCCTACCGTGGTGTTCGTTTCCAAAAAGGGGTTGCAAAAAATTTTGA
ACGTGCAAAAAAAGCTCCCAATCATCCAAAAAATTATTATCATGGATTCTAAAACGGATT
ACCAGGGATTTCAGTCGATGTACACGTTCGTCACATCTCATCTACCTCCCGGTTTTAATGA
ATACGATTTTGTGCCAGAGTCCTTCGATAGGGACAAGACAATTGCACTGATCATGAACTC
CTCTGGATCTACTGGTCTGCCTAAAGGTGTCGCTCTGCCTCATAGAACTGCCTGCGTGAGA
TTCTCGCATGCCAGAGATCCTATTTTTGGCAATCAAATCATTCCGGATACTGCGATTTTAA
GTGTTGTTCCATTCCATCACGGTTTTGGAATGTTTACTACACTCGGATATTTGATATGTGG
ATTTCGAGTCGTCTTAATGTATAGATTTGAAGAAGAGCTGTTTCTGAGGAGCCTTCAGGAT
TACAAGATTCAAAGTGCGCTGCTGGTGCCAACCCTATTCTCCTTCTTCGCCAAAAGCACTC
TGATTGACAAATACGATTTATCTAATTTACACGAAATTGCTTCTGGTGGCGCTCCCCTCTC
 
TAAGGAAGTCGGGGAAGCGGTTGCCAAGAGGTTCCATCTGCCAGGTATCAGGCAAGGAT
ATGGGCTCACTGAGACTACATCAGCTATTCTGATTACACCCGAGGGGGATGATAAACCGG
GCGCGGTCGGTAAAGTTGTTCCATTTTTTGAAGCGAAGGTTGTGGATCTGGATACCGGGA
AAACGCTGGGCGTTAATCAAAGAGGCGAACTGTGTGTGAGAGGTCCTATGATTATGTCCG
GTTATGTAAACAATCCGGAAGCGACCAACGCCTTGATTGACAAGGATGGATGGCTACATT
CTGGAGACATAGCTTACTGGGACGAAGACGAACACTTCTTCATCGTTGACCGCCTGAAGT
CTCTGATTAAGTACAAAGGCTATCAGGTGGCTCCCGCTGAATTGGAATCCATCTTGCTCCA
ACACCCCAACATCTTCGACGCAGGTGTCGCAGGTCTTCCCGACGATGACGCCGGTGAACT
TCCCGCCGCCGTTGTTGTTTTGGAGCACGGAAAGACGATGACGGAAAAAGAGATCGTGGA
TTACGTCGCCAGTCAAGTAACAACCGCGAAAAAGTTGCGCGGAGGAGTTGTGTTTGTGGA
CGAAGTACCGAAAGGTCTTACCGGAAAACTCGACGCAAGAAAAATCAGAGAGATCCTCA
TAAAGGCCAAGAAGGGCGGAAAGATCGCCGTGTAAGGTACCCGATCAGATTCTAGAGGG
CCCTATTCTATAGTGTCACCTAAATGCTAGAGCTCGCTGATCAGCCTCGACTGTGCCTTCT
AGTTGCCAGCCATCTGTTGTTTGCCCCTCCCCCGTGCCTTCCTTGACCCTGGAAGGTGCCA
CTCCCACTGTCCTTTCCTAATAAAATGAGGAAATTGCATCGCATTGTCTGAGTAGGTGTCA
TTCTATTCTGGGGGGTGGGGTGGGGCAGGACAGCAAGGGGGAGGATTGGGAAGACAATA
GCAGGCATGCTGGGGATGCGGTGGGCTCTATGGCTTCTGAGGCGGAAAGAACCAGCTGG
GGCTCTAGGGGGTATCCCCACGCGCCCTGTAGCGGCGCATTAAGCGCGGCGGGTGTGGTG
GTTACGCGCAGCGTGACCGCTACACTTGCCAGCGCCCTAGCGCCCGCTCCTTTCGCTTTCT
TCCCTTCCTTTCTCGCCACGTTCGCCGGCTTTCCCCGTCAAGCTCTAAATCGGGGGCTCCCT
TTAGGGTTCCGATTTAGTGCTTTACGGCACCTCGACCCCAAAAAACTTGATTAGGGTGATG
GTTCACGTAGTGGGCCATCGCCCTGATAGACGGTTTTTCGCCCTTTGACGTTGGAGTCCAC
GTTCTTTAATAGTGGACTCTTGTTCCAAACTGGAACAACACTCAACCCTATCTCGGTCTAT
TCTTTTGATTTATAAGGGATTTTGCCGATTTCGGCCTATTGGTTAAAAAATGAGCTGATTT
AACAAAAATTTAACGCGAATTAATTCTGTGGAATGTGTGTCAGTTAGGGTGTGGAAAGTC
CCCAGGCTCCCCAGCAGGCAGAAGTATGCAAAGCATGCATCTCAATTAGTCAGCAACCAG
GTGTGGAAAGTCCCCAGGCTCCCCAGCAGGCAGAAGTATGCAAAGCATGCATCTCAATTA
GTCAGCAACCATAGTCCCGCCCCTAACTCCGCCCATCCCGCCCCTAACTCCGCCCAGTTCC
GCCCATTCTCCGCCCCATGGCTGACTAATTTTTTTTATTTATGCAGAGGCCGAGGCCGCCT
CTGCCTCTGAGCTATTCCAGAAGTAGTGAGGAGGCTTTTTTGGAGGCCTAGGCTTTTGCAA
AAAGCTCCCGGGAGCTTGTATATCCATTTTCGGATCTGATCAAGAGACAGGATGAGGATC
GTTTCGCATGATTGAACAAGATGGATTGCACGCAGGTTCTCCGGCCGCTTGGGTGGAGAG
GCTATTCGGCTATGACTGGGCACAACAGACAATCGGCTGCTCTGATGCCGCCGTGTTCCG
GCTGTCAGCGCAGGGGCGCCCGGTTCTTTTTGTCAAGACCGACCTGTCCGGTGCCCTGAAT
GAACTGCAGGACGAGGCAGCGCGGCTATCGTGGCTGGCCACGACGGGCGTTCCTTGCGCA
GCTGTGCTCGACGTTGTCACTGAAGCGGGAAGGGACTGGCTGCTATTGGGCGAAGTGCCG
GGGCAGGATCTCCTGTCATCTCACCTTGCTCCTGCCGAGAAAGTATCCATCATGGCTGATG
CAATGCGGCGGCTGCATACGCTTGATCCGGCTACCTGCCCATTCGACCACCAAGCGAAAC
ATCGCATCGAGCGAGCACGTACTCGGATGGAAGCCGGTCTTGTCGATCAGGATGATCTGG
ACGAAGAGCATCAGGGGCTCGCGCCAGCCGAACTGTTCGCCAGGCTCAAGGCGCGCATG
CCCGACGGCGAGGATCTCGTCGTGACCCATGGCGATGCCTGCTTGCCGAATATCATGGTG
GAAAATGGCCGCTTTTCTGGATTCATCGACTGTGGCCGGCTGGGTGTGGCGGACCGCTAT
CAGGACATAGCGTTGGCTACCCGTGATATTGCTGAAGAGCTTGGCGGCGAATGGGCTGAC
CGCTTCCTCGTGCTTTACGGTATCGCCGCTCCCGATTCGCAGCGCATCGCCTTCTATCGCC
TTCTTGACGAGTTCTTCTGAGCGGGACTCTGGGGTTCGAAATGACCGACCAAGCGACGCC
CAACCTGCCATCACGAGATTTCGATTCCACCGCCGCCTTCTATGAAAGGTTGGGCTTCGGA
ATCGTTTTCCGGGACGCCGGCTGGATGATCCTCCAGCGCGGGGATCTCATGCTGGAGTTCT
TCGCCCACCCCAACTTGTTTATTGCAGCTTATAATGGTTACAAATAAAGCAATAGCATCAC
AAATTTCACAAATAAAGCATTTTTTTCACTGCATTCTAGTTGTGGTTTGTCCAAACTCATC
AATGTATCTTATCATGTCTGTATACCGTCGACCTCTAGCTAGAGCTTGGCGTAATCATGGT
CATAGCTGTTTCCTGTGTGAAATTGTTATCCGCTCACAATTCCACACAACATACGAGCCGG
AAGCATAAAGTGTAAAGCCTGGGGTGCCTAATGAGTGAGCTAACTCACATTAATTGCGTT
GCGCTCACTGCCCGCTTTCCAGTCGGGAAACCTGTCGTGCCAGCTGCATTAATGAATCGG
CCAACGCGCGGGGAGAGGCGGTTTGCGTATTGGGCGCTCTTCCGCTTCCTCGCTCACTGA
CTCGCTGCGCTCGGTCGTTCGGCTGCGGCGAGCGGTATCAGCTCACTCAAAGGCGGTAAT
ACGGTTATCCACAGAATCAGGGGATAACGCAGGAAAGAACATGTGAGCAAAAGGCCAGC
AAAAGGCCAGGAACCGTAAAAAGGCCGCGTTGCTGGCGTTTTTCCATAGGCTCCGCCCCC
CTGACGAGCATCACAAAAATCGACGCTCAAGTCAGAGGTGGCGAAACCCGACAGGACTA
TAAAGATACCAGGCGTTTCCCCCTGGAAGCTCCCTCGTGCGCTCTCCTGTTCCGACCCTGC
CGCTTACCGGATACCTGTCCGCCTTTCTCCCTTCGGGAAGCGTGGCGCTTTCTCATAGCTC
 
ACGCTGTAGGTATCTCAGTTCGGTGTAGGTCGTTCGCTCCAAGCTGGGCTGTGTGCACGA
ACCCCCCGTTCAGCCCGACCGCTGCGCCTTATCCGGTAACTATCGTCTTGAGTCCAACCCG
GTAAGACACGACTTATCGCCACTGGCAGCAGCCACTGGTAACAGGATTAGCAGAGCGAG
GTATGTAGGCGGTGCTACAGAGTTCTTGAAGTGGTGGCCTAACTACGGCTACACTAGAAG
AACAGTATTTGGTATCTGCGCTCTGCTGAAGCCAGTTACCTTCGGAAAAAGAGTTGGTAG
CTCTTGATCCGGCAAACAAACCACCGCTGGTAGCGGTGGTTTTTTTGTTTGCAAGCAGCAG
ATTACGCGCAGAAAAAAAGGATCTCAAGAAGATCCTTTGATCTTTTCTACGGGGTCTGAC
GCTCAGTGGAACGAAAACTCACGTTAAGGGATTTTGGTCATGAGATTATCAAAAAGGATC
TTCACCTAGATCCTTTTAAATTAAAAATGAAGTTTTAAATCAATCTAAAGTATATATGAGT
AAACTTGGTCTGACAGTTACCAATGCTTAATCAGTGAGGCACCTATCTCAGCGATCTGTCT
ATTTCGTTCATCCATAGTTGCCTGACTCCCCGTCGTGTAGATAACTACGATACGGGAGGGC
TTACCATCTGGCCCCAGTGCTGCAATGATACCGCGAGACCCACGCTCACCGGCTCCAGAT
TTATCAGCAATAAACCAGCCAGCCGGAAGGGCCGAGCGCAGAAGTGGTCCTGCAACTTTA
TCCGCCTCCATCCAGTCTATTAATTGTTGCCGGGAAGCTAGAGTAAGTAGTTCGCCAGTTA
ATAGTTTGCGCAACGTTGTTGCCATTGCTACAGGCATCGTGGTGTCACGCTCGTCGTTTGG
TATGGCTTCATTCAGCTCCGGTTCCCAACGATCAAGGCGAGTTACATGATCCCCCATGTTG
TGCAAAAAAGCGGTTAGCTCCTTCGGTCCTCCGATCGTTGTCAGAAGTAAGTTGGCCGCA
GTGTTATCACTCATGGTTATGGCAGCACTGCATAATTCTCTTACTGTCATGCCATCCGTAA
GATGCTTTTCTGTGACTGGTGAGTACTCAACCAAGTCATTCTGAGAATAGTGTATGCGGCG
ACCGAGTTGCTCTTGCCCGGCGTCAATACGGGATAATACCGCGCCACATAGCAGAACTTT
AAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACT
TTCACCAGCGTTTCTGGGTGAGCAAAAACAGGAAGGCAAAATGCCGCAAAAAAGGGAAT
AAGGGCGACACGGAAATGTTGAATACTCATACTCTTCCTTTTTCAATATTATTGAAGCATT
TATCAGGGTTATTGTCTCATGAGCGGATACATATTTGAATGTATTTAGAAAAATAAACAA
ATAGGGGTTCCGCGCACATTTCCCCGAAAAGTGCCACCTGACGTC。
Plasmid F ( SEQ ID NO. 204): 
GACGGATCGGGAGATCTCCCGATCCCCTATGGTGCACTCTCAGTACAATCTGCTCTGATGC
CGCATAGTTAAGCCAGTATCTGCTCCCTGCTTGTGTGTTGGAGGTCGCTGAGTAGTGCGCGAGC
AAAATTTAAGCTACAACAAGGCAAGGCTTGACCGACAATTGCATGAAGAATCTGCTTAGGGTT
AGGCGTTTTGCGCTGCTTCGCGATGTACGGGCCAGATATACGCGTTGACATTGATTATTGACTAG
TTATTAATAGTAATCAATTACGGGGTCATTAGTTCATAGCCCATATATGGAGTTCCGCGTTACATA
ACTTACGGTAAATGGCCCGCCTGGCTGACCGCCCAACGACCCCCGCCCATTGACGTCAATAATG
ACGTATGTTCCCATAGTAACGCCAATAGGGACTTTCCATTGACGTCAATGGGTGGAGTATTTACG
GTAAACTGCCCACTTGGCAGTACATCAAGTGTATCATATGCCAAGTACGCCCCCTATTGACGTCA
ATGACGGTAAATGGCCCGCCTGGCATTATGCCCAGTACATGACCTTATGGGACTTTCCTACTTGG
CAGTACATCTACGTATTAGTCATCGCTATTACCATGGTGATGCGGTTTTGGCAGTACATCAATGG
GCGTGGATAGCGGTTTGACTCACGGGGATTTCCAAGTCTCCACCCCATTGACGTCAATGGGAGT
TTGTTTTGGCACCAAAATCAACGGGACTTTCCAAAATGTCGTAACAACTCCGCCCCATTGACGC
AAATGGGCGGTAGGCGTGTACGGTGGGAGGTCTATATAAGCAGAGCTCTCTGGCTAACTAGAG
AACCCACTGCTTACTGGCTTATCGAAATTAATACGACTCACTATAGGGAAAGCTTGATCAGAAC
GGATCCGCCACCATGGAAGACGCCAAAAACATAAAGAAAGGCCCGGCGCCATTCTATCCGCTG
GAAGATGGAACCGCTGGAGAGCAACTGCATAAGGCTATGAAGAGATACGCCCTGGTTCCTGGA
ACAATTGCTTTTACAGATGCACATATCGAGGTGGACATCACTTACGCTGAGTACTTCGAAATGTC
CGTTCGGTTGGCAGAAGCTATGAAACGATATGGGCTGAATACAAATCACAGAATCGTCGTATGC
AGTGAAAACTCTCTTCAATTCTTTATGCCGGTGTTGGGCGCGTTATTTATCGGAGTTGCAGTTGC
GCCCGCGAACGACATTTATAATGAACGTGAATTGCTCAACAGTATGGGCATTTCGCAGCCTACC
GTGGTGTTCGTTTCCAAAAAGGGGTTGCAAAAAATTTTGAACGTGCAAAAAAAGCTCCCAATC
ATCCAAAAAATTATTATCATGGATTCTAAAACGGATTACCAGGGATTTCAGTCGATGTACACGTT
CGTCACATCTCATCTACCTCCCGGTTTTAATGAATACGATTTTGTGCCAGAGTCCTTCGATAGGG
ACAAGACAATTGCACTGATCATGAACTCCTCTGGATCTACTGGTCTGCCTAAAGGTGTCGCTCT
GCCTCATAGAACTGCCTGCGTGAGATTCTCGCATGCCAGAGATCCTATTTTTGGCAATCAAATCA
TTCCGGATACTGCGATTTTAAGTGTTGTTCCATTCCATCACGGTTTTGGAATGTTTACTACACTC
GGATATTTGATATGTGGATTTCGAGTCGTCTTAATGTATAGATTTGAAGAAGAGCTGTTTCTGAG
GAGCCTTCAGGATTACAAGATTCAAAGTGCGCTGCTGGTGCCAACCCTATTCTCCTTCTTCGCC
AAAAGCACTCTGATTGACAAATACGATTTATCTAATTTACACGAAATTGCTTCTGGTGGCGCTCC
CCTCTCTAAGGAAGTCGGGGAAGCGGTTGCCAAGAGGTTCCATCTGCCAGGTATCAGGCAAGG
ATATGGGCTCACTGAGACTACATCAGCTATTCTGATTACACCCGAGGGGGATGATAAACCGGGC
GCGGTCGGTAAAGTTGTTCCATTTTTTGAAGCGAAGGTTGTGGATCTGGATACCGGGAAAACG
 
CTGGGCGTTAATCAAAGAGGCGAACTGTGTGTGAGAGGTCCTATGATTATGTCCGGTTATGTAA
ACAATCCGGAAGCGACCAACGCCTTGATTGACAAGGATGGATGGCTACATTCTGGAGACATAG
CTTACTGGGACGAAGACGAACACTTCTTCATCGTTGACCGCCTGAAGTCTCTGATTAAGTACAA
AGGCTATCAGGTGGCTCCCGCTGAATTGGAATCCATCTTGCTCCAACACCCCAACATCTTCGAC
GCAGGTGTCGCAGGTCTTCCCGACGATGACGCCGGTGAACTTCCCGCCGCCGTTGTTGTTTTG
GAGCACGGAAAGACGATGACGGAAAAAGAGATCGTGGATTACGTCGCCAGTCAAGTAACAAC
CGCGAAAAAGTTGCGCGGAGGAGTTGTGTTTGTGGACGAAGTACCGAAAGGTCTTACCGGAA
AACTCGACGCAAGAAAAATCAGAGAGATCCTCATAAAGGCCAAGAAGGGCGGAAAGATCGCC
GTGTAAGGTACCCGATCAGATTCTAGAGGGCCCTATTCTATAGTGTCACCTAAATGCTAGAGCTC
GCTGATCAGCCTCGACTGTGCCTTCTAGTTGCCAGCCATCTGTTGTTTGCCCCTCCCCCGTGCCT
TCCTTGACCCTGGAAGGTGCCACTCCCACTGTCCTTTCCTAATAAAATGAGGAAATTGCATCGC
ATTGTCTGAGTAGGTGTCATTCTATTCTGGGGGGTGGGGTGGGGCAGGACAGCAAGGGGGAGG
ATTGGGAAGACAATAGCAGGCATGCTGGGGATGCGGTGGGCTCTATGGCTTCTGAGGCGGAAA
GAACCAGCTGGGGCTCTAGGGGGTATCCCCACGCGCCCTGTAGCGGCGCATTAAGCGCGGCGG
GTGTGGTGGTTACGCGCAGCGTGACCGCTACACTTGCCAGCGCCCTAGCGCCCGCTCCTTTCGC
TTTCTTCCCTTCCTTTCTCGCCACGTTCGCCGGCTTTCCCCGTCAAGCTCTAAATCGGGGGCTCC
CTTTAGGGTTCCGATTTAGTGCTTTACGGCACCTCGACCCCAAAAAACTTGATTAGGGTGATGG
TTCACGTAGTGGGCCATCGCCCTGATAGACGGTTTTTCGCCCTTTGACGTTGGAGTCCACGTTC
TTTAATAGTGGACTCTTGTTCCAAACTGGAACAACACTCAACCCTATCTCGGTCTATTCTTTTGA
TTTATAAGGGATTTTGCCGATTTCGGCCTATTGGTTAAAAAATGAGCTGATTTAACAAAAATTTA
ACGCGAATTAATTCTGTGGAATGTGTGTCAGTTAGGGTGTGGAAAGTCCCCAGGCTCCCCAGC
AGGCAGAAGTATGCAAAGCATGCATCTCAATTAGTCAGCAACCAGGTGTGGAAAGTCCCCAGG
CTCCCCAGCAGGCAGAAGTATGCAAAGCATGCATCTCAATTAGTCAGCAACCATAGTCCCGCCC
CTAACTCCGCCCATCCCGCCCCTAACTCCGCCCAGTTCCGCCCATTCTCCGCCCCATGGCTGAC
TAATTTTTTTTATTTATGCAGAGGCCGAGGCCGCCTCTGCCTCTGAGCTATTCCAGAAGTAGTGA
GGAGGCTTTTTTGGAGGCCTAGGCTTTTGCAAAAAGCTCCCGGGAGCTTGTATATCCATTTTCG
GATCTGATCAAGAGACAGGATGAGGATCGTTTCGCGCGGGACTCTGGGGTTCGAAATGACCGA
CCAAGCGACGCCCAACCTGCCATCACGAGATTTCGATTCCACCGCCGCCTTCTATGAAAGGTTG
GGCTTCGGAATCGTTTTCCGGGACGCCGGCTGGATGATCCTCCAGCGCGGGGATCTCATGCTGG
AGTTCTTCGCCCACCCCAACTTGTTTATTGCAGCTTATAATGGTTACAAATAAAGCAATAGCATC
ACAAATTTCACAAATAAAGCATTTTTTTCACTGCATTCTAGTTGTGGTTTGTCCAAACTCATCAA
TGTATCTTATCATGTCTGTATACCGTCGACCTCTAGCTAGAGCTTGGCGTAATCATGGTCATAGCT
GTTTCCTGTGTGAAATTGTTATCCGCTCACAATTCCACACAACATACGAGCCGGAAGCATAAAG
TGTAAAGCCTGGGGTGCCTAATGAGTGAGCTAACTCACATTAATTGCGTTGCGCTCACTGCCCG
CTTTCCAGTCGGGAAACCTGTCGTGCCAGCTGCATTAATGAATCGGCCAACGCGCGGGGAGAG
GCGGTTTGCGTATTGGGCGCTCTTCCGCTTCCTCGCTCACTGACTCGCTGCGCTCGGTCGTTCG
GCTGCGGCGAGCGGTATCAGCTCACTCAAAGGCGGTAATACGGTTATCCACAGAATCAGGGGA
TAACGCAGGAAAGAACATGTGAGCAAAAGGCCAGCAAAAGGCCAGGAACCGTAAAAAGGCC
GCGTTGCTGGCGTTTTTCCATAGGCTCCGCCCCCCTGACGAGCATCACAAAAATCGACGCTCAA
GTCAGAGGTGGCGAAACCCGACAGGACTATAAAGATACCAGGCGTTTCCCCCTGGAAGCTCCC
TCGTGCGCTCTCCTGTTCCGACCCTGCCGCTTACCGGATACCTGTCCGCCTTTCTCCCTTCGGGA
AGCGTGGCGCTTTCTCATAGCTCACGCTGTAGGTATCTCAGTTCGGTGTAGGTCGTTCGCTCCA
AGCTGGGCTGTGTGCACGAACCCCCCGTTCAGCCCGACCGCTGCGCCTTATCCGGTAACTATCG
TCTTGAGTCCAACCCGGTAAGACACGACTTATCGCCACTGGCAGCAGCCACTGGTAACAGGAT
TAGCAGAGCGAGGTATGTAGGCGGTGCTACAGAGTTCTTGAAGTGGTGGCCTAACTACGGCTA
CACTAGAAGAACAGTATTTGGTATCTGCGCTCTGCTGAAGCCAGTTACCTTCGGAAAAAGAGTT
GGTAGCTCTTGATCCGGCAAACAAACCACCGCTGGTAGCGGTGGTTTTTTTGTTTGCAAGCAG
CAGATTACGCGCAGAAAAAAAGGATCTCAAGAAGATCCTTTGATCTTTTCTACGGGGTCTGAC
GCTCAGTGGAACGAAAACTCACGTTAAGGGATTTTGGTCATGAGATTATCAAAAAGGATCTTCA
CCTAGATCCTTTTAAATTAAAAATGAAGTTTTAAATCAATCTAAAGTATATATGAGTAAACTTGGT
CTGACAGTTAGAAAAACTCATCGAGCATCAAATGAAACTGCAATTTATTCATATCAGGATTATCA
ATACCATATTTTTGAAAAAGCCGTTTCTGTAATGAAGGAGAAAACTCACCGAGGCAGTTCCATA
GGATGGCAAGATCCTGGTATCGGTCTGCGATTCCGACTCGTCCAACATCAATACAACCTATTAAT
TTCCCCTCGTCAAAAATAAGGTTATCAAGTGAGAAATCACCATGAGTGACGACTGAATCCGGTG
AGAATGGCAAAAGTTTATGCATTTCTTTCCAGACTTGTTCAACAGGCCAGCCATTACGCTCGTC
ATCAAAATCACTCGCATCAACCAAACCGTTATTCATTCGTGATTGCGCCTGAGCGAGACGAAAT
ACGCGATCGCTGTTAAAAGGACAATTACAAACAGGAATCGAATGCAACCGGCGCAGGAACACT
GCCAGCGCATCAACAATATTTTCACCTGAATCAGGATATTCTTCTAATACCTGGAATGCTGTTTT
CCCGGGGATCGCAGTGGTGAGTAACCATGCATCATCAGGAGTACGGATAAAATGCTTGATGGTC
GGAAGAGGCATAAATTCCGTCAGCCAGTTTAGTCTGACCATCTCATCTGTAACATCATTGGCAA
 
CGCTACCTTTGCCATGTTTCAGAAACAACTCTGGCGCATCGGGCTTCCCATACAATCGATAGATT
GTCGCACCTGATTGCCCGACATTATCGCGAGCCCATTTATACCCATATAAATCAGCATCCATGTTG
GAATTTAATCGCGGCCTAGAGCAAGACGTTTCCCGTTGAATATGGCTCATACTCTTCCTTTTTCA
ATATTATTGAAGCATTTATCAGGGTTATTGTCTCATGAGCGGATACATATTTGAATGTATTTAGAA
AAATAAACAAATAGGGGTTCCGCGCACATTTCCCCGAAAAGTGCCACCTGACGTC。
Plasmid G (SEQ ID NO. 205): 
GACGGATCGGGAGATCTCCCGATCCCCTATGGTGCACTCTCAGTACAATCTGCTCTGATGC
CGCATAGTTAAGCCAGTATCTGCTCCCTGCTTGTGTGTTGGAGGTCGCTGAGTAGTGCGCGAGC
AAAATTTAAGCTACAACAAGGCAAGGCTTGACCGACAATTGCATGAAGAATCTGCTTAGGGTT
AGGCGTTTTGCGCTGCTTCGCGATGTACGGGCCAGATATACGCGTTGACATTGATTATTGACTAG
TTATTAATAGTAATCAATTACGGGGTCATTAGTTCATAGCCCATATATGGAGTTCCGCGTTACATA
ACTTACGGTAAATGGCCCGCCTGGCTGACCGCCCAACGACCCCCGCCCATTGACGTCAATAATG
ACGTATGTTCCCATAGTAACGCCAATAGGGACTTTCCATTGACGTCAATGGGTGGAGTATTTACG
GTAAACTGCCCACTTGGCAGTACATCAAGTGTATCATATGCCAAGTACGCCCCCTATTGACGTCA
ATGACGGTAAATGGCCCGCCTGGCATTATGCCCAGTACATGACCTTATGGGACTTTCCTACTTGG
CAGTACATCTACGTATTAGTCATCGCTATTACCATGGTGATGCGGTTTTGGCAGTACATCAATGG
GCGTGGATAGCGGTTTGACTCACGGGGATTTCCAAGTCTCCACCCCATTGACGTCAATGGGAGT
TTGTTTTGGCACCAAAATCAACGGGACTTTCCAAAATGTCGTAACAACTCCGCCCCATTGACGC
AAATGGGCGGTAGGCGTGTACGGTGGGAGGTCTATATAAGCAGAGCTCTCTGGCTAACTAGAG
AACCCACTGCTTACTGGCTTATCGAAATTAATACGACTCACTATAGGGAAAGCTTGATCAGAAC
GGATCCGCCACCATGGAAGACGCCAAAAACATAAAGAAAGGCCCGGCGCCATTCTATCCGCTG
GAAGATGGAACCGCTGGAGAGCAACTGCATAAGGCTATGAAGAGATACGCCCTGGTTCCTGGA
ACAATTGCTTTTACAGATGCACATATCGAGGTGGACATCACTTACGCTGAGTACTTCGAAATGTC
CGTTCGGTTGGCAGAAGCTATGAAACGATATGGGCTGAATACAAATCACAGAATCGTCGTATGC
AGTGAAAACTCTCTTCAATTCTTTATGCCGGTGTTGGGCGCGTTATTTATCGGAGTTGCAGTTGC
GCCCGCGAACGACATTTATAATGAACGTGAATTGCTCAACAGTATGGGCATTTCGCAGCCTACC
GTGGTGTTCGTTTCCAAAAAGGGGTTGCAAAAAATTTTGAACGTGCAAAAAAAGCTCCCAATC
ATCCAAAAAATTATTATCATGGATTCTAAAACGGATTACCAGGGATTTCAGTCGATGTACACGTT
CGTCACATCTCATCTACCTCCCGGTTTTAATGAATACGATTTTGTGCCAGAGTCCTTCGATAGGG
ACAAGACAATTGCACTGATCATGAACTCCTCTGGATCTACTGGTCTGCCTAAAGGTGTCGCTCT
GCCTCATAGAACTGCCTGCGTGAGATTCTCGCATGCCAGAGATCCTATTTTTGGCAATCAAATCA
TTCCGGATACTGCGATTTTAAGTGTTGTTCCATTCCATCACGGTTTTGGAATGTTTACTACACTC
GGATATTTGATATGTGGATTTCGAGTCGTCTTAATGTATAGATTTGAAGAAGAGCTGTTTCTGAG
GAGCCTTCAGGATTACAAGATTCAAAGTGCGCTGCTGGTGCCAACCCTATTCTCCTTCTTCGCC
AAAAGCACTCTGATTGACAAATACGATTTATCTAATTTACACGAAATTGCTTCTGGTGGCGCTCC
CCTCTCTAAGGAAGTCGGGGAAGCGGTTGCCAAGAGGTTCCATCTGCCAGGTATCAGGCAAGG
ATATGGGCTCACTGAGACTACATCAGCTATTCTGATTACACCCGAGGGGGATGATAAACCGGGC
GCGGTCGGTAAAGTTGTTCCATTTTTTGAAGCGAAGGTTGTGGATCTGGATACCGGGAAAACG
CTGGGCGTTAATCAAAGAGGCGAACTGTGTGTGAGAGGTCCTATGATTATGTCCGGTTATGTAA
ACAATCCGGAAGCGACCAACGCCTTGATTGACAAGGATGGATGGCTACATTCTGGAGACATAG
CTTACTGGGACGAAGACGAACACTTCTTCATCGTTGACCGCCTGAAGTCTCTGATTAAGTACAA
AGGCTATCAGGTGGCTCCCGCTGAATTGGAATCCATCTTGCTCCAACACCCCAACATCTTCGAC
GCAGGTGTCGCAGGTCTTCCCGACGATGACGCCGGTGAACTTCCCGCCGCCGTTGTTGTTTTG
GAGCACGGAAAGACGATGACGGAAAAAGAGATCGTGGATTACGTCGCCAGTCAAGTAACAAC
CGCGAAAAAGTTGCGCGGAGGAGTTGTGTTTGTGGACGAAGTACCGAAAGGTCTTACCGGAA
AACTCGACGCAAGAAAAATCAGAGAGATCCTCATAAAGGCCAAGAAGGGCGGAAAGATCGCC
GTGTAAGGTACCCGATCAGATTCTAGAGGGCCCAAAAAAAAAAAAAAAAAAAAAAAAAAAAAA
GCATATGACTAAAAAAAAAAAAAAAAAAAAAAAAAAAAAAAAAAAAAAAAAAAAAAAAAAAA
AAAAAAAAAAAAAAAAAAAAAAGAGACCGTCTATAGTGTCACCTAAATGCTAGAGCTCGCTG
ATCAGCCTCGACTGTGCCTTCTAGTTGCCAGCCATCTGTTGTTTGCCCCTCCCCCGTGCCTTCCT
TGACCCTGGAAGGTGCCACTCCCACTGTCCTTTCCTAATAAAATGAGGAAATTGCATCGCATTG
TCTGAGTAGGTGTCATTCTATTCTGGGGGGTGGGGTGGGGCAGGACAGCAAGGGGGAGGATTG
GGAAGACAATAGCAGGCATGCTGGGGATGCGGTGGGCTCTATGGCTTCTGAGGCGGAAAGAAC
CAGCTGGGGCTCTAGGGGGTATCCCCACGCGCCCTGTAGCGGCGCATTAAGCGCGGCGGGTGT
GGTGGTTACGCGCAGCGTGACCGCTACACTTGCCAGCGCCCTAGCGCCCGCTCCTTTCGCTTTC
TTCCCTTCCTTTCTCGCCACGTTCGCCGGCTTTCCCCGTCAAGCTCTAAATCGGGGGCTCCCTTT
AGGGTTCCGATTTAGTGCTTTACGGCACCTCGACCCCAAAAAACTTGATTAGGGTGATGGTTCA
CGTAGTGGGCCATCGCCCTGATAGACGGTTTTTCGCCCTTTGACGTTGGAGTCCACGTTCTTTA
ATAGTGGACTCTTGTTCCAAACTGGAACAACACTCAACCCTATCTCGGTCTATTCTTTTGATTTA
 
TAAGGGATTTTGCCGATTTCGGCCTATTGGTTAAAAAATGAGCTGATTTAACAAAAATTTAACGC
GAATTAATTCTGTGGAATGTGTGTCAGTTAGGGTGTGGAAAGTCCCCAGGCTCCCCAGCAGGC
AGAAGTATGCAAAGCATGCATCTCAATTAGTCAGCAACCAGGTGTGGAAAGTCCCCAGGCTCC
CCAGCAGGCAGAAGTATGCAAAGCATGCATCTCAATTAGTCAGCAACCATAGTCCCGCCCCTAA
CTCCGCCCATCCCGCCCCTAACTCCGCCCAGTTCCGCCCATTCTCCGCCCCATGGCTGACTAATT
TTTTTTATTTATGCAGAGGCCGAGGCCGCCTCTGCCTCTGAGCTATTCCAGAAGTAGTGAGGAG
GCTTTTTTGGAGGCCTAGGCTTTTGCAAAAAGCTCCCGGGAGCTTGTATATCCATTTTCGGATCT
GATCAAGAGACAGGATGAGGATCGTTTCGCGCGGGACTCTGGGGTTCGAAATGACCGACCAAG
CGACGCCCAACCTGCCATCACGAGATTTCGATTCCACCGCCGCCTTCTATGAAAGGTTGGGCTT
CGGAATCGTTTTCCGGGACGCCGGCTGGATGATCCTCCAGCGCGGGGATCTCATGCTGGAGTTC
TTCGCCCACCCCAACTTGTTTATTGCAGCTTATAATGGTTACAAATAAAGCAATAGCATCACAAA
TTTCACAAATAAAGCATTTTTTTCACTGCATTCTAGTTGTGGTTTGTCCAAACTCATCAATGTATC
TTATCATGTCTGTATACCGTCGACCTCTAGCTAGAGCTTGGCGTAATCATGGTCATAGCTGTTTCC
TGTGTGAAATTGTTATCCGCTCACAATTCCACACAACATACGAGCCGGAAGCATAAAGTGTAAA
GCCTGGGGTGCCTAATGAGTGAGCTAACTCACATTAATTGCGTTGCGCTCACTGCCCGCTTTCC
AGTCGGGAAACCTGTCGTGCCAGCTGCATTAATGAATCGGCCAACGCGCGGGGAGAGGCGGTT
TGCGTATTGGGCGCTCTTCCGCTTCCTCGCTCACTGACTCGCTGCGCTCGGTCGTTCGGCTGCG
GCGAGCGGTATCAGCTCACTCAAAGGCGGTAATACGGTTATCCACAGAATCAGGGGATAACGC
AGGAAAGAACATGTGAGCAAAAGGCCAGCAAAAGGCCAGGAACCGTAAAAAGGCCGCGTTG
CTGGCGTTTTTCCATAGGCTCCGCCCCCCTGACGAGCATCACAAAAATCGACGCTCAAGTCAG
AGGTGGCGAAACCCGACAGGACTATAAAGATACCAGGCGTTTCCCCCTGGAAGCTCCCTCGTG
CGCTCTCCTGTTCCGACCCTGCCGCTTACCGGATACCTGTCCGCCTTTCTCCCTTCGGGAAGCG
TGGCGCTTTCTCATAGCTCACGCTGTAGGTATCTCAGTTCGGTGTAGGTCGTTCGCTCCAAGCT
GGGCTGTGTGCACGAACCCCCCGTTCAGCCCGACCGCTGCGCCTTATCCGGTAACTATCGTCTT
GAGTCCAACCCGGTAAGACACGACTTATCGCCACTGGCAGCAGCCACTGGTAACAGGATTAGC
AGAGCGAGGTATGTAGGCGGTGCTACAGAGTTCTTGAAGTGGTGGCCTAACTACGGCTACACT
AGAAGAACAGTATTTGGTATCTGCGCTCTGCTGAAGCCAGTTACCTTCGGAAAAAGAGTTGGT
AGCTCTTGATCCGGCAAACAAACCACCGCTGGTAGCGGTGGTTTTTTTGTTTGCAAGCAGCAG
ATTACGCGCAGAAAAAAAGGATCTCAAGAAGATCCTTTGATCTTTTCTACGGGGTCTGACGCTC
AGTGGAACGAAAACTCACGTTAAGGGATTTTGGTCATGAGATTATCAAAAAGGATCTTCACCTA
GATCCTTTTAAATTAAAAATGAAGTTTTAAATCAATCTAAAGTATATATGAGTAAACTTGGTCTG
ACAGTTAGAAAAACTCATCGAGCATCAAATGAAACTGCAATTTATTCATATCAGGATTATCAATA
CCATATTTTTGAAAAAGCCGTTTCTGTAATGAAGGAGAAAACTCACCGAGGCAGTTCCATAGGA
TGGCAAGATCCTGGTATCGGTCTGCGATTCCGACTCGTCCAACATCAATACAACCTATTAATTTC
CCCTCGTCAAAAATAAGGTTATCAAGTGAGAAATCACCATGAGTGACGACTGAATCCGGTGAG
AATGGCAAAAGTTTATGCATTTCTTTCCAGACTTGTTCAACAGGCCAGCCATTACGCTCGTCATC
AAAATCACTCGCATCAACCAAACCGTTATTCATTCGTGATTGCGCCTGAGCGAGACGAAATACG
CGATCGCTGTTAAAAGGACAATTACAAACAGGAATCGAATGCAACCGGCGCAGGAACACTGCC
AGCGCATCAACAATATTTTCACCTGAATCAGGATATTCTTCTAATACCTGGAATGCTGTTTTCCC
GGGGATCGCAGTGGTGAGTAACCATGCATCATCAGGAGTACGGATAAAATGCTTGATGGTCGGA
AGAGGCATAAATTCCGTCAGCCAGTTTAGTCTGACCATCTCATCTGTAACATCATTGGCAACGCT
ACCTTTGCCATGTTTCAGAAACAACTCTGGCGCATCGGGCTTCCCATACAATCGATAGATTGTC
GCACCTGATTGCCCGACATTATCGCGAGCCCATTTATACCCATATAAATCAGCATCCATGTTGGA
ATTTAATCGCGGCCTAGAGCAAGACGTTTCCCGTTGAATATGGCTCATACTCTTCCTTTTTCAAT
ATTATTGAAGCATTTATCAGGGTTATTGTCTCATGAGCGGATACATATTTGAATGTATTTAGAAAA
ATAAACAAATAGGGGTTCCGCGCACATTTCCCCGAAAAGTGCCACCTGACGTC。
Plasmid H (SEQ ID NO. 206):

GACGGATCGGGAGATCTCCCGATCCCCTATGGTGCACTCTCAGTACAATCTGCTCTGATGC
CGCATAGTTAAGCCAGTATCTGCTCCCTGCTTGTGTGTTGGAGGTCGCTGAGTAGTGCGCGAGC
AAAATTTAAGCTACAACAAGGCAAGGCTTGACCGACAATTGCATGAAGAATCTGCTTAGGGTT
AGGCGTTTTGCGCTGCTTCGCGATGTACGGGCCAGATATACGCGTTGACATTGATTATTGACTAG
TTATTAATAGTAATCAATTACGGGGTCATTAGTTCATAGCCCATATATGGAGTTCCGCGTTACATA
ACTTACGGTAAATGGCCCGCCTGGCTGACCGCCCAACGACCCCCGCCCATTGACGTCAATAATG
ACGTATGTTCCCATAGTAACGCCAATAGGGACTTTCCATTGACGTCAATGGGTGGAGTATTTACG
GTAAACTGCCCACTTGGCAGTACATCAAGTGTATCATATGCCAAGTACGCCCCCTATTGACGTCA
ATGACGGTAAATGGCCCGCCTGGCATTATGCCCAGTACATGACCTTATGGGACTTTCCTACTTGG
CAGTACATCTACGTATTAGTCATCGCTATTACCATGGTGATGCGGTTTTGGCAGTACATCAATGG
GCGTGGATAGCGGTTTGACTCACGGGGATTTCCAAGTCTCCACCCCATTGACGTCAATGGGAGT
TTGTTTTGGCACCAAAATCAACGGGACTTTCCAAAATGTCGTAACAACTCCGCCCCATTGACGC

AAATGGGCGGTAGGCGTGTACGGTGGGAGGTCTATATAAGCAGAGCTCTCTGGCTAACTAGAG
AACCCACTGCTTACTGGCTTATCGAAATTAATACGACTCACTATAGGGAAAGCTTTAATACGACT
CACTATAGGACAGATCGCCTGGAGACGCCATCCACGCTGTTTTGACCTCCATAGAAGACACCGG
GACCGATCCAGCCTCCGCGGCCGGGAACGGTGCATTGGAACGCGGATTCCCCGTGCCAAGAGT
GACGTAAGTACCGCAGTCACCGTCCTTGACACGGGATCCGCCACCATGGAAGACGCCAAAAAC
ATAAAGAAAGGCCCGGCGCCATTCTATCCGCTGGAAGATGGAACCGCTGGAGAGCAACTGCAT
AAGGCTATGAAGAGATACGCCCTGGTTCCTGGAACAATTGCTTTTACAGATGCACATATCGAGG
TGGACATCACTTACGCTGAGTACTTCGAAATGTCCGTTCGGTTGGCAGAAGCTATGAAACGATA
TGGGCTGAATACAAATCACAGAATCGTCGTATGCAGTGAAAACTCTCTTCAATTCTTTATGCCG
GTGTTGGGCGCGTTATTTATCGGAGTTGCAGTTGCGCCCGCGAACGACATTTATAATGAACGTG
AATTGCTCAACAGTATGGGCATTTCGCAGCCTACCGTGGTGTTCGTTTCCAAAAAGGGGTTGCA
AAAAATTTTGAACGTGCAAAAAAAGCTCCCAATCATCCAAAAAATTATTATCATGGATTCTAAA
ACGGATTACCAGGGATTTCAGTCGATGTACACGTTCGTCACATCTCATCTACCTCCCGGTTTTAA
TGAATACGATTTTGTGCCAGAGTCCTTCGATAGGGACAAGACAATTGCACTGATCATGAACTCC
TCTGGATCTACTGGTCTGCCTAAAGGTGTCGCTCTGCCTCATAGAACTGCCTGCGTGAGATTCT
CGCATGCCAGAGATCCTATTTTTGGCAATCAAATCATTCCGGATACTGCGATTTTAAGTGTTGTT
CCATTCCATCACGGTTTTGGAATGTTTACTACACTCGGATATTTGATATGTGGATTTCGAGTCGTC
TTAATGTATAGATTTGAAGAAGAGCTGTTTCTGAGGAGCCTTCAGGATTACAAGATTCAAAGTG
CGCTGCTGGTGCCAACCCTATTCTCCTTCTTCGCCAAAAGCACTCTGATTGACAAATACGATTTA
TCTAATTTACACGAAATTGCTTCTGGTGGCGCTCCCCTCTCTAAGGAAGTCGGGGAAGCGGTTG
CCAAGAGGTTCCATCTGCCAGGTATCAGGCAAGGATATGGGCTCACTGAGACTACATCAGCTAT
TCTGATTACACCCGAGGGGGATGATAAACCGGGCGCGGTCGGTAAAGTTGTTCCATTTTTTGAA
GCGAAGGTTGTGGATCTGGATACCGGGAAAACGCTGGGCGTTAATCAAAGAGGCGAACTGTGT
GTGAGAGGTCCTATGATTATGTCCGGTTATGTAAACAATCCGGAAGCGACCAACGCCTTGATTG
ACAAGGATGGATGGCTACATTCTGGAGACATAGCTTACTGGGACGAAGACGAACACTTCTTCAT
CGTTGACCGCCTGAAGTCTCTGATTAAGTACAAAGGCTATCAGGTGGCTCCCGCTGAATTGGAA
TCCATCTTGCTCCAACACCCCAACATCTTCGACGCAGGTGTCGCAGGTCTTCCCGACGATGACG
CCGGTGAACTTCCCGCCGCCGTTGTTGTTTTGGAGCACGGAAAGACGATGACGGAAAAAGAG
ATCGTGGATTACGTCGCCAGTCAAGTAACAACCGCGAAAAAGTTGCGCGGAGGAGTTGTGTTT
GTGGACGAAGTACCGAAAGGTCTTACCGGAAAACTCGACGCAAGAAAAATCAGAGAGATCCT
CATAAAGGCCAAGAAGGGCGGAAAGATCGCCGTGTAAGGTACCCGGGTGGCATCCCTGTGACC
CCTCCCCAGTGCCTCTCCTGGCCCTGGAAGTTGCCACTCCAGTGCCCACCAGCCTTGTCCTAAT
AAAATTAAGTTGCATCGGGCCCAAAAAAAAAAAAAAAAAAAAAAAAAAAAAAGCATATGACT
AAAAAAAAAAAAAAAAAAAAAAAAAAAAAAAAAAAAAAAAAAAAAAAAAAAAAAAAAAAAAAAA
AAAAAAAAAAAAAGAGACCGTCTATAGTGTCACCTAAATGCTAGAGCTCGCTGATCAGCCTCGA
CTGTGCCTTCTAGTTGCCAGCCATCTGTTGTTTGCCCCTCCCCCGTGCCTTCCTTGACCCTGGAA
GGTGCCACTCCCACTGTCCTTTCCTAATAAAATGAGGAAATTGCATCGCATTGTCTGAGTAGGT
GTCATTCTATTCTGGGGGGTGGGGTGGGGCAGGACAGCAAGGGGGAGGATTGGGAAGACAAT
AGCAGGCATGCTGGGGATGCGGTGGGCTCTATGGCTTCTGAGGCGGAAAGAACCAGCTGGGG
CTCTAGGGGGTATCCCCACGCGCCCTGTAGCGGCGCATTAAGCGCGGCGGGTGTGGTGGTTAC
GCGCAGCGTGACCGCTACACTTGCCAGCGCCCTAGCGCCCGCTCCTTTCGCTTTCTTCCCTTCC
TTTCTCGCCACGTTCGCCGGCTTTCCCCGTCAAGCTCTAAATCGGGGGCTCCCTTTAGGGTTCC
GATTTAGTGCTTTACGGCACCTCGACCCCAAAAAACTTGATTAGGGTGATGGTTCACGTAGTGG
GCCATCGCCCTGATAGACGGTTTTTCGCCCTTTGACGTTGGAGTCCACGTTCTTTAATAGTGGAC
TCTTGTTCCAAACTGGAACAACACTCAACCCTATCTCGGTCTATTCTTTTGATTTATAAGGGATT
TTGCCGATTTCGGCCTATTGGTTAAAAAATGAGCTGATTTAACAAAAATTTAACGCGAATTAATT
CTGTGGAATGTGTGTCAGTTAGGGTGTGGAAAGTCCCCAGGCTCCCCAGCAGGCAGAAGTATG
CAAAGCATGCATCTCAATTAGTCAGCAACCAGGTGTGGAAAGTCCCCAGGCTCCCCAGCAGGC
AGAAGTATGCAAAGCATGCATCTCAATTAGTCAGCAACCATAGTCCCGCCCCTAACTCCGCCCA
TCCCGCCCCTAACTCCGCCCAGTTCCGCCCATTCTCCGCCCCATGGCTGACTAATTTTTTTTATTT
ATGCAGAGGCCGAGGCCGCCTCTGCCTCTGAGCTATTCCAGAAGTAGTGAGGAGGCTTTTTTG
GAGGCCTAGGCTTTTGCAAAAAGCTCCCGGGAGCTTGTATATCCATTTTCGGATCTGATCAAGA
GACAGGATGAGGATCGTTTCGCGCGGGACTCTGGGGTTCGAAATGACCGACCAAGCGACGCCC
AACCTGCCATCACGAGATTTCGATTCCACCGCCGCCTTCTATGAAAGGTTGGGCTTCGGAATCG
TTTTCCGGGACGCCGGCTGGATGATCCTCCAGCGCGGGGATCTCATGCTGGAGTTCTTCGCCCA
CCCCAACTTGTTTATTGCAGCTTATAATGGTTACAAATAAAGCAATAGCATCACAAATTTCACAA
ATAAAGCATTTTTTTCACTGCATTCTAGTTGTGGTTTGTCCAAACTCATCAATGTATCTTATCATG
TCTGTATACCGTCGACCTCTAGCTAGAGCTTGGCGTAATCATGGTCATAGCTGTTTCCTGTGTGA
AATTGTTATCCGCTCACAATTCCACACAACATACGAGCCGGAAGCATAAAGTGTAAAGCCTGGG
GTGCCTAATGAGTGAGCTAACTCACATTAATTGCGTTGCGCTCACTGCCCGCTTTCCAGTCGGG

AAACCTGTCGTGCCAGCTGCATTAATGAATCGGCCAACGCGCGGGGAGAGGCGGTTTGCGTAT
TGGGCGCTCTTCCGCTTCCTCGCTCACTGACTCGCTGCGCTCGGTCGTTCGGCTGCGGCGAGCG
GTATCAGCTCACTCAAAGGCGGTAATACGGTTATCCACAGAATCAGGGGATAACGCAGGAAAG
AACATGTGAGCAAAAGGCCAGCAAAAGGCCAGGAACCGTAAAAAGGCCGCGTTGCTGGCGTT
TTTCCATAGGCTCCGCCCCCCTGACGAGCATCACAAAAATCGACGCTCAAGTCAGAGGTGGCG
AAACCCGACAGGACTATAAAGATACCAGGCGTTTCCCCCTGGAAGCTCCCTCGTGCGCTCTCCT
GTTCCGACCCTGCCGCTTACCGGATACCTGTCCGCCTTTCTCCCTTCGGGAAGCGTGGCGCTTT
CTCATAGCTCACGCTGTAGGTATCTCAGTTCGGTGTAGGTCGTTCGCTCCAAGCTGGGCTGTGT
GCACGAACCCCCCGTTCAGCCCGACCGCTGCGCCTTATCCGGTAACTATCGTCTTGAGTCCAAC
CCGGTAAGACACGACTTATCGCCACTGGCAGCAGCCACTGGTAACAGGATTAGCAGAGCGAGG
TATGTAGGCGGTGCTACAGAGTTCTTGAAGTGGTGGCCTAACTACGGCTACACTAGAAGAACA
GTATTTGGTATCTGCGCTCTGCTGAAGCCAGTTACCTTCGGAAAAAGAGTTGGTAGCTCTTGAT
CCGGCAAACAAACCACCGCTGGTAGCGGTGGTTTTTTTGTTTGCAAGCAGCAGATTACGCGCA
GAAAAAAAGGATCTCAAGAAGATCCTTTGATCTTTTCTACGGGGTCTGACGCTCAGTGGAACG
AAAACTCACGTTAAGGGATTTTGGTCATGAGATTATCAAAAAGGATCTTCACCTAGATCCTTTTA
AATTAAAAATGAAGTTTTAAATCAATCTAAAGTATATATGAGTAAACTTGGTCTGACAGTTAGAA
AAACTCATCGAGCATCAAATGAAACTGCAATTTATTCATATCAGGATTATCAATACCATATTTTTG
AAAAAGCCGTTTCTGTAATGAAGGAGAAAACTCACCGAGGCAGTTCCATAGGATGGCAAGATC
CTGGTATCGGTCTGCGATTCCGACTCGTCCAACATCAATACAACCTATTAATTTCCCCTCGTCAA
AAATAAGGTTATCAAGTGAGAAATCACCATGAGTGACGACTGAATCCGGTGAGAATGGCAAAA
GTTTATGCATTTCTTTCCAGACTTGTTCAACAGGCCAGCCATTACGCTCGTCATCAAAATCACTC
GCATCAACCAAACCGTTATTCATTCGTGATTGCGCCTGAGCGAGACGAAATACGCGATCGCTGT
TAAAAGGACAATTACAAACAGGAATCGAATGCAACCGGCGCAGGAACACTGCCAGCGCATCA
ACAATATTTTCACCTGAATCAGGATATTCTTCTAATACCTGGAATGCTGTTTTCCCGGGGATCGC
AGTGGTGAGTAACCATGCATCATCAGGAGTACGGATAAAATGCTTGATGGTCGGAAGAGGCATA
AATTCCGTCAGCCAGTTTAGTCTGACCATCTCATCTGTAACATCATTGGCAACGCTACCTTTGCC
ATGTTTCAGAAACAACTCTGGCGCATCGGGCTTCCCATACAATCGATAGATTGTCGCACCTGATT
GCCCGACATTATCGCGAGCCCATTTATACCCATATAAATCAGCATCCATGTTGGAATTTAATCGCG
GCCTAGAGCAAGACGTTTCCCGTTGAATATGGCTCATACTCTTCCTTTTTCAATATTATTGAAGC
ATTTATCAGGGTTATTGTCTCATGAGCGGATACATATTTGAATGTATTTAGAAAAATAAACAAATA
GGGGTTCCGCGCACATTTCCCCGAAAAGTGCCACCTGACGTC.

## Claims

1. A polynucleotide comprising a modified poly(A), wherein the number of nucleotides forming the modified poly(A) is at least 30, and the modified poly(A) comprises:
i) at least two R elements defined as polynucleotides consisting of A nucleotide; and
ii) at least one S element, wherein the S element:
a) is a nucleotide other than A nucleotide, or
b) consists of at least two nucleotides, wherein the nucleotides at both ends are not A nucleotide;
wherein the total number of the S element is one less than the total number of the R element, the S element is connected to the R element, and each of the S elements is flanked at both ends by the R element.

2. The polynucleotide of claim 1, wherein the modified poly(A) satisfies one of the followings:
(1) the number of the nucleotides of the modified poly(A) is in a range of 30-70, 30-80, 30-90, 30-100, 30-110, 30-120, 30-130, 30-140, 40-70, 40-80, 40-90, 40-100, 40-110, 40-120, 40-130, 40-140, 50-70, 50-80, 50-90, 50-100, 50-110, 50-120, 50-130, 50-140, 60-70, 60-80, 60-90, 60-100, 60-110, 60-120, 60-130, 60-140, 70-80, 70-90, 70-100, 70-110, 70-120, 70-130, 70-140, 80-90, 80-100, 80-110, 80-120, 80-130, 80-140, 90-100, 90-110, 90-120, 90-130 or 90-140;
preferably, the number of the nucleotides of the modified poly(A) is in a range of 31-70, 35-70, 41-70, 45-70, 51-70, 55-70, 61-70, 65-70, 30-75, 35-75, 40-75, 45-75, 50-75, 55-75, 60-75, 65-75, 70-75, 30-79, 35-79, 40-79, 45-79, 50-79, 55-79, 60-79, 65-79, 70-79, 75-79, 81-99, 85-99, 90-99, 95-99, 101-109, 105-109, 111-119, 115-119, 105-119, 109-119, 115-119, 122-125, 127-129 or 133-140;
(2) the number of A nucleotide of at least one R element is in a range of 5-20, 8-20, 10-20, 15-20, 5-30, 8-30, 10-30, 20-30, 5-40, 8-40, 10-40, 20-40, 30-40, 5-50, 8-50, 10-50, 20-50, 30-50, 40-50, 5-60, 8-60, 10-60, 20-60, 30-60, 40-60, 50-60, 5-70, 8-70, 10-70, 20-70, 30-70, 40-70, 50-70, 60-70, 5-80, 8-80, 10-80, 20-80, 30-80, 40-80, 50-80, 60-80, 70-80, 5-90, 8-90, 10-90, 20-90, 30-90, 40-90, 50-90, 60-90, 70-90, 80-90, 5-100, 8-100, 10-100, 20-100, 30-100, 40-100, 50-100, 60-100, 70-100, 80-100, 90-100, 5-110, 8-110, 10-110, 20-110, 30-110, 40-110, 50-110, 60-110, 70-110, 80-110, 90-110 or 100-110;
preferably, the number of A nucleotide of at least one R element is in a range of 5-19, 8-19, 10-19, 11-19, 13-19, 16-19, 5-18, 8-18, 10-18, 11-18, 13-18, 16-18, 21-29, 31-39, 41-44, 46-49, 51-54, 56-59, 61-64, 66-69, 71-79, 81-99 or greater than 100;
(3) the number of the nucleotides of at least one S element is at least 1, 2, 5, 10, 15 or 20, and the number of the nucleotides of at least one S element is no more than 15, no more than 20, no more than 30, no more than 35 or no more than 40; preferably, the number of the nucleotides of at least one S element is 1, 2-10, 2-15, 2-20, 2-25, 3-10, 3-15, 3-20 or 3-25;
(4) the number of the S element is 1, at least 2, at least 5, at least 8 or at least 10, preferably, the number of the S element is 1, 2, 3 or 4.

3. The polynucleotide of claim 1 or 2, wherein the number of the nucleotides of the modified poly(A) is in a range of 40-70, 40-80, 40-90, 40-100, 40-110, 40-120, 40-130, 40-140, 50-70, 50-80, 50-90, 50-100, 50-110, 50-120, 50-130, 50-140, 60-70, 60-80, 60-90, 60-100, 60-110, 60-120, 60-130, 60-140, 70-80, 70-90, 70-100, 70-110, 70-120, 70-130, 70-140, 80-90, 80-100, 80-110, 80-120, 80-130, 80-140, 90-100, 90-110, 90-120, 90-130 or 90-140.

4. The polynucleotide of any one of claims 1-3, wherein the number of A nucleotide of at least one R element of the modified poly(A) is in a range of 5-20, 8-20, 10-20, 15-20, 5-30, 8-30, 10-30, 20-30, 5-40, 8-40, 10-40, 20-40, 30-40, 5-50, 8-50, 10-50, 20-50, 30-50, 40-50, 5-60, 8-60, 10-60, 20-60, 30-60, 40-60 or 50-60.

5. The polynucleotide of any one of claims 1-4, wherein the number of the nucleotides of the modified poly(A) is 30-80, and the number of A nucleotide of at least one R element of the modified poly(A) is less than 35.

6. The polynucleotide of any one of claims 1-5, wherein the number of the nucleotides of the modified poly(A) is 30-70.

7. The polynucleotide of any one of claims 1-6, wherein the number of the nucleotides of the modified poly(A) is in a range of 41-70, 45-70, 51-70, 55-70, 58-70 or 61-70.

8. The polynucleotide of any one of claims 1-7, wherein the number of A nucleotide of at least one R element of the modified poly(A) is less than 20.

9. The polynucleotide of any one of claims 1-8, wherein the number of A nucleotide of at least one R element of the modified poly(A) is in a range of 5-19, 8-19, 10-19, 11-19, 13-19, 16-19, 5-18, 8-18, 10-18, 11-18, 13-18 or 16-18.

10. The polynucleotide of any one of claims 1-4, wherein the number of the nucleotides of the modified poly(A) is 71-79, and the modified poly(A) further satisfies at least one of the followings:
(1) the number of A nucleotide of at least one R element is less than 35, 36-39, 41-54, 56-59, 61-64, or greater than 65; and
(2) the number of A nucleotide of at least one R element is not an integer between 35 and 65.

11. The polynucleotide of any one of claims 1-10, wherein the modified poly(A) comprises at least three R elements or at least four R elements.

12. The polynucleotide of any one of claims 1-11, wherein the modified poly(A) comprising a nucleotide sequence at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identical to one of the nucleotide sequences of SEQ ID NOs: 2-100 and 212-247, wherein the R element remains unchanged; or
the nucleotide sequence of the modified poly(A) has at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one of the nucleotide sequences of SEQ ID NOs: 2-100 and 212-247, wherein the R element remains unchanged; or
the modified poly(A) comprises any of the nucleotide sequences of SEQ ID NOs: 2-100 and 212-247; or
the nucleotide sequence of the modified poly(A) is set forth in any one of SEQ ID NOs: 2-100 and 212-247.

13. The polynucleotide of any one of claims 1-12, wherein the polynucleotide further comprises at least one of 5'-UTR, a nucleic acid encoding a polypeptide or protein of interest, and 3'-UTR that are located upstream of the modified poly(A); or
the polynucleotide can be transcribed in vitro to an RNA.

14. The polynucleotide of any one of claims 1-11, wherein the modified poly(A) comprises a nucleotide sequence having at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one of the nucleotide sequences of SEQ ID NOs: 102-200 and 248-283, wherein the R element remains unchanged; or
the nucleotide sequence of the modified poly(A) has at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% identity to one of the nucleotide sequences of SEQ ID NOs: 102-200 and 248-283, wherein the R element remains unchanged; or
the modified poly(A) comprises any of the nucleotide sequences of SEQ ID NOs: 102-200 and 248-283; or
the nucleotide sequence of the modified poly(A) is set forth in any of SEQ ID NOs: 102-200 and 248-283.

15. The polynucleotide of any one of claims 1-14, wherein the polynucleotide comprises a modified nucleotide;
preferably, the polynucleotide contains a modified nucleoside;
preferably, the modified nucleoside comprises at least one of a modified uridine, a modified cytidine, a modified adenosine and a modified guanosine;
preferably, 100% of uridines in the polynucleotide are modified.

16. A vector comprising the polynucleotide of any one of claims 1-15.

17. The vector of claim 16, wherein the vector is an expression vector; further, the vector further comprises at least one of a promoter, 5'-UTR and 3'-UTR that are located upstream of the modified poly(A); further, the modified poly(A), the promoter, the 5'-UTR and the 3'-UTR are operably linked to each other;
further, the vector further comprises a nucleic acid encoding a polypeptide or protein of interest, and the nucleic acid encoding a polypeptide or protein of interest is located between the 5'-UTR and the 3'-UTR.

18. A host cell comprising the polynucleotide of any one of claims 1-15 or a vector of any one of claims 16-17;
further, the host cell is an antigen-presenting cell; furthermore, the host cell is dendritic cell, monocyte, or macrophage.

19. A method for preparing RNA, comprising a step of transcribing the polynucleotide of any one of claims 1-13:
or, comprising a step of preparing an RNA by chemical synthesis based on the nucleotide sequence of the polynucleotide of any one of claims 1-13.

20. An RNA obtained by the method of claim 19;
preferably, the RNA is mRNA.

21. A method for preparing a polynucleotide, comprising a step of introducing the polynucleotide of any one of claims 1-13 into a host cell and then culturing the host cell containing the polynucleotide; further, the host cell comprises *Escherichia coli.*

22. A method for preparing a polypeptide or protein, comprising:
transcribing the polynucleotide of any one of claims 1-13 to an mRNA; and
translating the mRNA into a polypeptide or protein.

23. A lipid nanoparticle (LNP) comprising the polynucleotide of any one of claims 1-15 or the RNA of claim 20.

24. A pharmaceutical composition comprising the polynucleotide of any one of claims 1-15, the vector of any one of claims 16-17, the host cell of claim 18, the RNA of claim 20, the polypeptide or protein prepared by the method for preparing a polypeptide or protein of claim 22, or the lipid nanoparticle of claim 23, and a pharmaceutically acceptable excipient.

25. Use of the polynucleotide of any one of claims 1-15, the vector of any one of claims 16-17, the host cell of claim 18, the RNA of claim 20, the polypeptide or protein prepared by the method for preparing a polypeptide or protein of claim 22, the lipid nanoparticle of claim 23 or the pharmaceutical composition according to claim 24 in the preparation of a medicament;
preferably, the medicament is used in gene therapy, gene vaccination or protein replacement therapy;
further, the medicament is used in the treatment and/or prevention of a disease; preferably, the medicament is a vaccine;
preferably, the disease is selected from the group consisting of rare diseases, infectious diseases, cancers, hereditary diseases, autoimmune diseases, diabetes, neurodegenerative diseases, cardiovascular diseases, renal vascular diseases and metabolic diseases;
preferably, the cancers comprise one or more of lung cancer, gastric cancer, liver cancer, esophageal cancer, colon cancer, pancreatic cancer, brain cancer, lymphoma, leukemia and prostate cancer; the hereditary diseases comprise one or more of hemophilia, thalassemia and Gaucher's disease; the rare diseases comprise one or more of Brittle Bone Disease, Wilson disease, spinal muscular atrophy, Huntington's disease, Rett syndrome, amyotrophic lateral sclerosis, Duchenne Type Muscular Dystrophy, Friedrichs ataxia, Methylmalonic Acidemia, cystic fibrosis, glycogen storage disease 1a, glycogen storage disease III, Crigler-Najjar syndrome, ornithine transcarbamylase deficiency, Propionic Acidemia, phenylketonuria, hemophilia A, hemophilia B, β-thalassemia, Lafora disease, Dravet syndrome, Alexander disease, Leber's congenital amaurosis, myelodysplastic syndromes, and Homocystinuria due to CBS deficiency;
preferably, the medicament is a nucleic acid drug, wherein the nucleic acid comprises at least one of RNA, messenger RNA (mRNA), DNA, plasmid, ribosomal RNA (rRNA), single guide RNA (sgRNA) and cas9 mRNA.
